# EUROPEAN PATENT APPLICATION

(11) **EP 4 847 490 A1**
(43) Date of publication of application: **02.12.2026**
(21) Application number: 25744860.5
(22) Date of filing: 20.01.2025
(51) Int. Cl.: C07D 249/04, A01N 43/78, A01N 43/647, A01P 3/00, C07D 401/12, C07D 403/12, C07D 417/12

(54) **FIVE-MEMBER HETEROARYL COMPOUND AND BACTERICIDE FOR AGRICULTURAL AND HORTICULTURAL USE**

(30) Priority: 22.01.2024 JP 2024007491; 19.04.2024 JP 2024068592; 26.06.2024 JP 2024103174; 21.10.2024 JP 2024185251
(71) Applicant: Nippon Soda Co., Ltd., Tokyo 100-7010 (JP)
(72) Inventor: NISHIKI, Haruka, Odawara-shi, Kanagawa 250-0280 (JP); KOBAYASHI, Hiroyuki, Odawara-shi, Kanagawa 250-0280 (JP); KASHIWA, Shuntaro, Odawara-shi, Kanagawa 250-0280 (JP); SHIBAYAMA, Kotaro, Odawara-shi, Kanagawa 250-0280 (JP); SUZUKI, Hiroto, Odawara-shi, Kanagawa 250-0280 (JP); KUWAHARA, Raito, Odawara-shi, Kanagawa 250-0280 (JP); KUBOTA, Kento, Odawara-shi, Kanagawa 250-0280 (JP); FUJIMORI, Haruka, Odawara-shi, Kanagawa 250-0280 (JP)
(74) Representative: Sonnenhauser, Thomas Martin
(86) International application number: PCT/JP2025/001582
(87) International publication number: WO 2025/159046

(57) **Abstract**

A compound represented by Formula (Z-I) or a salt thereof is provided (in the formula, Q¹ and Q² each represent a phenyl group or the like, B¹, B², and B³ each represent an oxygen atom, a nitrogen atom, a group represented by NR¹, or a group represented by CR², R¹ represents an alkyl group or the like, R² represents a hydrogen atom or the like, Z^{a} represents an oxygen atom or the like, T represents a partial structure (T-1) or (T-2), Z represents an oxygen atom or the like, R⁶ represents a hydrogen atom or the like, Z^{b} represents an alkoxy group or the like, R⁴ and R⁵ each represent a hydrogen atom or the like, and n represents any integer of 1 to 3).

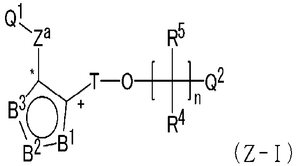

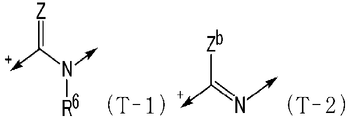

## Description

### TECHNICAL FIELD

The present invention relates to a 5-membered heteroaryl compound and an agricultural and horticultural fungicide. More specifically, the present invention relates to a 5-membered heteroaryl compound having excellent fungicidal activity against pathogenic fungi affecting agricultural and horticultural plants, having excellent safety, and being industrially advantageously synthesizable, and to an agricultural and horticultural fungicide containing the 5-membered heteroaryl compound as an active ingredient. Priority is claimed on Japanese Patent Application No. 2024-007491, filed January 22, 2024, Japanese Patent Application No. 2024-068592, filed April 19, 2024, Japanese Patent Application No. 2024-103174, filed June 26, 2024, and Japanese Patent Application No. 2024-185251, filed October 21, 2024, the contents of which are incorporated herein by reference.

### BACKGROUND ART

Various compounds having fungicidal activity against pathogenic fungi affecting agricultural and horticultural plants have been proposed. In order to put such compounds into practical use as agricultural and horticultural fungicides, it is required not only that the efficacy be sufficiently high, but also that resistance to the agents be less likely to develop, that no phytotoxicity to plants or soil contamination be caused, and that toxicity to livestock, fish, and the like be low.

Meanwhile, Patent Document 1 discloses a compound represented by Formula (A) or the like.

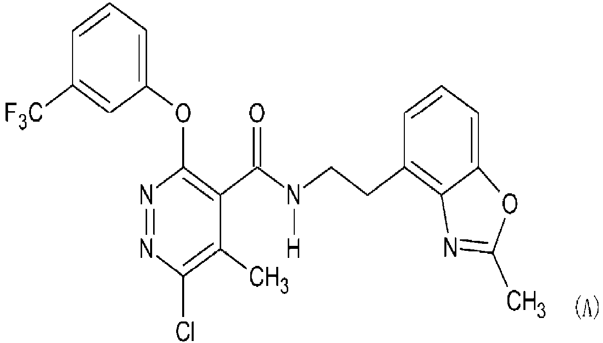

Patent Document 2 discloses a compound represented by Formula (B) or the like.

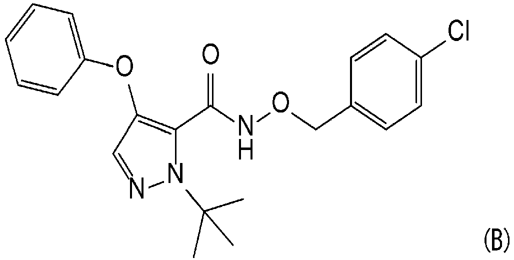

### Citation List

### Patent Documents

Patent Document 1: Published Japanese Translation No. 2022-508277 of the PCT International Publication
Patent Document 2: PCT International Publication No. WO2024/204842

### SUMMARY OF INVENTION

### Technical Problem

An object of the present invention is to provide a 5-membered heteroaryl compound having excellent fungicidal activity against pathogenic fungi affecting agricultural and horticultural plants, having excellent safety, and being industrially advantageously synthesizable, and to provide an agricultural and horticultural fungicide containing the 5-membered heteroaryl compound as an active ingredient.

### Solution to Problem

As a result of intensive studies to achieve the above-described object, the present invention has been completed to include the following aspects.
(1) A compound represented by Formula (Z-I) or a salt thereof. 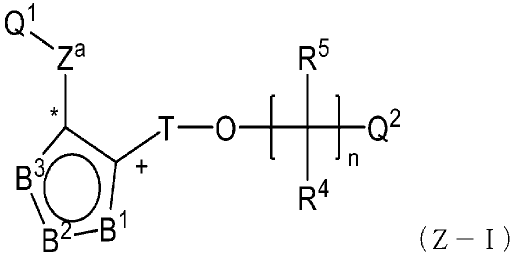 [in Formula (Z-I),
   Q¹ represents a substituted or unsubstituted phenyl group, a substituted or unsubstituted naphthyl group, a substituted or unsubstituted 5- or 6-membered heterocyclyl group, or a substituted or unsubstituted 9- or 10-membered heterocyclyl group,
   Q² represents a substituted or unsubstituted phenyl group, a substituted or unsubstituted naphthyl group, a substituted or unsubstituted 5- or 6-membered heterocyclyl group, or a substituted or unsubstituted 9- or 10-membered heterocyclyl group,
   in a partial structure of Formula (III), 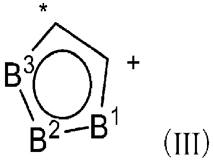
   B¹ represents an oxygen atom, a nitrogen atom, a group represented by a formula: NR¹, or a group represented by a formula: CR²,
   B² represents an oxygen atom, a nitrogen atom, a group represented by a formula: NR¹, or a group represented by a formula: CR²,
   B³ represents an oxygen atom, a nitrogen atom, a group represented by a formula: NR¹, or a group represented by a formula: CR², any one of B¹, B², and B³ represents an oxygen atom or a group represented by the formula: NR¹, and the remaining two each represent a nitrogen atom or a group represented by the formula: CR²,
   R¹'s each independently represent a substituted or unsubstituted C1-6 alkyl group or a substituted or unsubstituted C3-6 cycloalkyl group,
   R²'s each independently represent a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted 5- or 6-membered heterocyclyl group, a substituted or unsubstituted phenyloxy group, a substituted or unsubstituted naphthyloxy group, a substituted or unsubstituted 5- or 6-membered heterocyclyloxy group, a substituted or unsubstituted amino group, a substituted or unsubstituted aminocarbonyl group, a nitro group, a cyano group, or a halogeno group,
   a carbon atom marked with * in the partial structure corresponds to a carbon atom marked with * in Formula (Z-I), and a carbon atom marked with + in the partial structure corresponds to a carbon atom marked with + in Formula (Z-I),
   Z^{a} represents an oxygen atom or a sulfur atom,
   T represents a partial structure (T-1) or a partial structure (T-2), 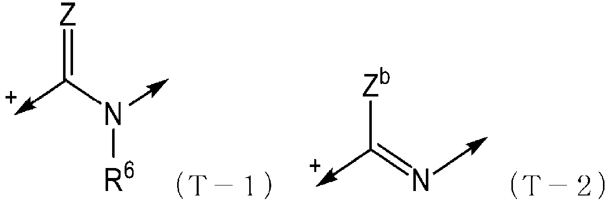
   in the partial structure (T-1) or the partial structure (T-2), an arrow marked with + bonds to the carbon atom marked with + in the partial structure of Formula (III), and the other arrow bonds to an oxygen atom in Formula (Z-I),
   in the partial structure (T-1), Z represents an oxygen atom or a sulfur atom,
   R⁶ represents a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted 5- or 6-membered heterocyclyl group,
   in the partial structure (T-2), Z^{b} represents a C1-6 alkoxy group or a C1-6 alkylthio group,
   R⁴'s each independently represent a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted 5- or 6-membered heterocyclyl group, or a halogeno group,
   R⁵'s each independently represent a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted 5- or 6-membered heterocyclyl group, or a halogeno group, and R⁴ and R⁵ on the same carbon atom may together form a divalent organic group, and
   n represents any integer of 1 to 3 ]
(2) The compound or a salt thereof according to (1), in which Formula (Z-I) is represented by Formula (I). 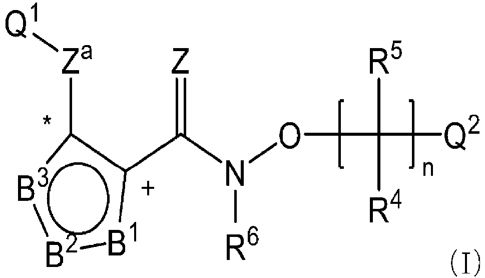 [in Formula (I),
   Q¹, Q², B¹, B², B³, Z^{a}, R⁴, R⁵, and n have the same meanings as Q¹, Q², B¹, B², B³, Z^{a}, R⁴, R⁵, and n in Formula (Z-I), and
   Z and R⁶ have the same meanings as Z and R⁶ in the partial structure (T-1) ]
(3) The compound or a salt thereof according to (2),
   in which B¹ is a group represented by the formula: NR¹,
   B² is a nitrogen atom,
   B³ is a nitrogen atom,
   Z^{a} is an oxygen atom,
   Z is an oxygen atom,
   R⁶ is a hydrogen atom, and
   n is 1.
(4) An agricultural and horticultural fungicide containing, as an active ingredient: at least one selected from the group consisting of the compound according to any one of (1) to (3) and a salt thereof.
(5) The agricultural and horticultural fungicide according to (4), in which the agricultural and horticultural fungicide is for seed treatment.
(6) The agricultural and horticultural fungicide according to (4) or (5), further containing: another active ingredient.
(7) The agricultural and horticultural fungicide according to (6), in which the other active ingredient is at least one selected from the group consisting of the following compounds:
   a nucleic acid biosynthesis inhibitor, a mitosis inhibitor, a cell division inhibitor, a respiration inhibitor, an amino acid or protein synthesis inhibitor, a signal transduction inhibitor, a lipid or cell membrane synthesis inhibitor, a sterol biosynthesis inhibitor in cell membranes, a cell wall synthesis inhibitor, a melanin biosynthesis inhibitor, a host plant resistance inducer, and an agent having multiple points of action; and
   an acetylcholinesterase inhibitor, a GABA-gated chloride ion channel blocker, a sodium channel modulator, a nicotinic acetylcholine receptor competitive modulator, a nicotinic acetylcholine receptor allosteric modulator, a glutamate-gated chloride ion channel allosteric modulator, a juvenile hormone mimic, another non-specific inhibitor, a chordotonal organ TRPV channel modulator, a mite growth inhibitor, a microbially derived disruptor of insect midgut membranes, a mitochondrial ATP synthase inhibitor, an oxidative phosphorylation uncoupler that disrupts the proton gradient, a nicotinic acetylcholine receptor channel blocker, a chitin biosynthesis inhibitor type 0, a chitin biosynthesis inhibitor type 1, a molting inhibitor, an ecdysone receptor agonist, an octopamine receptor agonist, a mitochondrial electron transport chain complex III inhibitor, a mitochondrial electron transport chain complex I inhibitor, a voltagedependent sodium channel blocker, an acetyl-CoA carboxylase inhibitor, a mitochondrial electron transport chain complex IV inhibitor, a mitochondrial electron transport chain complex II inhibitor, a ryanodine receptor modulator, a chordotonal organ modulator with an unspecified target site, and a GABA-gated chloride ion channel allosteric modulator.
(8) The agricultural and horticultural fungicide according to (6), in which the other active ingredient is at least one selected from the group consisting of the following compounds:
   benalaxyl, benalaxyl-M, furalaxyl, metalaxyl, metalaxyl-M, oxadixyl, clozylacon, and ofurace;
   bupirimate, dimethirimol, and ethirimol;
   hymexazol and octhilinone;
   oxolinic acid;
   benomyl, carbendazim, chlorfenazole, fuberidazole, thiabendazole, thiophanate, thiophanate-methyl, diethofencarb, zoxamide, and ethaboxam;
   pencycuron;
   fluopicolide and fluopimomide;
   diflumetorim and tolfenpyrad;
   benodanil, flutolanil, mepronil, isofetamid, fluopyram, fenfuram, flumecyclox, carboxin, oxycarboxin, thifluzamide, benzovindiflupyr, bixafen, fluxapyroxad, furametpyr, isopyrazam, penflufen, penthiopyrad, sedaxane, boscalid, pydiflumetofen, isoflucypram, pyraziflumid, and inpyrfluxam;
   azoxystrobin, coumoxystrobin, coumethoxystrobin, enoxastrobin, flufenoxystrobin, picoxystrobin, pyraoxystrobin, pyraclostrobin, pyrametostrobin, triclopyricarb, kresoxim-methyl, trifloxystrobin, dimoxystrobin, fenaminstrobin, metominostrobin, orysastrobin, famoxadone, fluoxastrobin, fenamidone, pyribencarb, metyltetraprole, and mandestrobin;
   cyazofamid, amisulbrom, and fenpicoxamid;
   binapacryl, meptyldinocap, dinocap, fluazinam, and ferimzone;
   fentin acetate, fentin chloride, and fentin hydroxide;
   silthiofam;
   ametoctradin;
   andoprim, cyprodinil, mepanipyrim, and pyrimethanil;
   blasticidin-S, kasugamycin, kasugamycin hydrochloride, streptomycin, and oxytetracycline;
   quinoxyfen and proquinazid;
   fenpiclonil, fludioxonil, chlozolinate, iprodione, procymidone, and vinclozolin;
   edifenphos, iprobenfos, pyrazophos, and isoprothiolane;
   biphenyl, chloroneb, dicloran, quintozene, tecnazene, tolclofos-methyl, and etridiazole;
   iodocarb, propamocarb, propamocarb hydrochloride, propamocarb-fosetylate, and prothiocarb;
   Bacillus subtilis, Bacillus subtilis strain QST713, Bacillus subtilis strain FZB24, Bacillus subtilis strain MBI600, Bacillus subtilis strain D747, and Bacillus amyloliquefaciens;
   cajeput or tea tree extracts;
   triforine, pyrifenox, pyrisoxazole, fenarimol, flurprimidol, nuarimol, imazalil, imazalil sulfate, oxpoconazole, oxpoconazole fumarate, pefurazoate, prochloraz, triflumizole, viniconazole, azaconazole, bitertanol, bromuconazole, cyproconazole, diclobutrazol, difenoconazole, diniconazole, diniconazole-M, epoxiconazole, etaconazole, fenbuconazole, fluquinconazole, flusilazole, flutriafol, fluconazole, fluconazole-cis, hexaconazole, imibenconazole, ipconazole, metconazole, myclobutanil, penconazole, propiconazole, fluquinconazole, simeconazole, tebuconazole, tetraconazole, triadimefon, triadimenol, triticonazole, prothioconazole, voriconazole, and mefentrifluconazole;
   aldimorph, dodemorph, dodemorph acetate, fenpropimorph, tridemorph, fenpropidin, piperalin, and spiroxamine;
   fenhexamid and fenpyrazamine;
   pyributicarb, naftifine, and terbinafine;
   validamycin;
   polyoxin and polyoxorim;
   dimethomorph, flumorph, pyrimorph, benthiavalicarb, benthiavalicarbisopropyl, iprovalicarb, valifenalate, and mandipropamid;
   fthalide, pyroquilon, and tricyclazole;
   carpropamid, diclocymet, and fenoxanil;
   tolprocarb;
   acibenzolar-S-methyl;
   probenazole, tiadinil, isotianil, diclobentiazox, ipfentrifluconazole, laminarin, giant knotweed extract, phosphorous acid, and phosphites;
   cymoxanil, fosetyl-aluminum, phosphoric acid, phosphates, tecloftalam, triazoxide, flusulfamide, diclomezine, methasulfocarb, cyflufenamid, metrafenone, pyriofenone, dodine, dodine free base, and flutianil;
   copper or copper salts, Bordeaux mixture, copper hydroxide, copper naphthenate, copper oxide, copper oxychloride, copper sulfate, sulfur, sulfur products, calcium polysulfide, ferbam, mancozeb, maneb, mancopper, metiram, polycarbamate, propineb, thiram, zineb, ziram, captan, captafol, folpet, chlorothalonil, dichlofluanid, tolylfluanid, guazatine, guazatine triacetate or iminoctadine triacetate, iminoctadine tris(albesilate), anilazine, dithianon, quinomethionate, and fluoroimide;
   DBEDC, fluorofolpet, guazatine acetate, bis(8-quinolinolato)copper(II), propamidine, chloropicrin, cyproflam, Agrobacterium, bethoxazin, diphenylamine, methyl isothiocyanate or MITC, mildiomycin, capsaicin, cufraneb, cyprosulfamide, dazomet, debacarb, dichlorophen, difenzoquat, difenzoquat methylsulfonate, flumetover, fosetyl-calcium, fosetyl-sodium, irumamycin, natamycin, nitrothal-isopropyl, oxamocarb, pyrrolnitrin, tebufloquin, tolnifanide, zarilamide, Algophase, amicarthiazol, oxathiapiprolin, fluoxapiprolin, zinc metiram, benthiazole, triclamide, uniconazole, oxyfenthiin, picarbutrazox, diclobentiazox, quinofumelin, thiuram, ambam, Agrobacterium radiobacter, Coniothyrium minitans, Pseudomonas fluorescens, Pseudomonas rhodesia, Talaromyces flavus, Trichoderma atroviride, non-pathogenic Erwinia carotovora, Bacillus simplex, Variovorax paradoxus, Lactobacillus plantarum, florylpicoxamid, pyrapropoyne, fluindapyr, aminopyrifen, pyridachlometyl, ipflufenoquin, and dipymetitrone;
   flufenoxadiazam, flumetylsulforim, and feneptamidquin;
   alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC, xylylcarb, phenothiocarb, MIPC, MPMC, MTMC, aldoxycarb, alixycarb, aminocarb, bufencarb, cloethocarb, metam-sodium, and promecarb;
   acephate, azamethiphos, azinphos-ethyl, azinphos-methyl, cadusafos, chlorethoxyphos, chlorfenvinphos, chlormephos, chlorpyrifos, chlorpyrifos-methyl, coumaphos, cyanophos, demeton-S-methyl, diazinon, dichlorvos, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isocarbophos, isopropyl=O-(methoxyaminothiophosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion, bromophos-ethyl, BRP, carbophenothion, cyanofenphos, CYAP, demeton-S-methylsulfone, dialifos, dichlofenthion, dioxabenzofos, etrimfos, fensulfothion, flupyrazofos, fonofos, formothion, phosmethylan, isazofos, iodofenphos, metacrifos, pirimiphos-ethyl, phosphocarb, propaphos, prothoate, and sulprofos;
   acetoprole, chlordane, endosulfan, ethiprole, fipronil, pyrafluprole, pyriprole, camphechlor, heptachlor, dienochlor, and flufiprole;
   acrinathrin, allethrin, d-cis-trans-allethrin, d-trans-allethrin, bifenthrin, bioallethrin, bioallethrin-S-cyclopentenyl isomer, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zetacypermethrin, cyphenothrin [(1R)-trans isomer], deltamethrin, empenthrin [(EZ)-(1R)-isomer], esfenvalerate, etofenprox, fenpropathrin, fenvalerate, flucythrinate, flumethrin, tau-fluvalinate, halfenprox, imiprothrin, kadethrin, permethrin, phenothrin [(1R)-trans isomer], prallethrin, pyrethlam, pyrethrin, resmethrin, silafluofen, tefluthrin, tetramethrin, tetramethrin [(1R)-isomer], tralomethrin, transfluthrin, allethrin, pyrethrin I, pyrethrin II, profluthrin, dimefluthrin, bioethanomethrin, biopermethrin, transpermethrin, fenfluthrin, fenpyrithrin, flubrocythrinate, flufenprox, metofluthrin, protriphenbut, pyresmethrin, terallethrin, kappa-bifenthrin, chloroprallethrin, heptafluthrin, meperfluthrin, epsilonmetofluthrin, momfluorothrin, epsilon-momfluorothrin, kappa-tefluthrin, tetramethylfluthrin, and bioethanomethrin;
   DDT and methoxychlor;
   acetamiprid, clothianidin, dinotefuran, imidacloprid, nitenpyram, nithiazine, thiacloprid, thiamethoxam, nicotine, sulfoxaflor, flupyradifurone, triflumezopyrim, dichloromezotiaz, and flupyrimin;
   spinetoram and spinosad;
   abamectin, emamectin, emamectin benzoate, lepimectin, milbemectin, ivermectin, selamectin, doramectin, eprinomectin, ivermectin, moxidectin, selamectin, milbemycin, milbemycin oxime, and nemadectin;
   hydroprene, kinoprene, methoprene, fenoxycarb, pyriproxyfen, diofenolan, epofenonane, and triprene;
   methyl bromide, alkyl halides, chloropicrin, sodium aluminum fluoride, sulfuryl fluoride, borax, boric acid, disodium octaborate, sodium borate, sodium metaborate, tartar emetic, dazomet, metam, metam-potassium, and metam-sodium;
   flonicamid, pymetrozine, pyrifluquinazon, and afidopyropen;
   clofentezine, diflovidazin, hexythiazox, and etoxazole;
   Bacillus thuringiensis subsp. israelensis, Bacillus sphaericus, Bacillus thuringiensis subsp. aizawai, Bacillus thuringiensis subsp. kurstaki, Bacillus thuringiensis subsp. tenebrionis, Cry1Ab, Cry1Ac, Cry1Fa, Cry1A.105, Cry2Ab, Vip3A, mCry3A, Cry3Ab, Cry3Bb, Cry34Ab1/Cry35Ab1, and Bacillus sphaericus;
   diafenthiuron, azocyclotin, cyhexatin, fenbutatin oxide, propargite, and tetradifon;
   chlorfenapyr, DNOC, sulfluramid, binapacryl, dinobuton, and dinocap;
   bensultap, cartap hydrochloride, nereistoxin, thiocyclam, and thiosultap-sodium;
   bistrifluron, chlorfluazuron, diflubenzuron, flucycloxuron, flufenoxuron, hexaflumuron, lufenuron, novaluron, noviflumuron, teflubenzuron, triflumuron, buprofezin, and fluazuron;
   buprofezin;
   cyromazine;
   chromafenozide, halofenozide, methoxyfenozide, and tebufenozide;
   amitraz, demiditraz, and chlordimeform;
   hydramethylnon, acequinocyl, fluacrypyrim, and bifenazate;
   fenazaquin, fenpyroximate, pyridaben, pyrimidifen, tebufenpyrad, tolfenpyrad, and rotenone;
   indoxacarb and metaflumizone;
   spirodiclofen, spiromesifen, spirotetramat, and spiropidion;
   aluminum phosphide, calcium phosphide, zinc phosphide, phosphine, cyanide, calcium cyanide, sodium cyanide, and potassium cyanide;
   cyenopyrafen, cyflumetofen, and pyflubumide;
   chlorantraniliprole, cyantraniliprole, cyclaniliprole, flubendiamide, cyhalodiamide, tetrachlorantraniliprole, and tetraniliprole;
   flonicamid;
   broflanilide, fluxametamide, isocycloseram, afoxolaner, fluralaner, lotilaner, and sarolaner;
   azadirachtin, benzoximate, bifenazate, bromopropylate, chinomethionat, cryolite, dicofol, lime sulfur, mancozeb, pyridalyl, benclothiaz, sulfur, amidoflumet, 1,3-dichloropropene, DCIP, phenisobromolate, benzomate, metaldehyde, chlorobenzilate, clothiazoben, dicyclanil, phenoxacrim, fentrifanil, flubenzimine, flufenazine, gossyplure, japonilure, methoxyadiazone, petroleum or oil, potassium oleate, tetrasul, triarathene, afidopyropen, flufiprole, fluensulfone, meperfluthrin, tetramethylfluthrin, tralopyril, dimefluthrin, methylneodecanamide, fluralaner, afoxolaner, fluxametamide, 5-[5-(3,5-dichlorophenyl)-5-trifluoromethyl-4,5-dihydroisoxazol-3-yl]-2-(1H-1,2,4-triazol-1-yl)benzonitrile, broflanilide, other metadiamides, Steinernema carpocapsae, Steinernema glaseri, Pasteuria penetrans, Paecilomyces tenuipes, Paecilomyces fumosoroseus, Beauveria bassiana, Beauveria brongniartii, Metarhizium anisopliae, Verticillium lecanii, acynonapyr, benzpyrimoxan, flometoquin, fluhexafon, oxazosulfyl, and tyclopyrazoflor;
   fenbendazole, albendazole, triclabendazole, oxybendazole, mebendazole, oxfendazole, parbendazole, flubendazole, febantel, netobimin, thiophanate, thiabendazole, and cambendazole;
   closantel, oxyclozanide, rafoxanide, and niclosamide;
   nitroxynil and nitroscanate;
   pyrantel and morantel;
   levamisole and tetramisole;
   praziquantel and epsiprantel;
   cyclodiene, ryania, clorsulon, metronidazole, demiditraz, piperazine, diethylcarbamazine, dichlorophen, monepantel, tribendimidine, amidantel, thiacetarsamide, melarsomine, and arsenamide; and
   abscisic acid, kinetin, benzylaminopurine, 1,3-diphenylurea, forchlorfenuron, thidiazuron, chlorfenuron, dihydrozeatin, gibberellin A, gibberellin A4, gibberellin A7, gibberellin A3, 1-methylcyclopropene, N-acetylaminoethoxyvinylglycine or aviglycine, aminooxyacetic acid, silver nitrate, cobalt chloride, IAA, 4-CPA, cloprop, 2,4-D, MCPB, indole-3-butyric acid, dichlorprop, phenothiol, 1-naphthylacetamide, ethychlozate, cloxyfonac, maleic hydrazide, 2,3,5-triiodobenzoic acid, salicylic acid, methyl salicylate, (-)-jasmonic acid, methyl jasmonate, (+)-strigol, (+)-deoxystrigol, (+)-orobanchol, (+)-sorgolactone, 4-oxo-4-(2-phenylethyl)aminobutyric acid, ethephon, chlormequat, mepiquat chloride, benzyladenine, 5-aminolevulinic acid, and daminozide.
(9) A production method for a compound represented by Formula (I-3), the production method including: a step of reacting a compound represented by Formula (im-5) with a compound represented by Formula (im-6) or Formula (im-6-2) in the presence of a condensing agent when U¹ in Formula (im-5) is a hydroxyl group, or in the presence of an acid scavenger when U¹ is a halogeno group. 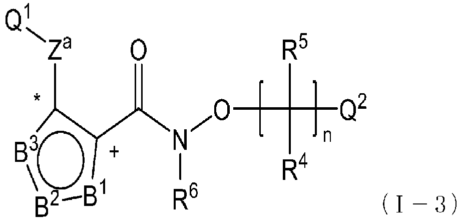 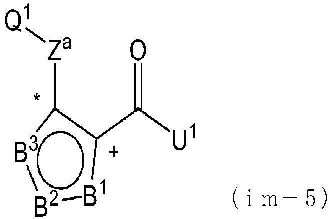 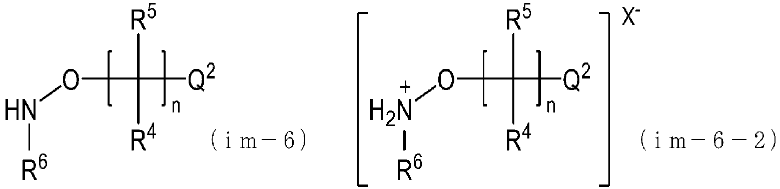 [in the formulae,
   Q¹ represents a substituted or unsubstituted phenyl group, a substituted or unsubstituted naphthyl group, a substituted or unsubstituted 5- or 6-membered heterocyclyl group, or a substituted or unsubstituted 9- or 10-membered heterocyclyl group,
   Q² represents a substituted or unsubstituted phenyl group, a substituted or unsubstituted naphthyl group, a substituted or unsubstituted 5- or 6-membered heterocyclyl group, or a substituted or unsubstituted 9- or 10-membered heterocyclyl group,
   in the following partial structure, 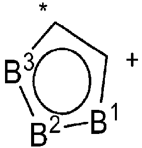
   B¹ represents an oxygen atom, a nitrogen atom, a group represented by a formula: NR¹, or a group represented by a formula: CR²,
   B² represents an oxygen atom, a nitrogen atom, a group represented by a formula: NR¹, or a group represented by a formula: CR²,
   B³ represents an oxygen atom, a nitrogen atom, a group represented by a formula: NR¹, or a group represented by a formula: CR²,
   any one of B¹, B², and B³ represents an oxygen atom or a group represented by the formula: NR¹, and the remaining two each represent a nitrogen atom or a group represented by the formula: CR²,
   R¹'s each independently represent a substituted or unsubstituted C1-6 alkyl group or a substituted or unsubstituted C3-6 cycloalkyl group,
   R²'s each independently represent a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted 5- or 6-membered heterocyclyl group, a substituted or unsubstituted phenyloxy group, a substituted or unsubstituted naphthyloxy group, a substituted or unsubstituted 5- or 6-membered heterocyclyloxy group, a substituted or unsubstituted amino group, a substituted or unsubstituted aminocarbonyl group, a nitro group, a cyano group, or a halogeno group,
   a carbon atom marked with * in the partial structure corresponds to a carbon atom marked with * in Formula (I-3) or Formula (im-5), and a carbon atom marked with + in the partial structure corresponds to a carbon atom marked with + in Formula (I-3) or Formula (im-5),
   Z^{a} represents an oxygen atom or a sulfur atom,
   R⁴'s each independently represent a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted 5- or 6-membered heterocyclyl group, or a halogeno group,
   R⁵'s each independently represent a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted 5- or 6-membered heterocyclyl group, or a halogeno group,
   R⁴ and R⁵ on the same carbon atom may together form a divalent organic group,
   n represents any integer of 1 to 3,
   R⁶ represents a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted 5- or 6-membered heterocyclyl group,
   X⁻ represents a counter anion, and
   U¹ represents a hydroxyl group or a halogeno group ]
(10) A compound represented by Formula (im-5B) or a salt thereof. 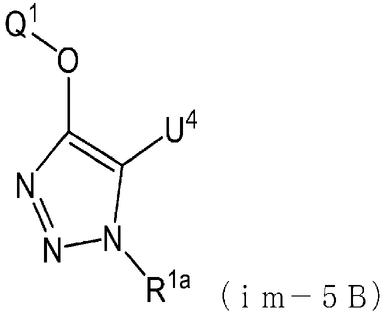 [in Formula (im-5B),
   Q¹ represents a substituted or unsubstituted phenyl group, a substituted or unsubstituted naphthyl group, a substituted or unsubstituted 5- or 6-membered heterocyclyl group, or a substituted or unsubstituted 9- or 10-membered heterocyclyl group,
   R^{1a} represents a substituted or unsubstituted C2-6 alkyl group or a substituted or unsubstituted C3-6 cycloalkyl group, and
   U⁴ represents a carboxyl group, a C1-6 alkoxycarbonyl group, or a halogenocarbonyl group ]
(11) A compound represented by Formula (im-25) or a salt thereof. 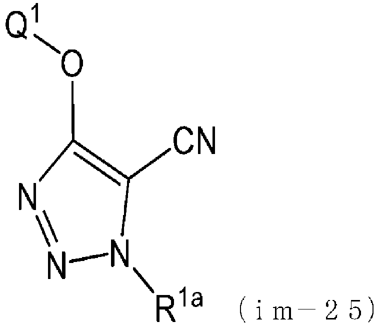 In Formula (im-25),
   Q¹ represents a substituted or unsubstituted phenyl group, a substituted or unsubstituted naphthyl group, a substituted or unsubstituted 5- or 6-membered heterocyclyl group, or a substituted or unsubstituted 9- or 10-membered heterocyclyl group, and
   R^{1a} represents a substituted or unsubstituted C2-6 alkyl group or a substituted or unsubstituted C3-6 cycloalkyl group.

### Advantageous Effects of Invention

The 5-membered heteroaryl compound according to the aspect of the present invention has excellent fungicidal activity against pathogenic fungi affecting agricultural and horticultural plants, has excellent safety, and can be industrially advantageously synthesized. The agricultural and horticultural fungicide according to the aspect of the present invention can effectively control diseases of agricultural and horticultural plants.

### DESCRIPTION OF EMBODIMENTS

The 5-membered heteroaryl compound according to the embodiment of the present invention is a compound represented by Formula (Z-I) (which may hereinafter be referred to as "compound (Z-I)") or a salt of the compound (Z-I).

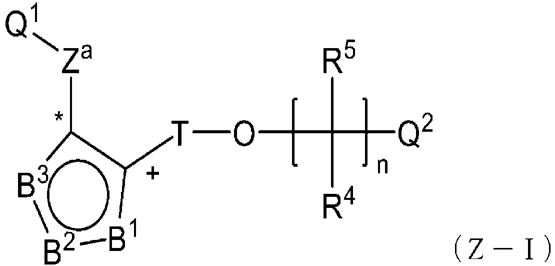

In the present invention, the term "unsubstituted" means that only a group serving as a mother nucleus is present. In a case where the group serving as a mother nucleus is described only with the name without a description of "substituted", it means "unsubstituted" unless otherwise specified.

On the other hand, the term "substituted" means that any hydrogen atom in the group serving as a mother nucleus is substituted with a group (a substituent) having a structure that is the same as or different from the structure of the mother nucleus. Accordingly, the "substituent" is another group which is bonded to the group serving as a mother nucleus. The number of substituents may be one, or two or more. The two or more substituents may be the same as or different from each other.

The term "C1-6" or the like represents that the number of carbon atoms in the group serving as a mother nucleus is 1 to 6. The number of carbon atoms in the substituent is not included in the number of carbon atoms. For example, a butyl group having an ethoxy group as a substituent is classified as a C2 alkoxy C4 alkyl group.

The "substituent" is not particularly limited as long as it is chemically acceptable and has the effect of the present invention.

Examples of groups that can serve as the "substituent" include the following.
C1-6 alkyl groups such as a methyl group, an ethyl group, an n-propyl group, an i-propyl group, an n-butyl group, an s-butyl group, an i-butyl group, a t-butyl group, an n-pentyl group, and an n-hexyl group;
C2-6 alkenyl groups such as a vinyl group, a 1-propenyl group, a 2-propenyl group (allyl group), a 1-butenyl group, a 2-butenyl group, a 3-butenyl group, a 1-methyl-2-propenyl group, and a 2-methyl-2-propenyl group;
C2-6 alkynyl groups such as an ethynyl group, a 1-propynyl group, a 2-propynyl group, a 1-butynyl group, a 2-butynyl group, a 3-butynyl group, and a 1-methyl-2-propynyl group;
C3-6 cycloalkyl groups such as a cyclopropyl group, a cyclobutyl group, a cyclopentyl group, and a cyclohexyl group;
aryl groups such as a phenyl group, a tolyl group, a xylyl group, a trimethylphenyl group, and a naphthyl group;
phenyl C1-6 alkyl groups such as a benzyl group and a phenethyl group;
3- to 6-membered heterocyclyl groups;
3- to 6-membered heterocyclyl C1-6 alkyl groups;
a hydroxyl group;
C1-6 alkoxy groups such as a methoxy group, an ethoxy group, an n-propoxy group, an i-propoxy group, an n-butoxy group, an s-butoxy group, an i-butoxy group, and a t-butoxy group;
C2-6 alkenyloxy groups such as a vinyloxy group, an allyloxy group, a propenyloxy group, and a butenyloxy group;
C2-6 alkynyloxy groups such as an ethynyloxy group and a propargyloxy group;
a phenoxy group and a naphthoxy group;
phenyl C1-6 alkoxy groups such as a benzyloxy group and a phenethyloxy group;
5- or 6-membered heteroaryloxy groups such as a thiazolyloxy group and a pyridyloxy group;
5- or 6-membered heteroaryl C1-6 alkyloxy groups such as a thiazolylmethyloxy group and a pyridylmethyloxy group;
a formyl group;
C1-6 alkylcarbonyl groups such as an acetyl group and a propionyl group;
a formyloxy group;
C1-6 alkylcarbonyloxy groups such as an acetyloxy group and a propionyloxy group;
a benzoyl group;
C1-6 alkoxycarbonyl groups such as a methoxycarbonyl group, an ethoxycarbonyl group, an n-propoxycarbonyl group, an i-propoxycarbonyl group, an n-butoxycarbonyl group, and a t-butoxycarbonyl group;
C1-6 alkoxycarbonyloxy groups such as a methoxycarbonyloxy group, an ethoxycarbonyloxy group, an n-propoxycarbonyloxy group, an i-propoxycarbonyloxy group, an n-butoxycarbonyloxy group, and a t-butoxycarbonyloxy group;
a carboxyl group;
halogeno groups such as a fluoro group, a chloro group, a bromo group, and an iodo group;
C1-6 haloalkyl groups such as a chloromethyl group, a chloroethyl group, a trifluoromethyl group, a 1,2-dichloro-n-propyl group, a 1-fluoro-n-butyl group, and a perfluoro-n-pentyl group;
C2-6 haloalkenyl groups such as a 2-chloro-1-propenyl group and a 2-fluoro-1-butenyl group;
C2-6 haloalkynyl groups such as a 4,4-dichloro-1-butynyl group, a 4-fluoro-1-pentynyl group, and a 5-bromo-2-pentynyl group;
C1-6 haloalkoxy groups such as a trifluoromethoxy group, a 2-chloro-n-propoxy group, and a 2,3-dichlorobutoxy group;
C2-6 haloalkenyloxy groups such as a 2-chloropropenyloxy group and a 3-bromobutenyloxy group;
C1-6 haloalkylcarbonyl groups such as a chloroacetyl group, a trifluoroacetyl group, and a trichloroacetyl group;
an amino group;
C1-6 alkyl-substituted amino groups such as a methylamino group, a dimethylamino group, and a diethylamino group;
an anilino group and a naphthylamino group;
phenyl C1-6 alkylamino groups such as a benzylamino group and a phenethylamino group;
a formylamino group;
C1-6 alkylcarbonylamino groups such as an acetylamino group, a propanoylamino group, a butyrylamino group, and an i-propylcarbonylamino group;
C1-6 alkoxycarbonylamino groups such as a methoxycarbonylamino group, an ethoxycarbonylamino group, an n-propoxycarbonylamino group, and an i-propoxycarbonylamino group;
unsubstituted or substituted aminocarbonyl groups such as an aminocarbonyl group, a dimethylaminocarbonyl group, a phenylaminocarbonyl group, and an N-phenyl-N-methylaminocarbonyl group;
imino C1-6 alkyl groups such as an iminomethyl group, a (1-imino)ethyl group, and a (1-imino)-n-propyl group;
substituted or unsubstituted N-hydroxyimino C1-6 alkyl groups such as an N-hydroxyiminomethyl group, a (1-(N-hydroxy)imino)ethyl group, a (1-(N-hydroxy)imino)propyl group, an N-methoxyiminomethyl group, and a (1-(N-methoxy)imino)ethyl group;
an aminocarbonyloxy group;
C1-6 alkyl-substituted aminocarbonyloxy groups such as an ethylaminocarbonyloxy group and a dimethylaminocarbonyloxy group;
a mercapto group;
C1-6 alkylthio groups such as a methylthio group, an ethylthio group, an n-propylthio group, an i-propylthio group, an n-butylthio group, an i-butylthio group, an s-butylthio group, and a t-butylthio group;
C1-6 haloalkylthio groups such as a trifluoromethylthio group and a 2,2,2-trifluoroethylthio group;
a phenylthio group and a naphthylthio group;
5- or 6-membered heteroarylthio groups such as a thiazolylthio group and a pyridylthio group;
C1-6 alkylsulfinyl groups such as a methylsulfinyl group, an ethylsulfinyl group, and a t-butylsulfinyl group;
C1-6 haloalkylsulfinyl groups such as a trifluoromethylsulfinyl group and a 2,2,2-trifluoroethylsulfinyl group;
a phenylsulfinyl group;
5- or 6-membered heteroarylsulfinyl groups such as a thiazolylsulfinyl group and a pyridylsulfinyl group;
C1-6 alkylsulfonyl groups such as a methylsulfonyl group, an ethylsulfonyl group, and a t-butylsulfonyl group;
C1-6 haloalkylsulfonyl groups such as a trifluoromethylsulfonyl group and a 2,2,2-trifluoroethylsulfonyl group;
a phenylsulfonyl group;
5- or 6-membered heteroarylsulfonyl groups such as a thiazolylsulfonyl group and a pyridylsulfonyl group;
C1-6 alkylsulfonyloxy groups such as a methylsulfonyloxy group, an ethylsulfonyloxy group, and a t-butylsulfonyloxy group;
C1-6 haloalkylsulfonyloxy groups such as a trifluoromethylsulfonyloxy group and a 2,2,2-trifluoroethylsulfonyloxy group;
tri(C1-6 alkyl)-substituted silyl groups such as a trimethylsilyl group, a triethylsilyl group, and a t-butyldimethylsilyl group;
a triphenylsilyl group;
a cyano group; and a nitro group.

In addition, in these "substituents", any hydrogen atom in the above-described substituent may be substituted by a group having a different structure. Examples of the "substituent" in this case include a C1-6 alkyl group, a C1-6 haloalkyl group, a C1-6 alkoxy group, a C1-6 haloalkoxy group, a halogeno group, a cyano group, and a nitro group.

In addition, the above-described "3- to 6-membered heterocyclyl group" contains, as a ring-constituting atom, 1 to 4 heteroatoms selected from the group consisting of a nitrogen atom, an oxygen atom, and a sulfur atom. The heterocyclyl group may be monocyclic or polycyclic. In a polycyclic heterocyclyl group, as long as at least one ring is a heterocycle, the remaining rings may each be a saturated alicyclic ring, an unsaturated alicyclic ring, or an aromatic ring. Examples of the "3- to 6-membered heterocyclyl group" include a 3- to 6-membered saturated heterocyclyl group, a 5- or 6-membered heteroaryl group, and a 5- or 6-membered partially unsaturated heterocyclyl group.

Examples of the "3- to 6-membered saturated heterocyclyl group" include an aziridinyl group, an epoxy group, an azetidinyl group, an oxetanyl group, a pyrrolidinyl group, a tetrahydrofuranyl group, a thiazolidinyl group, a piperidyl group, a piperazinyl group, a morpholinyl group, a dioxolanyl group, and a dioxanyl group.

Examples of the "5-membered heteroaryl group" include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group.

Examples of the "6-membered heteroaryl group" include a pyridyl group, a pyrazinyl group, a pyrimidinyl group, a pyridazinyl group, and a triazinyl group.

Examples of the "5- or 6-membered partially unsaturated heterocyclyl group" include 5-membered partially unsaturated heterocyclyl groups such as a pyrrolinyl group, a dihydrofuranyl group, a dihydrothiophenyl group, an imidazolinyl group, a pyrazolinyl group, an oxazolinyl group, an isoxazolinyl group, a thiazolinyl group, and an isothiazolinyl group; and 6-membered partially unsaturated heterocyclyl groups such as a dihydropyranyl group.

Q¹ in Formula (Z-I) represents a substituted or unsubstituted phenyl group, a substituted or unsubstituted naphthyl group, a substituted or unsubstituted 5- or 6-membered heterocyclyl group, or a substituted or unsubstituted 9- or 10-membered heterocyclyl group.

Q² in Formula (Z-I) represents a substituted or unsubstituted phenyl group, a substituted or unsubstituted naphthyl group, a substituted or unsubstituted 5- or 6-membered heterocyclyl group, or a substituted or unsubstituted 9- or 10-membered heterocyclyl group.

The heterocyclyl group contains, as a ring-constituting atom, at least one heteroatom selected from the group consisting of a nitrogen atom, an oxygen atom, and a sulfur atom. The heterocyclyl group may be monocyclic or polycyclic. In a polycyclic heterocyclyl group, as long as at least one ring is a heterocycle, the remaining rings may each be a saturated alicyclic ring, an unsaturated alicyclic ring, or an aromatic ring.

Examples of the "5- or 6-membered heterocyclyl group" for Q¹ or Q² include a 5- or 6-membered saturated heterocyclyl group, a 5- or 6-membered heteroaryl group, and a 5- or 6-membered partially unsaturated heterocyclyl group.

Examples of the "5- or 6-membered saturated heterocyclyl group" for Q¹ or Q² include a pyrrolidinyl group, a tetrahydrofuranyl group, a tetrahydropyranyl group, a thiazolidinyl group, a piperidyl group, a piperazinyl group, a morpholinyl group, a dioxolanyl group, and a dioxanyl group.

Examples of the "5-membered heteroaryl group" for Q¹ or Q² include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group.

Examples of the "6-membered heteroaryl group" for Q¹ or Q² include a pyridyl group, a pyrazinyl group, a pyrimidinyl group, a pyridazinyl group, and a triazinyl group.

Examples of the "5- or 6-membered partially unsaturated heterocyclyl group" for Q¹ or Q² include 5-membered partially unsaturated heterocyclyl groups such as a pyrrolinyl group, a dihydrofuranyl group, a dihydrothiophenyl group, an imidazolinyl group, a pyrazolinyl group, an oxazolinyl group, an isoxazolinyl group, a thiazolinyl group, and an isothiazolinyl group; and 6-membered partially unsaturated heterocyclyl groups such as a dihydropyranyl group.

Examples of the "9-membered heterocyclyl group" for Q¹ or Q² include an indolyl group, an isoindolyl group, a benzofuranyl group, a benzothienyl group, an indazolyl group, a benzoimidazolyl group, a benzoxazolyl group, a benzoisoxazolyl group, a benzothiazolyl group, a benzoisothiazolyl group, and a benzodioxolanyl group.

Examples of the "10-membered heterocyclyl group" for Q¹ or Q² include a quinolinyl group, an isoquinolinyl group, a cinnolinyl group, a phthalazinyl group, a quinazolinyl group, and a quinoxalinyl group.

The substituent on the "5- or 6-membered heterocyclyl group", "phenyl group", "naphthyl group", or "9- or 10-membered heterocyclyl group" for Q¹ or Q² is preferably a halogeno group, a C1-6 alkyl group, a C2-6 alkenyl group, a C1-6 haloalkyl group, a hydroxyl group, a C1-6 alkoxy group, a C1-6 haloalkoxy group, a C1-6 alkoxy group substituted with a C1-6 alkoxy group, a C1-6 alkylcarbonyl group, a C1-6 alkylthio group, a C1-6 alkylsulfonyl group, a C3-6 cycloalkyl group, a phenyl group, a phenyloxy group, a 5-membered heteroaryl group, a 6-membered heteroaryl group, a cyano group, a nitro group, a tri(C1-6 alkyl)silyl group; an amino group substituted with a C1-6 alkyl group or a C1-6 alkylcarbonyl group; a carboxamide group substituted with a C1-6 alkyl group; a phenyl group substituted with any one or more substituents of a C1-6 alkyl group, a C1-6 alkoxy group, a halogeno group, a C1-6 haloalkyl group, and a C1-6 haloalkoxy group; a 5-membered heteroaryl group substituted with any one or more substituents of a C1-6 alkyl group, a C1-6 alkoxy group, a halogeno group, a C1-6 haloalkyl group, and a C1-6 haloalkoxy group; or a 6-membered heteroaryl group substituted with any one or more substituents of a C1-6 alkyl group, a C1-6 alkoxy group, a halogeno group, a C1-6 haloalkyl group, and a C1-6 haloalkoxy group.

Two substituents on the "5- or 6-membered heterocyclyl group", "phenyl group", "naphthyl group", or "9- or 10-membered heterocyclyl group" for Q¹ or Q² may together form a divalent organic group; or two substituents in a phenyl group substituted with two substituents, a 5-membered heteroaryl group substituted with two substituents, or a 6-membered heteroaryl group substituted with two substituents may together form a divalent organic group. Examples of the divalent organic group include C3-6 alkylene groups such as a trimethylene group and a tetramethylene group; and C1-3 alkylenedioxy groups such as a methylenedioxy group and a dimethylenedioxy group.

For example, it is preferable that two substituents on a phenyl group together form a trimethylene group, thereby forming an indanyl group as Q¹ or Q².

Among the above, Q¹ in Formula (Z-I) preferably represents a substituted or unsubstituted phenyl group, a substituted or unsubstituted naphthyl group, a substituted or unsubstituted 6-membered heterocyclyl group, or a substituted or unsubstituted 9-membered heterocyclyl group; more preferably represents a substituted or unsubstituted phenyl group, a substituted or unsubstituted naphthyl group, a substituted or unsubstituted pyridyl group, or a substituted or unsubstituted benzodioxolanyl group; and still more preferably represents a substituted or unsubstituted phenyl group.

The substituent on the "5- or 6-membered heterocyclyl group", "phenyl group", "naphthyl group", or "9- or 10-membered heterocyclyl group" for Q¹ is more preferably a halogeno group, a C1-6 alkyl group, a C1-6 haloalkyl group, a C1-6 alkoxy group, a C1-6 haloalkoxy group, a C1-6 alkylcarbonyl group, a C1-6 alkylthio group, a C1-6 alkylsulfonyl group, a C3-6 cycloalkyl group, a phenyl group, a phenyloxy group, a cyano group, a nitro group, a tri C1-6 alkylsilyl group, an amino group substituted with a C1-6 alkyl group or a C1-6 alkylcarbonyl group, and/or a carboxamide group substituted with a C1-6 alkyl group; still more preferably a halogeno group, a C1-6 alkyl group, a C1-6 haloalkyl group, a C1-6 alkoxy group, a C1-6 haloalkoxy group, a C1-6 alkylcarbonyl group, a C1-6 alkylthio group, a C3-6 cycloalkyl group, a cyano group, a nitro group, and/or an amino group substituted with a C1-6 alkyl group; and even more preferably a halogeno group, a C1-6 alkyl group, a C1-6 haloalkyl group, a C1-6 alkoxy group, a C1-6 haloalkoxy group, or a cyano group.

Among these, the substituent on the "phenyl group" is more preferably at least one or more of a halogeno group, a C1-6 alkyl group, a C1-6 haloalkyl group, a C1-6 alkoxy group, a C1-6 haloalkoxy group, a C1-6 alkylthio group, a cyano group, and an amino group substituted with a C1-6 alkyl group; still more preferably at least one or more of a halogeno group, a C1-6 alkyl group, a C1-6 haloalkyl group, a C1-6 alkoxy group, a C1-6 alkylthio group, a cyano group, and an amino group substituted with a C1-6 alkyl group; and particularly preferably at least one or more of a halogeno group, a C1-6 alkyl group, a C1-6 haloalkyl group, a C1-6 alkoxy group, and a C1-6 alkylthio group.

The substituent on the "6-membered heterocyclyl group" is more preferably a C1-6 haloalkyl group.

When the "5- or 6-membered heterocyclyl group", "phenyl group", "naphthyl group", or "9- or 10-membered heterocyclyl group" for Q¹ has a substituent, the number of substituents is preferably 1 or 2.

In addition, Q¹ may represent an indanyl group formed by two substituents on a phenyl group together forming a trimethylene group.

Q² in Formula (Z-I) more preferably represents a substituted or unsubstituted phenyl group, a substituted or unsubstituted naphthyl group, a substituted or unsubstituted thienyl group, a substituted or unsubstituted thiazolyl group, a substituted or unsubstituted pyridyl group, a substituted or unsubstituted benzodioxolanyl group, or a substituted or unsubstituted quinolinyl group; and still more preferably represents a substituted or unsubstituted phenyl group.

The substituent on the "phenyl group", "naphthyl group", "5- or 6-membered heterocyclyl group", or "9- or 10-membered heterocyclyl group" for Q² is more preferably a halogeno group, a C1-6 alkyl group, a C1-6 haloalkyl group, a C1-6 alkoxy group, a C1-6 alkoxy group substituted with a C1-6 alkoxy group, a C1-6 alkylsulfonyl group, a cyano group, or an amino group substituted with a C1-6 alkyl group.

Among these, the substituent on the "phenyl group" is still more preferably at least one or more of a halogeno group, a C1-6 alkyl group, and a C1-6 alkylsulfonyl group.

When the "phenyl group", "naphthyl group", "5- or 6-membered heterocyclyl group", or "9- or 10-membered heterocyclyl group" for Q² has a substituent, the number of substituents is preferably 1 or 2, and more preferably 2.

In addition, Q² may represent an indanyl group formed by two substituents on a phenyl group together forming a trimethylene group.

Each symbol in a partial structure of Formula (III) has the following meanings.

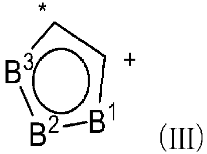

A carbon atom marked with * in the partial structure corresponds to a carbon atom marked with * in Formula (Z-I); and a carbon atom marked with + in the partial structure corresponds to a carbon atom marked with + in Formula (Z-I).

B¹ represents an oxygen atom, a nitrogen atom, a group represented by a formula: NR¹, or a group represented by a formula: CR².

B² represents an oxygen atom, a nitrogen atom, a group represented by a formula: NR¹, or a group represented by a formula: CR².

B³ represents an oxygen atom, a nitrogen atom, a group represented by a formula: NR¹, or a group represented by a formula: CR².

Here, any one of B¹, B², and B³ represents an oxygen atom or a group represented by the formula: NR¹, and the remaining two each represent a nitrogen atom or a group represented by the formula: CR².

Specific examples of the partial structure of Formula (III) include the following.

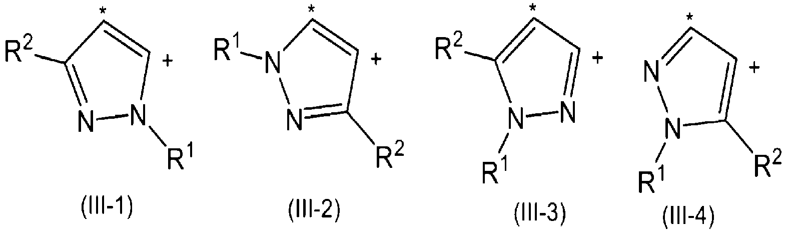

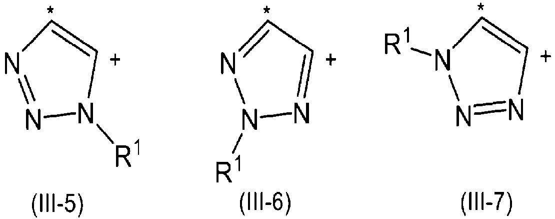

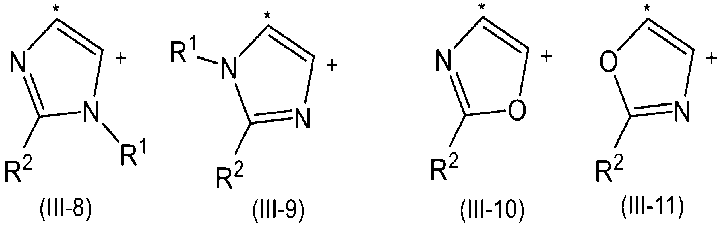

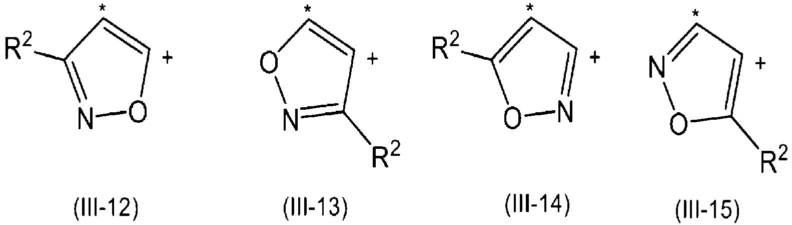

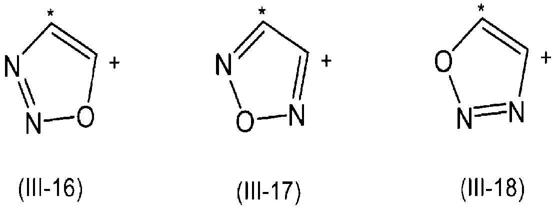

R¹'s each independently represent a substituted or unsubstituted C1-6 alkyl group or a substituted or unsubstituted C3-6 cycloalkyl group.

R²'s each independently represent a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted 5- or 6-membered heterocyclyl group, a substituted or unsubstituted phenyloxy group, a substituted or unsubstituted naphthyloxy group, a substituted or unsubstituted 5- or 6-membered heterocyclyloxy group, a substituted or unsubstituted amino group, a substituted or unsubstituted aminocarbonyl group, a nitro group, a cyano group, or a halogeno group.

The "C1-6 alkyl group" for R¹ and R² may be linear or branched. Examples of the "C1-6 alkyl group" for R¹ include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group, and an i-hexyl group.

Examples of the "C3-6 cycloalkyl group" for R¹ and R² include a cyclopropyl group, a cyclobutyl group, a cyclopentyl group, and a cyclohexyl group.

Examples of the "C2-6 alkenyl group" for R² include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenyl group.

Examples of the "C2-6 alkynyl group" for R² include an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group.

Examples of the "C1-6 alkoxy group" for R² include a methoxy group, an ethoxy group, an n-propoxy group, an i-propoxy group, an n-butoxy group, an s-butoxy group, an i-butoxy group, and a t-butoxy group.

Examples of the "C1-6 alkylcarbonyl group" for R² include an acetyl group, a propionyl group, an n-propylcarbonyl group, an i-propylcarbonyl group, an n-butylcarbonyl group, and a t-butylcarbonyl group.

Examples of the "C1-6 alkoxycarbonyl group" for R² include a methoxycarbonyl group, an ethoxycarbonyl group, an n-propoxycarbonyl group, an i-propoxycarbonyl group, an n-butoxycarbonyl group, and a t-butoxycarbonyl group.

Examples of the "C1-6 alkylcarbonyloxy group" for R² include an acetyloxy group, a propionyloxy group, an n-propylcarbonyloxy group, an i-propylcarbonyloxy group, an n-butylcarbonyloxy group, and a t-butylcarbonyloxy group.

Examples of the "C1-6 alkoxycarbonyloxy group" for R² include a methoxycarbonyloxy group, an ethoxycarbonyloxy group, an n-propoxycarbonyloxy group, an i-propoxycarbonyloxy group, an n-butoxycarbonyloxy group, and a t-butoxycarbonyloxy group.

Examples of the "C1-6 alkylthio group" for R² include a methylthio group, an ethylthio group, an n-propylthio group, an n-butylthio group, an n-pentylthio group, an n-hexylthio group, an i-propylthio group, and an i-butylthio group.

Examples of the "C1-6 alkylsulfinyl group" for R² include a methylsulfinyl group, an ethylsulfinyl group, and a t-butylsulfinyl group.

Examples of the "C1-6 alkylsulfonyl group" for R² include a methylsulfonyl group, an ethylsulfonyl group, and a t-butylsulfonyl group.

The substituent on the "C1-6 alkyl group", "C2-6 alkenyl group", "C2-6 alkynyl group", "C1-6 alkoxy group", "C1-6 alkylcarbonyl group", "C1-6 alkoxycarbonyl group", "C1-6 alkylcarbonyloxy group", "C1-6 alkoxycarbonyloxy group", "C1-6 alkylthio group", "C1-6 alkylsulfinyl group", or "C1-6 alkylsulfonyl group" for R¹ and R² is preferably at least one or more of a halogeno group such as a fluoro group, a chloro group, a bromo group, and an iodo group; a hydroxyl group; a C1-6 alkoxy group such as a methoxy group, an ethoxy group, an n-propoxy group, an i-propoxy group, an n-butoxy group, an s-butoxy group, an i-butoxy group, and a t-butoxy group; a C1-6 haloalkoxy group such as a 2-chloro-n-propoxy group, a 2,3-dichlorobutoxy group, and a trifluoromethoxy group; a C3-6 cycloalkyl group; a phenyl group; a 5-membered heteroaryl group; a 6-membered heteroaryl group; a cyano group;
a phenyl group substituted with at least one or more of substituents of a C1-6 alkyl group, a C1-6 alkoxy group, a halogeno group, a C1-6 haloalkyl group, and a C1-6 haloalkoxy group; a 5-membered heteroaryl group substituted with any one or more substituents of a C1-6 alkyl group, a C1-6 alkoxy group, a halogeno group, a C1-6 haloalkyl group, and a C1-6 haloalkoxy group;
a 6-membered heteroaryl group substituted with any one or more substituents of a C1-6 alkyl group, a C1-6 alkoxy group, a halogeno group, a C1-6 haloalkyl group, or a C1-6 haloalkoxy group; and a halogeno group, a C1-6 alkoxy group, or a C3-6 cycloalkyl group is more preferable.

The "5- or 6-membered heterocyclyl group" for R² is a group containing, as a ring-constituting atom, 1, 2, 3, or 4 heteroatoms selected from the group consisting of a nitrogen atom, an oxygen atom, and a sulfur atom. When there are two or more heteroatoms, they may be the same or different.

Examples of the "5- or 6-membered heterocyclyl group" include a pyrrolidinyl group, a tetrahydrofuranyl group, a thiazolidinyl group, a tetrahydro-2H-pyranyl group, a piperidyl group, a piperazinyl group, a morpholinyl group, a dioxolanyl group, a dioxanyl group, a pyrrolinyl group, a dihydrofuranyl group, an imidazolinyl group, a pyrazolinyl group, an oxazolinyl group, an isoxazolinyl group, and a dihydropyranyl group; and a pyrazolinyl group is preferable.

Examples of the "5- or 6-membered heterocyclyloxy group" for R² include a pyrrolidinyloxy group, a tetrahydrofuranyloxy group, a thiazolidinyloxy group, a tetrahydro-2H-pyranyloxy group, a piperidyloxy group, a piperazinyloxy group, a morpholinyloxy group, a dioxolanyloxy group, a dioxanyloxy group, a pyrrolinyloxy group, a dihydrofuranyloxy group, an imidazolinyloxy group, a pyrazolinyloxy group, an oxazolinyloxy group, an isoxazolinyloxy group, and a dihydropyranyloxy group.

The substituent on the "C3-6 cycloalkyl group", "phenyl group", "naphthyl group", "5- or 6-membered heterocyclyl group", "phenyloxy group", "naphthyloxy group", and "5- or 6-membered heterocyclyloxy group" for R¹ and R² is preferably a halogeno group, a C1-6 alkyl group, a C1-6 haloalkyl group, a hydroxyl group, a C1-6 alkoxy group, a C1-6 haloalkoxy group, a C3-6 cycloalkyl group, a phenyl group, a 5-membered heteroaryl group, a 6-membered heteroaryl group, or a cyano group; a phenyl group substituted with any one or more substituents of a C1-6 alkyl group, a C1-6 alkoxy group, a halogeno group, a C1-6 haloalkyl group, and a C1-6 haloalkoxy group; a 5-membered heteroaryl group substituted with any one or more substituents of a C1-6 alkyl group, a C1-6 alkoxy group, a halogeno group, a C1-6 haloalkyl group, and a C1-6 haloalkoxy group; or a 6-membered heteroaryl group substituted with any one or more substituents of a C1-6 alkyl group, a C1-6 alkoxy group, a halogeno group, a C1-6 haloalkyl group, and a C1-6 haloalkoxy group; and more preferably a halogeno group.

The "amino group" for R² is a group represented by NH₂. In the "substituted amino group", a hydrogen atom in the group represented by NH₂ is substituted by another group.

The "aminocarbonyl group" for R² is a group represented by NH₂CO. In the "substituted aminocarbonyl group", a hydrogen atom in the group represented by NH₂CO is substituted by another group.

The substituent on the "amino group" or "aminocarbonyl group" for R² is preferably a C1-6 alkyl group, a C1-6 haloalkyl group, a C1-6 alkoxycarbonyl group, a C3-6 cycloalkyl group, a phenyl group, a 5-membered heteroaryl group, a 6-membered heteroaryl group; a phenyl group substituted with any one or more substituents of a C1-6 alkyl group, a C1-6 alkoxy group, a halogeno group, a C1-6 haloalkyl group, and a C1-6 haloalkoxy group; a 5-membered heteroaryl group substituted with any one or more substituents of a C1-6 alkyl group, a C1-6 alkoxy group, a halogeno group, a C1-6 haloalkyl group, and a C1-6 haloalkoxy group; or a 6-membered heteroaryl group substituted with any one or more substituents of a C1-6 alkyl group, a C1-6 alkoxy group, a halogeno group, a C1-6 haloalkyl group, and a C1-6 haloalkoxy group; more preferably a C1-6 alkyl group or a C1-6 alkoxycarbonyl group; and still more preferably a C1-6 alkyl group.

Examples of the "halogeno group" for R² include a fluoro group, a chloro group, a bromo group, and an iodo group.

Among the above, R¹'s each independently preferably represent a C1-6 alkyl group; a C1-6 alkyl group substituted with a halogeno group, a C1-6 alkoxy group, or a C3-6 cycloalkyl group; or a C3-6 cycloalkyl group.

R²'s each independently preferably represent a hydrogen atom, a substituted or unsubstituted C1-6 alkyl group, a substituted or unsubstituted C3-6 cycloalkyl group, a cyano group, or a halogeno group; more preferably represent a hydrogen atom, a C1-6 alkyl group, a C3-6 cycloalkyl group, a cyano group, or a halogeno group; and still more preferably represent a hydrogen atom, a cyano group, or a halogeno group.

The above-described partial structure of Formula (III) is preferably the partial structure of Formula (III-5) shown above.

That is, it is preferable that B¹ is a group represented by the formula: NR¹, B² is a nitrogen atom, and B³ is a nitrogen atom.

Z^{a} in Formula (Z-I) represents an oxygen atom or a sulfur atom.

Z^{a} in Formula (Z-I) is preferably an oxygen atom.

T in Formula (Z-I) represents a partial structure (T-1) or a partial structure (T-2).

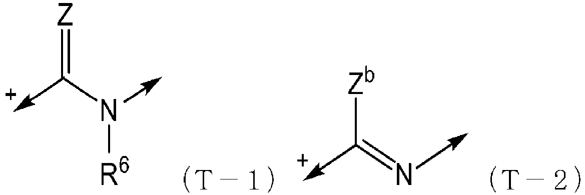

In the above-described partial structures (T-1) and (T-2), an arrow marked with + bonds to the carbon atom marked with + in the partial structure of Formula (III), and the other arrow bonds to the oxygen atom in Formula (Z-I).

Z in the above-described partial structure (T-1) represents an oxygen atom or a sulfur atom, and preferably represents an oxygen atom.

R⁶ in the partial structure (T-1) represents a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted 5- or 6-membered heterocyclyl group.

Examples of the "substituted or unsubstituted C1-6 alkyl group", "substituted or unsubstituted C2-6 alkenyl group", "substituted or unsubstituted C2-6 alkynyl group", "substituted or unsubstituted C1-6 alkylcarbonyl group", "substituted or unsubstituted C1-6 alkoxycarbonyl group", "substituted or unsubstituted C3-6 cycloalkyl group", "substituted or unsubstituted phenyl group", "substituted or unsubstituted naphthyl group", and "substituted or unsubstituted 5- or 6-membered heterocyclyl group" for R⁶ include the same groups as those described for R² above.

Among these, R⁶ in the partial structure (T-1) is preferably a hydrogen atom or an unsubstituted C1-6 alkyl group, and more preferably a hydrogen atom.

Z^{b} in the partial structure (T-2) represents a C1-6 alkoxy group or a C1-6 alkylthio group.

Examples of the "C1-6 alkoxy group" and "C1-6 alkylthio group" for Z^{b} include the same groups as those described for R² above.

Among these, Z^{b} in the partial structure (T-2) is preferably a methoxy group or a methylthio group.

Among the above, it is preferable that T in Formula (Z-I) represents the partial structure (T-1).

R⁴'s in Formula (Z-I) each independently represent a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted 5- or 6-membered heterocyclyl group, or a halogeno group.

R⁵'s in Formula (Z-I) each independently represent a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted 5- or 6-membered heterocyclyl group, or a halogeno group.

Examples of the "substituted or unsubstituted C1-6 alkyl group", "substituted or unsubstituted C2-6 alkenyl group", "substituted or unsubstituted C2-6 alkynyl group", "substituted or unsubstituted C3-6 cycloalkyl group", "substituted or unsubstituted phenyl group", "substituted or unsubstituted naphthyl group", "substituted or unsubstituted 5- or 6-membered heterocyclyl group", and "halogeno group" for R⁴ and R⁵ include the same groups as those described for R² above.

R⁴ and R⁵ on the same carbon atom may together form a divalent organic group. Here, examples of the divalent organic group include C2-5 alkylene groups such as a dimethylene group, a trimethylene group, and a tetramethylene group.

Among the above, it is preferable that R⁴ and R⁵ in Formula (Z-I) are hydrogen atoms. Alternatively, R⁴ and R⁵ on the same carbon atom may together form a trimethylene group.

n is the number of repetitions of the structural unit in [], and represents any integer of 1 to 3.

n is preferably 1 or 2, and more preferably 1.

The 5-membered heteroaryl compound according to the embodiment of the present invention is preferably a compound represented by Formula (I) (hereinafter, sometimes referred to as "compound (I)") or a salt thereof. Each symbol in Formula (I) has the same meaning as those in Formula (Z-I).

In Formula (I), it is preferable that B¹ is a group represented by the formula: NR¹, B² is a nitrogen atom, and B³ is a nitrogen atom.

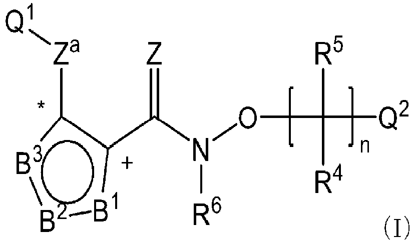

An example of the 5-membered heteroaryl compound according to the embodiment of the present invention is a compound represented by Formula (I-A) or a salt thereof.

Each symbol in Formula (I-A) has the same meaning as those in Formula (I).

In Formula (I-A), it is preferable that B¹ is a group represented by the formula: NR¹, B² is a nitrogen atom, and B³ is a nitrogen atom.

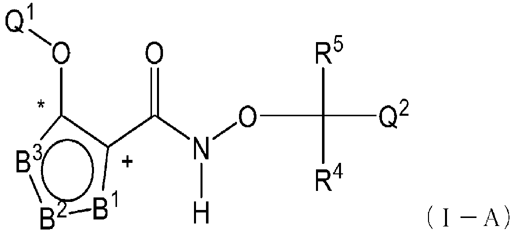

The salt of the compound (Z-I) is not particularly limited as long as it is an agriculturally and horticulturally acceptable salt. Examples of the salt of the compound (Z-I) include salts of inorganic acids such as hydrochloric acid and sulfuric acid; salts of organic acids such as acetic acid and lactic acid; salts of alkali metals such as lithium, sodium, and potassium; salts of alkaline earth metals such as calcium and magnesium; salts of transition metals such as iron and copper; ammonia salts; and salts of organic bases such as triethylamine, tributylamine, pyridine, and hydrazine.

A production method for the compound (Z-I) or the salt of the compound (Z-I) is not particularly limited. For example, the compound (Z-I) or the salt of the compound (Z-I) can be obtained by known production methods described in Examples and the like. In addition, the salt of the compound (Z-I) can be obtained from the compound (Z-I) by a known method.

### Production method for compound (I)

### Production method 1

The compound of Formula (I) can be produced by a method including a step of reacting a compound of Formula (im-1) with a compound of Formula (im-2) as shown in Scheme 1.

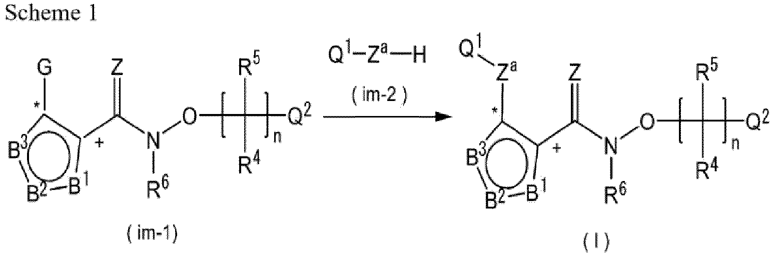

The compound of Formula (im-1) is a compound in which G is a halogeno group and the other symbols are the same as those defined in the compound of Formula (I).

The compound of Formula (im-2) is a compound in which the symbols in the formula are the same as those defined in the compound of Formula (I).

In each formula in the scheme, it is preferable that B¹ is a group represented by the formula: NR¹, B² is a nitrogen atom, and B³ is a nitrogen atom.

The compound of Formula (I) can be produced by reacting the compound of Formula (im-1) with the compound of Formula (im-2) by a known method. The reaction may be carried out in the presence of an acid scavenger.

Examples of the acid scavenger include common inorganic bases and organic bases, and preferred examples thereof include an alkali metal carbonate, an alkaline earth metal acetate, a tertiary amine, and an aromatic amine.

The compound of Formula (im-2) may be commercially available or may be produced by a known method.

The compound of Formula (im-1) can be produced by one or more of methods described herein (see Production method 4).

### Production method 2

A compound of Formula (I-2) can be produced by a method including a step of reacting a compound of Formula (im-3) with a compound of Formula (im-4) as shown in Scheme 2.

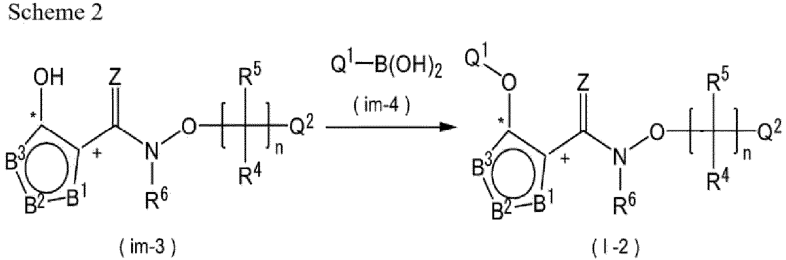

Here, the compound of Formula (I-2) is the compound (I) in which Z^{a} is oxygen.

The compound of Formula (im-3) is a compound in which the symbols in the formula are the same as those defined in the compound of Formula (I-2).

The compound of Formula (im-4) is a compound in which Q¹ is the same as that defined in the compound of Formula (I-2).

In each formula in the scheme, it is preferable that B¹ is a group represented by the formula: NR¹, B² is a nitrogen atom, and B³ is a nitrogen atom.

The compound of Formula (I-2) can be produced by reacting the compound of Formula (im-3) with the compound of Formula (im-4) in the presence of an organic base and a copper catalyst using oxygen as an oxidizing agent. This is so-called Chan-Lam-Evans coupling.

Examples of the copper catalyst include, but are not limited to, divalent copper catalysts such as copper(II) chloride, copper(II) bromide, copper(II) oxide, copper(II) acetate, copper(II) sulfate, copper(II) bis(2,2,6,6-tetramethyl-3,5-heptanedionate), and copper(II) trifluoromethanesulfonate.

Examples of the organic base include, but are not limited to, tertiary amines such as triethylamine and aromatic amines such as pyridine.

The compound of Formula (im-4) may be commercially available or may be produced by a known method.

The compound of Formula (im-3) can be produced by one or more of methods described herein (see Production method 5).

### Production method 3

A compound of Formula (I-3) can be produced by a method including a step of reacting a compound of Formula (im-5) with a compound of Formula (im-6) or Formula (im-6-2) as shown in Scheme 3.

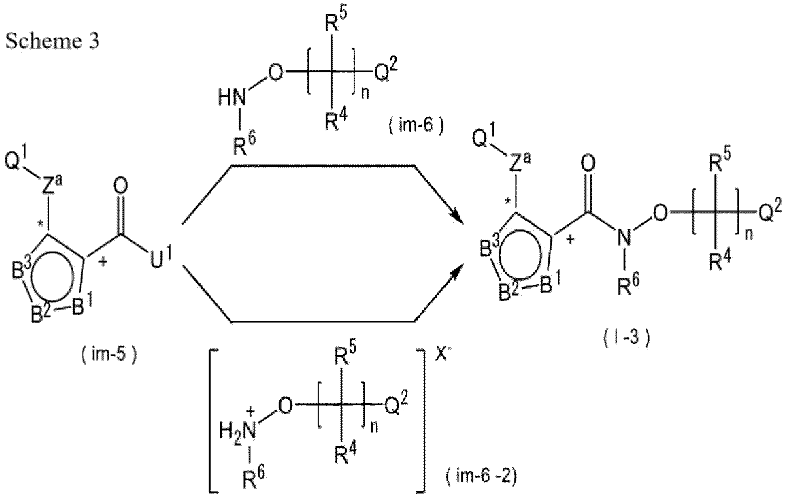

Here, the compound of Formula (I-3) is the compound (I) in which Z is an oxygen atom.

In the compound of Formula (im-5), U¹ is a hydroxyl group or a halogeno group, and is preferably a hydroxyl group. The other symbols are the same as those defined in the compound of Formula (I).

The compound of Formula (im-6) is a compound in which the symbols in the formula are the same as those defined in the compound of Formula (I).

In the compound of Formula (im-6-2), X⁻ represents a counter anion, examples thereof include a chloride ion, a bromide ion, and an iodide ion, and a chloride ion is preferable. The other symbols are the same as those defined in the compound of Formula (I-3).

In each formula in the scheme, it is preferable that B¹ is a group represented by the formula: NR¹, B² is a nitrogen atom, and B³ is a nitrogen atom.

The compound of Formula (I-3) can be produced by reacting the compound of Formula (im-5), in which U¹ is a hydroxyl group, with the compound of Formula (im-6) in the presence of a condensing agent.

Examples of the condensing agent include, but are not limited to, a halogenating agent (for example, phosgene, phosphorus tribromide, phosphorus trichloride, phosphorus pentachloride, phosphorus oxychloride, oxalyl chloride, or thionyl chloride), a dehydrating agent (for example, ethyl chloroformate, methyl chloroformate, isopropyl chloroformate, isobutyl chloroformate, or methanesulfonyl chloride), and carbonyldiimidazole.

In addition, the compound of Formula (I-3) can also be produced by reacting the compound of Formula (im-5), in which U¹ is a hydroxyl group, with the compound of Formula (im-6-2) in the presence of an organic base and a condensing agent. 1-Hydroxybenzotriazole can also be used in combination.

As the organic base, a tertiary amine such as triethylamine can be used.

As the condensing agent, carbodiimides (for example, N,N'-dicyclohexylcarbodiimide (DCC)), 1-hydroxy-7-azabenzotriazole (HOAt), O-(7-azabenzotriazol-1-yl)-N,N,N',N'-tetramethyluronium hexafluorophosphate (HATU), propylphosphonic anhydride, or the like can be used; and HATU (1-[bis(dimethylamino)methylene]-1H-1,2,3-triazolo[4,5-b]pyridinium 3-oxide hexafluorophosphate) is preferably used.

The compound of Formula (I-3) can also be produced by reacting the compound of Formula (im-5), in which U¹ is a halogen atom, with the compound of Formula (im-6) or (im-6-2) in the presence of an acid scavenger by a known method.

Examples of the acid scavenger include common inorganic bases and organic bases, and preferred examples thereof include an alkali metal carbonate, an alkaline earth metal acetate, a tertiary amine, and an aromatic amine.

The compound of Formula (im-5) may be commercially available or may be produced according to a method described in a document (for example, Published Japanese Translation No. 2022-508277 of the PCT International Publication or Published Japanese Translation No. 2022-514651 of the PCT International Publication). In addition, the compound of Formula (im-5) can be produced by one or more of methods described herein (see Production methods 6 and 7).

The compound of Formula (im-6) or (im-6-2) may be commercially available. In addition, the compound of Formula (im-6) or (im-6-2) can be produced by one or more of methods described herein (see Production method 8).

Here, the compound of Formula (I-3) can be converted to a compound in which Z is a sulfur atom, by using a thionating agent.

Examples of the thionating agent include, but are not limited to, sulfur, hydrosulfuric acid, sodium sulfide, sodium hydrosulfide, boron trisulfide, bis(diethylaluminum) sulfide, ammonium sulfide, phosphorus pentasulfide, and Lawesson's reagent.

As necessary, the reaction may be carried out in the presence of an inorganic base or an organic base. An alkali metal carbonate, an alkaline earth metal acetate, a tertiary amine, or an aromatic amine is preferable.

### Production method 4

A compound of Formula (im-1a) can be produced by a method including a step of reacting a compound of Formula (im-7) with a compound of Formula (im-6) or (im-6-2) as shown in Scheme 4.

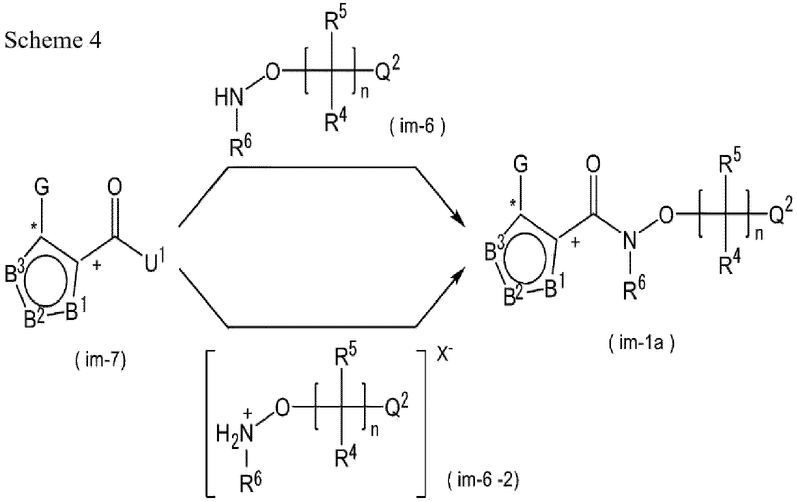

Here, the compound of Formula (im-1a) is the compound of Formula (im-1), in which Z is an oxygen atom.

The compound of Formula (im-7) is a compound in which G is a halogeno group, U¹ is a hydroxyl group or a halogeno group, and the other symbols are the same as those defined in the compound of Formula (im-1).

In each formula in the scheme, it is preferable that B¹ is a group represented by the formula: NR¹, B² is a nitrogen atom, and B³ is a nitrogen atom.

As reagents and reaction conditions used in Production method 4, those described in Production method 3 can be used.

The compound of Formula (im-7) may be commercially available.

Here, the compound of Formula (im-1a) can be converted to a compound in which Z is a sulfur atom, by using a thionating agent. The conversion may be carried out by the method described in Production method 3.

### Production method 5

A compound of Formula (im-3a) can be produced by a method including a step of reacting a compound of Formula (im-8) with a compound of Formula (im-6) or (im-6-2) as shown in Scheme 5.

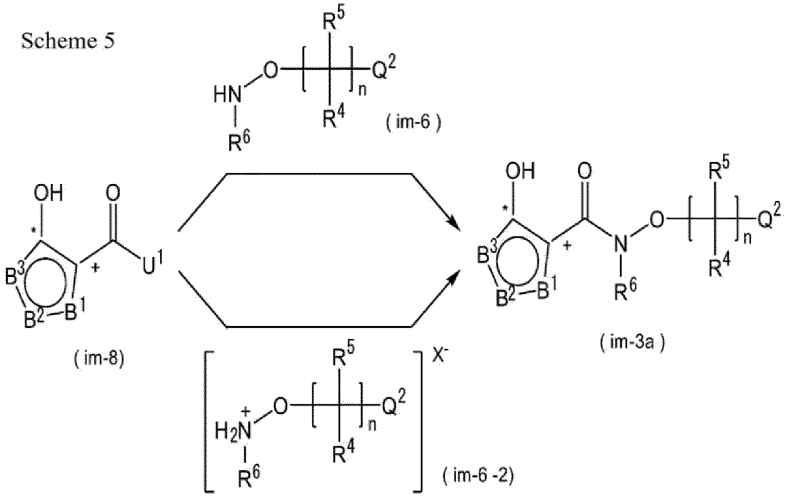

Here, the compound of Formula (im-3a) is a compound of Formula (im-3), in which Z is an oxygen atom.

The compound of Formula (im-8) is a compound in which U¹ is a hydroxyl group or a halogeno group and the other symbols are the same as those defined in the compound of Formula (im-3).

In each formula in the scheme, it is preferable that B¹ is a group represented by the formula: NR¹, B² is a nitrogen atom, and B³ is a nitrogen atom.

As reagents and reaction conditions used in Production method 5, those described in Production method 3 can be used.

The compound of Formula (im-8) may be commercially available. In addition, the compound of Formula (im-8) can be produced by one or more of methods described herein (see Production method 7).

Here, the compound of Formula (im-3a) can be converted to a compound in which Z is a sulfur atom, by using a thionating agent. The conversion may be carried out by the method described in Production method 3.

### Production method 6

The compound of Formula (im-5) can be produced by a method including a step of reacting a compound of Formula (im-9) with a compound of Formula (im-2) as shown in Scheme 6, and a step of converting a compound of Formula (im-10) obtained by the reaction into the compound of Formula (im-5).

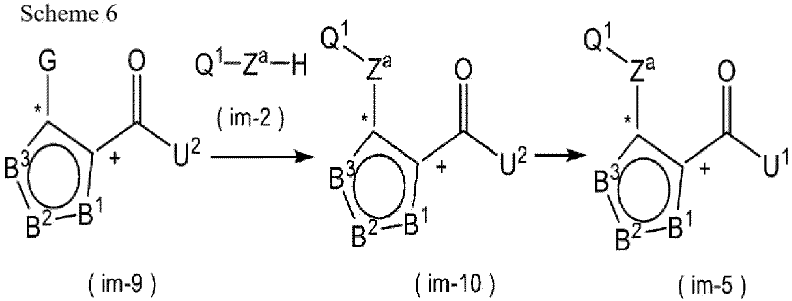

The compound of Formula (im-9) is a compound in which U² is a C1-6 alkoxy group, G is a halogeno group, and the other symbols are the same as those defined in the compound of Formula (im-5).

The compound of Formula (im-10) is a compound in which U² is a C1-6 alkoxy group and the other symbols are the same as those defined in the compound of Formula (im-5).

In each formula in the scheme, it is preferable that B¹ is a group represented by the formula: NR¹, B² is a nitrogen atom, and B³ is a nitrogen atom.

The compound of Formula (im-10) can be produced by reacting the compound of Formula (im-9) with the compound of Formula (im-2) by a known method. The reaction may be carried out in the presence of an acid scavenger.

Examples of the acid scavenger include common inorganic bases and organic bases. An alkali metal carbonate, an alkaline earth metal acetate, a tertiary amine, or an aromatic amine is preferable.

The compound of Formula (im-5), in which U¹ is a hydroxyl group, can be produced by hydrolyzing the compound of Formula (im-10) under acidic conditions or basic conditions.

Examples thereof include a method of providing acidic conditions by using trifluoroacetic acid, and a method of providing basic conditions by using lithium hydroxide in a mixed solvent system of tetrahydrofuran (THF) and water.

The compound of Formula (im-5), in which U¹ is a halogeno group, can be produced by reacting the compound of Formula (im-5), in which U¹ is a hydroxyl group, with a halogenating agent.

The compound of Formula (im-9) may be commercially available or may be produced by a known method.

### Production method 7

The compound of Formula (im-5a) can be produced by a method including a step of reacting a compound of Formula (im-12) with a compound of Formula (im-13) as shown in Scheme 7, and a step of converting a compound of Formula (im-10a) obtained by the reaction into the compound of Formula (im-5a).

In addition, the compound of Formula (im-8a) can be produced by a method including a step of converting the compound of Formula (im-12) into the compound of Formula (im-8a).

The compound of Formula (im-12) can also be produced by a method including a reaction step of performing a reaction between a compound of Formula (im-11) and a boron compound and then oxidation.

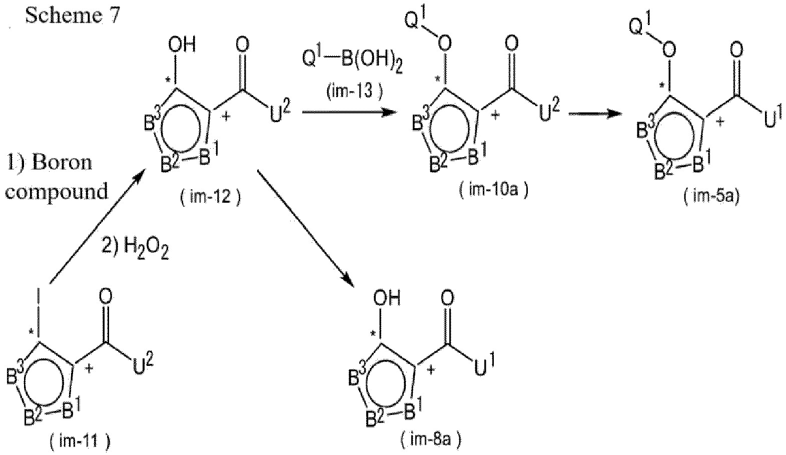

Here, the compound of Formula (im-5a) is the compound of Formula (im-5), in which Z^{a} is an oxygen atom.

The compound of Formula (im-11) is a compound in which U² is a C1-6 alkoxy group and the other symbols are the same as those defined in the compound of Formula (Z-I).

The compound of Formula (im-12) is a compound in which U² is a C1-6 alkoxy group and the other symbols are the same as those defined in the compound of Formula (Z-I).

The compound of Formula (im-13) is a compound in which Q¹ is the same as that defined in the compound of Formula (Z-I).

The compound of Formula (im-10a) is a compound in which U² is a C1-6 alkoxy group and the other symbols are the same as those defined in the compound of Formula (Z-I).

The compound of Formula (im-8a) is the same as the compound of Formula (im-8) described above.

In each formula in the scheme, it is preferable that B¹ is a group represented by the formula: NR¹, B² is a nitrogen atom, and B³ is a nitrogen atom.

The compound of Formula (im-10a) can be produced by reacting the compound of Formula (im-12) with the compound of Formula (im-13) in the presence of an organic base and a copper catalyst, using oxygen as a reoxidizing agent. This is so-called Chan-Lam-Evans coupling.

Examples of the copper catalyst include, but are not limited to, divalent copper catalysts such as copper(II) chloride, copper(II) bromide, copper(II) oxide, copper(II) acetate, copper(II) sulfate, copper(II) bis(2,2,6,6-tetramethyl-3,5-heptanedionate), and copper(II) trifluoromethanesulfonate.

Examples of the organic base include, but are not limited to, tertiary amines such as triethylamine and aromatic amines such as pyridine.

The compound of Formula (im-5a), in which U¹ is a hydroxyl group, can be produced by hydrolyzing the compound of Formula (im-10a) under acidic conditions or basic conditions.

Examples thereof include a method of providing acidic conditions by using trifluoroacetic acid, and a method of providing basic conditions by using lithium hydroxide in a mixed solvent system of an alcohol such as methanol, tetrahydrofuran (THF), and water. Among these, the method of providing basic conditions is preferable.

The compound of Formula (im-5a), in which U¹ is a halogeno group, can be produced by reacting the compound of Formula (im-5a), in which U¹ is a hydroxyl group, with a halogenating agent.

The compound of Formula (im-8a), in which U¹ is a hydroxyl group, can be produced by hydrolyzing the compound of Formula (im-12) under acidic conditions or basic conditions.

Examples thereof include a method of providing acidic conditions by using trifluoroacetic acid, and a method of providing basic conditions by using lithium hydroxide in a mixed solvent system of THF and water.

The compound of Formula (im-8a), in which U¹ is a halogeno group, can be produced by reacting the compound of Formula (im-8a), in which U¹ is a hydroxyl group, with a halogenating agent.

The compound of Formula (im-13) may be commercially available or may be produced by a known method.

The compound of Formula (im-12) may be commercially available or may be produced by a known method.

In addition, the compound of Formula (im-12) can also be produced by reacting the compound of Formula (im-11) with a boron compound in the presence of a palladium catalyst to convert it into an organoboron compound, and further oxidizing it.

The conversion to an organoboron compound is a so-called Miyaura-Ishiyama borylation reaction.

Examples of the palladium catalyst include, but are not limited to, tetrakis(triphenylphosphine)palladium(0), bis(dibenzylideneacetone)palladium(0), tris(dibenzylideneacetone)dipalladium(0), bis(triphenylphosphine)palladium(II) dichloride, [1,1'-bis(diphenylphosphino)ferrocene]dichloropalladium(II), and [1,1'-bis(di-tert-butylphosphino)ferrocene]dichloropalladium(II).

Examples of the boron compound include, but are not limited to, pinacoldiborane, pinacolborane, and diboronic acid.

By oxidizing the obtained organoboron compound using hydrogen peroxide under basic conditions, the compound of Formula (im-12), in which the carbon-boron bond has become a carbon-oxygen bond, can be produced.

The compound of Formula (im-11) may be commercially available or may be produced by a known method.

### Production method 8

A compound of Formula (im-6a) can be produced by a method including a step of reacting N-hydroxyphthalimide represented by Formula (im-19) with a compound of Formula (im-20) as shown in Scheme 8, and a step of reacting a compound of Formula (im-21) obtained by the reaction with hydrazines.

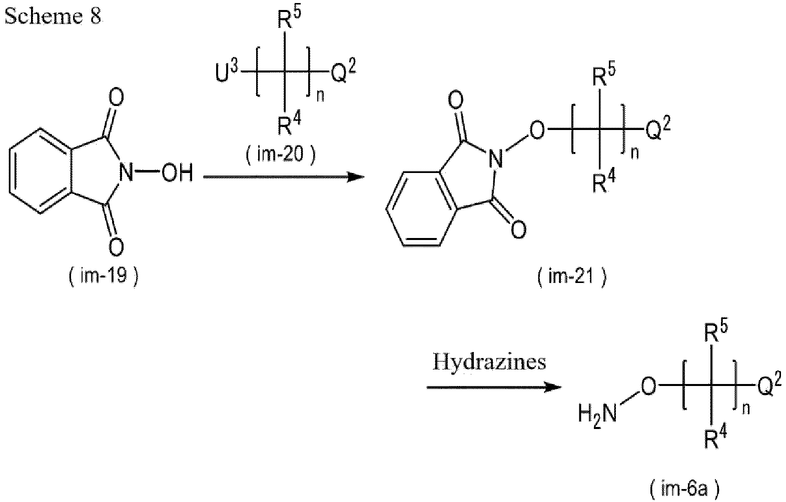

Here, the compound of Formula (im-6a) is a compound of Formula (im-6), in which R⁶ is a hydrogen atom.

The compound of Formula (im-20) is a compound in which U³ is a hydroxyl group or a halogeno group and the other symbols are the same as those defined in the compound of Formula (im-6).

The compound of Formula (im-21) is a compound in which the symbols in the formula are the same as those defined in the compound of Formula (im-6).

The compound of Formula (im-21) can be produced by reacting the compound of Formula (im-20), in which U³ is a hydroxyl group, with the compound of Formula (im-19) in the presence of a dialkyl azodicarboxylate and triphenylphosphine. This is a so-called Mitsunobu reaction.

Examples of the dialkyl azodicarboxylate include, but are not limited to, diethyl azodicarboxylate and diisopropyl azodicarboxylate.

The compound of Formula (im-21) can be produced by reacting the compound of Formula (im-20), in which U³ is a halogen atom, with the compound of Formula (im-19) in the presence of an acid scavenger by a known method.

Examples of the acid scavenger include common inorganic bases and organic bases. An alkali metal carbonate, an alkaline earth metal acetate, a tertiary amine, or an aromatic amine is preferable.

The compound of Formula (im-6a) can be produced by reacting the compound of Formula (im-21) with hydrazines.

Examples of the hydrazines include hydrazine monohydrate and methylhydrazine.

The compound of Formula (im-19) may be commercially available or may be produced by a known method.

The compound of Formula (im-20) may be commercially available or may be produced by a known method.

### Production method 9

A compound of Formula (im-5c) can be produced by a method including a step of reacting a compound of Formula (im-24) with a compound of Formula (im-2a) as shown in Scheme 9, and a step of converting a compound of Formula (im-25) obtained by the reaction into the compound of Formula (im-5c).

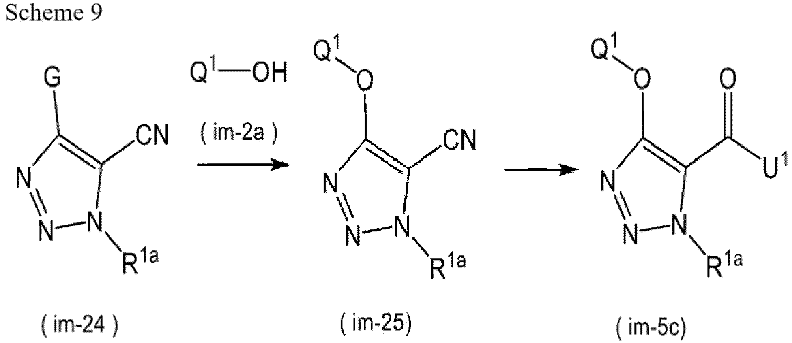

The compound of Formula (im-24) is a compound in which G is a halogeno group and R^{1a} is the same as that defined in a compound of Formula (im-5B) below.

The compound of Formula (im-2a) is a compound in which Q¹ is the same as that defined in the compound of Formula (im-5B) below.

The compound of Formula (im-25) is a compound in which Q¹ and R^{1a} are the same as those defined in the compound of Formula (im-5B) below.

The compound of Formula (im-5c) is a compound in which U¹ represents a hydroxyl group or a halogeno group and Q¹ and R^{1a} are the same as those defined in the compound of Formula (im-5B) below.

The compound of Formula (im-25) can be produced by reacting the compound of Formula (im-24) with the compound of Formula (im-2a) by a known method. The reaction may be carried out in the presence of an acid scavenger.

Examples of the acid scavenger include common inorganic bases and organic bases. An alkali metal carbonate, an alkaline earth metal acetate, a tertiary amine, or an aromatic amine is preferable, and an alkali metal carbonate such as potassium carbonate and cesium carbonate, or a tertiary amine such as dimethylglycine is more preferable.

A reaction temperature is appropriately set, and is, for example, approximately 100°C to 200°C. A reaction time is appropriately set, and is, for example, approximately 0.5 hours to 6 hours.

The compound of Formula (im-5c), in which U¹ is a hydroxyl group, can be produced by hydrolyzing the compound of Formula (im-25) under acidic conditions or basic conditions.

Examples thereof include a method of providing acidic conditions by using trifluoroacetic acid, and a method of providing basic conditions by using lithium hydroxide in a mixed solvent system of tetrahydrofuran (THF) and water, or a mixed solvent system of an alcohol such as methanol, THF, and water. Among these, the method of providing basic conditions is preferable.

A hydrolysis temperature is appropriately set, and is, for example, approximately 0°C to 60°C. A hydrolysis time is appropriately set, and is, for example, approximately 1 hour to 24 hours.

Alternatively, when an amide is obtained without hydrolysis to a carboxylic acid by using an aqueous solution of an alkali metal hydroxide such as sodium hydroxide in an alcohol solvent system such as ethanol, the amide can be converted into the carboxylic acid by reaction with sodium nitrite or a nitrous acid ester in a solvent of acetic acid.

A reaction temperature is appropriately set, and is, for example, approximately 0°C to 100°C. A hydrolysis time is appropriately set, and is, for example, approximately 1 hour to 6 hours, preferably approximately 0°C to 10°C.

The compound of Formula (im-5c), in which U¹ is a halogeno group, can be produced by reacting the compound of Formula (im-5c), in which U¹ is a hydroxyl group, with a halogenating agent.

The compound of Formula (im-24) may be commercially available or may be produced by a known method.

### Production intermediate

A compound represented by Formula (im-5B) or a salt thereof may serve as a production intermediate for the compound (Z-I).

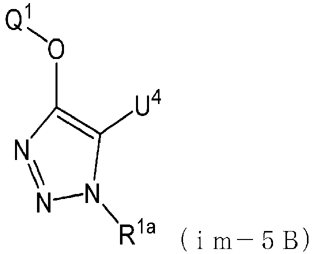

In Formula (im-5B), Q¹ is the same as that defined in the compound of Formula (Z-I). Among these, Q¹ is preferably a substituted phenyl group. The substituent on the phenyl group is preferably a halogeno group, a C1-6 alkyl group, or a C1-6 haloalkyl group, and more preferably a C1-6 haloalkyl group.

R^{1a} represents a substituted or unsubstituted C2-6 alkyl group or a substituted or unsubstituted C3-6 cycloalkyl group. The substituent on the "C2-6 alkyl group" and the "C3-6 cycloalkyl group" is preferably a halogeno group. Among these, R^{1a} is preferably a t-butyl group.

U⁴ represents a carboxyl group, a C1-6 alkoxycarbonyl group, or a halogenocarbonyl group. Among these, U⁴ is preferably a carboxyl group or a C1-6 alkoxycarbonyl group.

### Production intermediate

A compound represented by Formula (f-1) (hereinafter, sometimes referred to as "compound (f-1)") or a compound represented by Formula (f-2) (hereinafter, sometimes referred to as "compound (f-2)") may serve as a production intermediate for the compound (Z-I).

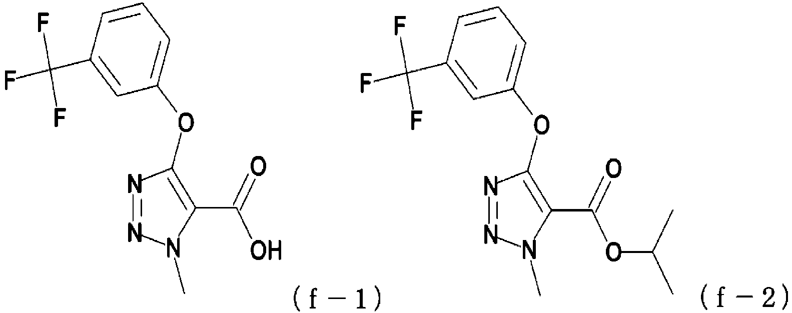

### Production intermediate

A compound represented by Formula (im-25) or a salt thereof may serve as a production intermediate for the compound (Z-I).

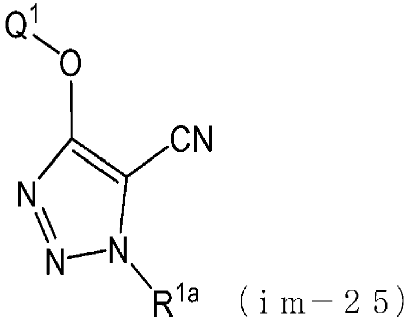

In Formula (im-25), Q¹ is the same as that defined in the compound of Formula (Z-I). Among these, Q¹ is preferably a substituted or unsubstituted phenyl group. The substituent on the "phenyl group" is preferably a halogeno group, a C1-6 alkyl group, or a C1-6 haloalkyl group, more preferably a halogeno group or a C1-6 haloalkyl group, and still more preferably a C1-6 haloalkyl group.

R^{1a} represents a substituted or unsubstituted C2-6 alkyl group or a substituted or unsubstituted C3-6 cycloalkyl group. The substituent on the "C2-6 alkyl group" and the "C3-6 cycloalkyl group" is preferably a halogeno group. Among these, R^{1a} is preferably an unsubstituted C2-6 alkyl group, and more preferably a t-butyl group.

The agricultural and horticultural fungicide according to the embodiment of the present invention contains, as an active ingredient, at least one selected from the group consisting of the compound (Z-I) and the salt of the compound (Z-I) (which may hereinafter be referred to as "active ingredient (Z-I)"). The amount of the active ingredient (Z-I) contained in the agricultural and horticultural fungicide according to the embodiment of the present invention is not particularly limited as long as it exhibits a fungicidal effect.

The agricultural and horticultural fungicide according to the embodiment of the present invention can be used to control plant diseases caused by a wide variety of filamentous fungi, for example, fungi belonging to Oomycetes, Ascomycetes, Deuteromycetes, Basidiomycetes, and Zygomycetes.

Examples of the plant diseases (pathogenic fungi) to be controlled are shown below.
Sugar beet: Cercospora leaf spot (Cercospora beticola), Aphanomyces root rot (Aphanomyces cochlioides), root rot (Thanatephorus cucumeris), leaf rot (Thanatephorus cucumeris), rust (Uromyces betae), powdery mildew (Oidium sp.), Ramularia leaf spot (Ramularia beticola), damping-off (Aphanomyces cochlioides, Pythium ultimum), and the like
Peanut: brown leaf spot (Mycosphaerella arachidis), Ascochyta leaf spot (Ascochyta sp.), rust (Puccinia arachidis), damping-off (Pythium debaryanum), Alternaria leaf spot (Alternaria alternata), southern blight (Sclerotium rolfsii), late leaf spot (Mycosphaerella berkeleyi), black root rot (Calonectria ilicicola), and the like
Cucumber: powdery mildew (Sphaerotheca fuliginea), downy mildew (Pseudoperonospora cubensis), gummy stem blight (Mycosphaerella melonis), Fusarium wilt (Fusarium oxysporum), Sclerotinia rot (Sclerotinia sclerotiorum), gray mold (Botrytis cinerea), anthracnose (Colletotrichum orbiculare), scab (Cladosporium cucumerinum), Corynespora leaf spot (Corynespora cassiicola), damping-off (Pythium debaryanum, Rhizoctonia solani Kuhn), Phomopsis root rot (Phomopsis sp.), angular leaf spot (Pseudomonas syringae pv. lachrymans), and the like
Tomato: gray mold (Botrytis cinerea), leaf mold (Cladosporium fulvum), late blight (Phytophthora infestans), Verticillium wilt (Verticillium albo-atrum, Verticillium dahliae), powdery mildew (Oidium neolycopersici), early blight (Alternaria solani), sooty leaf mold (Pseudocercospora fuligena), bacterial wilt (Ralstonia solanacearum), Sclerotinia rot (Sclerotinia sclerotiorum), and the like
Eggplant: gray mold (Botrytis cinerea), black blight (Corynespora melongenae), powdery mildew (Erysiphe cichoracearum), leaf mold (Mycovellosiella nattrassii), Sclerotinia rot (Sclerotinia sclerotiorum), Verticillium wilt (Verticillium dahliae), Phomopsis blight (Phomopsis vexans), and the like
Pepper: Phytophthora blight (Phytophthora capsici), gray mold (Botrytis cinerea), Sclerotinia rot (Sclerotinia sclerotiorum), anthracnose (Colletotrichum aenigma, Colletotrichum capsici, Colletotrichum fructicola, Colletotrichum jiangxiense), powdery mildew (Leveillula taurica), and the like
Strawberry: gray mold (Botrytis cinerea), powdery mildew (Sphaerotheca humuli), anthracnose (Colletotrichum acutatum, Colletotrichum fragariae), Phytophthora crown rot (Phytophthora cactorum), Rhizopus rot (Rhizopus stolonifer), Fusarium wilt (Fusarium oxysporum), Verticillium wilt (Verticillium dahliae), Sclerotinia rot (Sclerotinia sclerotiorum), and the like
Onion: neck rot (Botrytis allii), gray mold (Botrytis cinerea), Botrytis leaf blight (Botrytis squamosa), downy mildew (Peronospora destructor), Phytophthora porri blight (Phytophthora porri), Ciborinia leaf blight (Ciborinia allii), small sclerotial rot (Botrytis squamosa), Fusarium basal rot (Fusarium oxysporum), pink root (Pyrenochaeta terrestris), white rot (Sclerotium cepivorum), rust (Puccinia allii), southern blight (Sclerotium rolfsii), and the like
Welsh onion: soft rot (Pectobacterium carotovorum), downy mildew (Peronospora destructor), leaf blight (Pleospora allii), white rot (Sclerotium cepivorum), rust (Puccinia allii), Botrytis leaf blight (Botrytis squamosa), southern blight (Sclerotium rolfsii), pink root (Pyrenochaeta terrestris), and the like
Cabbage: clubroot (Plasmodiophora brassicae), soft rot (Erwinia carotovora), black rot (Xanthomonas campestris pv. campestris), bacterial leaf spot (Pseudomonas syringae pv. maculicola, P. s. pv. alisalensis), downy mildew (Peronospora parasitica), Sclerotinia rot (Sclerotinia sclerotiorum), dark leaf spot (Alternaria brassicicola), gray mold (Botrytis cinerea), black leg (Phoma lingam), Pythium rot (Pythium aphanidermatum, Pythium ultimum), white rust (Albugo macrospora), and the like
Lettuce: bacterial rot (Pseudomonas cichorii, Pseudomonas marginalis), soft rot (Pectobacterium carotovorum), downy mildew (Bremia lactucae), gray mold (Botrytis cinerea), Sclerotinia rot (Sclerotinia sclerotiorum), big vein disease (Mirafiori lettuce big-vein ophiovirus), root rot (Fusarium oxysporum), bottom rot (Rhizoctonia solani), powdery mildew (Golovinomyces orontii), and the like
Common bean: Sclerotinia rot (Sclerotinia sclerotiorum), gray mold (Botrytis cinerea), anthracnose (Colletotrichum lindemuthianum), angular leaf spot (Phaeoisariopsis griseola), and the like
Pea: Mycosphaerella blight, gray mold (Botrytis cinerea), Sclerotinia rot (Sclerotinia sclerotiorum), powdery mildew (Erysiphe pisi), and the like

Apple: powdery mildew (Podosphaera leucotricha), scab (Venturia inaequalis), Monilinia leaf blight (Monilinia mali), Brooks fruit spot (Mycosphaerella pomi), Valsa canker (Valsa mali), Alternaria blotch (Alternaria mali), rust (Gymnosporangium yamadae), ring rot (Botryosphaeria berengeriana), anthracnose (Glomerella cingulata, Colletotrichum acutatum), Marssonina blotch (Diplocarpon mali), fly speck (Zygophiala jamaicensis), sooty blotch (Gloeodes pomigena), violet root rot (Helicobasidium mompa), white root rot (Rosellinia necatrix), gray mold (Botrytis cinerea), fire blight (Erwinia amylovora), silver leaf (Chondrostereum purpureum), crown gall (Rhizobium radiobacter, Rhizobium rhizogenes), and the like
Japanese apricot: scab (Cladosporium carpophilum), gray mold (Botrytis cinerea), brown rot (Monilinia mumecola), sooty blotch (Peltaster sp.), plum pockets (Taphrina pruni), shot hole (Phloeosporella padi), and the like
Persimmon: powdery mildew (Phyllactinia kakicola), anthracnose (Gloeosporium kaki), angular leaf spot (Cercospora kaki), circular leaf spot (Mycosphaerella nawae), gray mold (Botrytis cinerea), fly speck (Zygophiala jamaicensis), and the like
Peach: brown rot (Monilinia fructicola, Monilia fructigena), scab (Cladosporium carpophilum), Phomopsis rot (Phomopsis sp.), bacterial spot (Xanthomonas campestris pv. pruni), leaf curl (Taphrina deformans), anthracnose (Colletotrichum gloeosporioides), brown leaf spot (Phloeosporella padi), Coriolus versicolor rot (Coriolus versicolor), and the like
Almond: brown rot (Monilinia laxa), shot hole (Stigmina carpophila), scab (Cladosporium carpophilum), red leaf blotch (Polystigma rubrum), Alternaria leaf spot (Alternaria alternata), anthracnose (Colletotrichum gloeosporioides), and the like
Sweet cherry: brown rot (Monilinia fructicola), anthracnose (Colletotrichum acutatum), Alternaria leaf spot (Alternaria sp.), young fruit rot (Monilinia kusanoi), brown leaf spot (Mycosphaerella cerasella), powdery mildew (Podosphaera tridactyla), and the like
Grape: gray mold (Botrytis cinerea), powdery mildew (Uncinula necator), ripe rot (Glomerella cingulata, Colletotrichum acutatum), downy mildew (Plasmopara viticola), anthracnose (Elsinoe ampelina), leaf spot (Pseudocercospora vitis), black rot (Guignardia bidwellii), white rot (Coniella castaneicola), rust (Phakopsora ampelopsidis), white cottony snow symptom (pathogen unidentified), crown gall (Rhizobium radiobacter, Rhizobium vitis), and the like
Pear: scab (Venturia nashicola), rust (Gymnosporangium asiaticum), black spot (Alternaria kikuchiana), ring rot (Botryosphaeria berengeriana), powdery mildew (Phyllactinia mali), Phomopsis canker (Phomopsis fukushii), brown spot (Stemphylium vesicarium), anthracnose (Glomerella cingulata), and the like
Tea: gray blight (Pestalotiopsis longiseta, P. theae), anthracnose (Colletotrichum theae-sinensis), net blister blight (Exobasidium reticulatum), bacterial shoot blight (Pseudomonas syringae), blister blight (Exobasidium vexans), and the like
Citrus: scab (Elsinoe fawcettii), blue mold (Penicillium italicum), green mold (Penicillium digitatum), gray mold (Botrytis cinerea), melanose (Diaporthe citri), canker (Xanthomonas campestris pv. citri), powdery mildew (Oidium sp.), Phytophthora rot (Phytophthora citrophthora), anthracnose (Colletotrichum fioriniae), and the like
Kiwifruit: bacterial blossom blight (Pseudomonas marginalis, Pseudomonas syringae, Pseudomonas viridiflava), bacterial canker (Pseudomonas syringae), gray mold (Botrytis cinerea), soft rot (Botryosphaeria dothidea, Diaporthe sp., Lasiodiplodia theobromae), sooty spot (Pseudocercospora actinidiae), and the like
Olive: anthracnose (Colletotrichum acutatum, Colletotrichum gloeosporioides), peacock spot (Spilocaea oleaginea), and the like
Chestnut: anthracnose (Colletotrichum gloeosporioides), and the like

Wheat: powdery mildew (Blumeria graminis f. sp. tritici), Fusarium head blight (Gibberella zeae, Fusarium avenaceum, Fusarium culmorum, Fusarium crookwellense, Microdochium nivale), leaf rust (Puccinia recondita), stripe rust (Puccinia striiformis), Pythium snow blight (Pythium iwayamai), pink snow mold (Monographella nivalis), eyespot (Pseudocercosporella herpotrichoides), Septoria leaf blotch (Septoria tritici), glume blotch (Leptosphaeria nodorum), Typhula snow blight (Typhula incarnata), Sclerotinia snow blight (Myriosclerotinia borealis), take-all (Gaeumannomyces graminis), ergot (Claviceps purpurea), common bunt (Tilletia caries), loose smut (Ustilago nuda), blast (Pyricularia grisea), damping-off (Pythium spp., Fusarium spp., Rhizoctonia spp.), seedling blight (Pythium spp., Fusarium spp., Rhizoctonia spp.), and the like
Barley: stripe (Pyrenophora graminea), net blotch (Pyrenophora teres), scald (Rhynchosporium secalis), loose smut (Ustilago tritici, U. nuda), damping-off (Pythium spp., Fusarium spp., Rhizoctonia spp.), seedling blight (Pythium spp., Fusarium spp., Rhizoctonia spp.), and the like
Rice: blast (Pyricularia oryzae), sheath blight (Rhizoctonia solani), bakanae disease (Gibberella fujikuroi), brown spot (Cochliobolus miyabeanus), seedling blight (Pythium graminicola), bacterial leaf blight (Xanthomonas oryzae), bacterial seedling blight (Burkholderia plantarii), bacterial brown stripe (Acidovorax avenae), bacterial grain rot (Burkholderia glumae), Cercospora leaf spot (Cercospora oryzae), false smut (Ustilaginoidea virens), discolored kernels (Alternaria alternata, Curvularia intermedia), black kernel (Alternaria padwickii), red discoloration (Epicoccum purpurascens), and the like
Tobacco: Sclerotinia rot (Sclerotinia sclerotiorum), powdery mildew (Erysiphe cichoracearum), black shank (Phytophthora nicotianae), and the like
Tulip: gray mold (Botrytis cinerea), fire (Botrytis tulipae), leaf rot (Rhizoctonia solani), bulb rot (Fusarium oxysporum), skin rot (Rhizoctonia solani), and the like
Rose: black spot (Diplocarpon rosae), powdery mildew (Erysiphe simulans, Podosphaera pannosa), gray mold (Botrytis cinerea), and the like
Chrysanthemum: gray mold (Botrytis cinerea), white rust (Puccinia horiana), downy mildew (Paraperonospora minor, Peronospora danica), Pythium root rot (Pythium aphanidermatum, Pythium dissotocum, Pythium helicoides, Pythium oedochilum, Pythium sylvaticum), Rhizoctonia damping-off (Rhizoctonia solani), Fusarium damping-off (Fusarium solani), and the like
Gerbera: gray mold (Botrytis cinerea), powdery mildew (Podosphaera xanthii), and the like
Lily: leaf blight (Botrytis elliptica, Pestalotiopsis sp.), gray mold (Botrytis cinerea), and the like
Sunflower: downy mildew (Plasmopara halstedii), Sclerotinia rot (Sclerotinia sclerotiorum), gray mold (Botrytis cinerea), and the like
Bentgrass: Sclerotinia snow mold (Sclerotinia borealis), large patch (Rhizoctonia solani), brown patch (Rhizoctonia solani), dollar spot (Sclerotinia homoeocarpa), blast (Pyricularia sp.), Pythium blight (Pythium aphanidermatum), anthracnose (Colletotrichum graminicola), and the like
Orchard grass: powdery mildew (Erysiphe graminis) and the like
Soybean: purple seed stain (Cercospora kikuchii), downy mildew (Peronospora manshurica), Phytophthora root and stem rot (Phytophthora sojae), rust (Phakopsora pachyrhizi), Sclerotinia rot (Sclerotinia sclerotiorum), anthracnose (Colletotrichum truncatum), gray mold (Botrytis cinerea), scab (Elsinoe glycines), pod and stem blight (Diaporthe phaseolorum var. sojae), damping-off (Pythium spp., Fusarium spp., Rhizoctonia spp.), seedling blight (Pythium spp., Fusarium spp., Rhizoctonia spp.), and the like
Potato: late blight (Phytophthora infestans), early blight (Alternaria solani), black scurf (Thanatephorus cucumeris), Verticillium wilt (Verticillium albo-atrum, V. dahliae, V. nigrescens), black leg (Pectobacterium atrosepticum), soft rot (Pectobacterium carotovorum), gray mold (Botrytis cinerea), scab (Streptomyces spp.), Sclerotinia rot (Sclerotinia sclerotiorum), and the like
Yam: Cylindrosporium leaf spot (Cylindrosporium dioscoreae), anthracnose (Colletotrichum gloeosporioides), blue mold (Penicillium sclerotigenum), and the like
Sweet potato: violet root rot (Helicobasidium mompa), Fusarium wilt (Fusarium oxysporum), and the like
Taro: Phytophthora blight (Phytophthora colocasiae), Rhizoctonia rot (Rhizoctonia solani), and the like
Ginger: rhizome rot (Pythium ultimum, Pythium myriotylum), Phyllosticta leaf spot (Phyllosticta zingiberis), and the like
Banana: Panama disease (Fusarium oxysporum), Sigatoka disease (Mycosphaerella fijiensis, M. musicola), and the like
Mango: anthracnose (Colletotrichum aenigma), bacterial black spot (Xanthomonas campestris), stem-end rot (Diaporthe pseudophoenicicola, Lasiodiplodia theobromae, Lasiodiplodia spp., Neofusicoccum parvum, Neofusicoccum sp.), gray mold (Botrytis cinerea), and the like
Rapeseed: Sclerotinia rot (Sclerotinia sclerotiorum), black leg (Phoma lingam), Alternaria black spot (Alternaria brassicae), powdery mildew (Erysiphe cruciferarum, Erysiphe cichoracearum, Oidium matthiolae), downy mildew (Peronospora parasitica), and the like
Coffee: rust (Hemileia vastatrix), anthracnose (Colletotrichum coffeanum), brown eye spot (Cercospora coffeicola), and the like
Sugarcane: brown rust (Puccinia melanocephala), and the like
Corn: zonate leaf spot (Gloeocercospora sorghi), rust (Puccinia sorghi), southern rust (Puccinia polysora), smut (Ustilago maydis), southern leaf blight (Cochliobolus heterostrophus), northern leaf blight (Setosphaeria turcica), damping-off (Pythium spp., Fusarium spp., Rhizoctonia spp.), seedling blight (Pythium spp., Fusarium spp., Rhizoctonia spp.), and the like
Cotton: damping-off (Pythium sp.), rust (Phakopsora gossypii), grey mildew (Mycosphaerella areola), anthracnose (Glomerella gossypii), and the like
Hop: downy mildew (Pseudoperonospora humuli), powdery mildew (Oidium sp., Podosphaera macularis), gray mold (Botrytis cinerea), and the like

The agricultural and horticultural fungicide according to the embodiment of the present invention is preferably used for plants such as cereals, vegetables, root vegetables, tuber crops, trees such as fruit trees, tea, coffee, and cacao, pasture grasses, turf grasses, and cotton.

The agricultural and horticultural fungicide according to the embodiment of the present invention can be applied to various parts of plants, for example, leaves, stems, petioles, flowers, buds, fruits, seeds, sprouts, roots, tubers, root tubers, shoots, and cuttings. In addition, improved varieties, variants, cultivars, mutants, hybrids, and genetically modified organisms (GMOs) of these plants can also be targeted.

The agricultural and horticultural fungicide according to the embodiment of the present invention can be used for seed treatment, foliar application, soil application, water-surface application, and the like, which are carried out to control various diseases occurring in agricultural and horticultural crops including flowers, turf grasses, and pasture grasses.

The agricultural and horticultural fungicide according to the embodiment of the present invention may contain other components in addition to the active ingredient (Z-I). Examples of the other components include a known carrier used for formulation. In addition, examples of the other components include conventionally known fungicides, insecticides and acaricides, nematicides, soil pest control agents, plant growth regulators, synergists, fertilizers, soil conditioners, and animal feeds (which may hereinafter be referred to as "active ingredient (II)"). The agricultural and horticultural fungicide according to the embodiment of the present invention may exhibit a synergistic effect by containing both the active ingredient (Z-I) and the active ingredient (II).

Specific examples of fungicides as the active ingredient (II) that can be mixed with or used in combination with the agricultural and horticultural fungicide according to the embodiment of the present invention are shown below.
(1) Nucleic acid biosynthesis inhibitors:
   (a) RNA polymerase I inhibitors: benalaxyl, benalaxyl-M, furalaxyl, metalaxyl, metalaxyl-M, oxadixyl, clozylacon, and ofurace;
   (b) Adenosine deaminase inhibitors: bupirimate, dimethirimol, and ethirimol;
   (c) DNA/RNA synthesis inhibitors: hymexazol and octhilinone; and
   (d) DNA topoisomerase II inhibitor: oxolinic acid
(2) Mitosis inhibitors and cell division inhibitors:
   (a) β-tubulin polymerization inhibitors: benomyl, carbendazim, chlorfenazole, fuberidazole, thiabendazole, thiophanate, thiophanate-methyl, diethofencarb, zoxamide, and ethaboxam;
   (b) Cell division inhibitor: pencycuron; and
   (c) Delocalization inhibitors of spectrin-like proteins: fluopicolide and fluopimomide
(3) Respiration inhibitors:
   (a) Complex I-NADH oxidoreductase inhibitors: diflumetorim and tolfenpyrad;
   (b) Complex II-succinate dehydrogenase inhibitors: benodanil, flutolanil, mepronil, isofetamid, fluopyram, fenfuram, flumecyclox, carboxin, oxycarboxin, thifluzamide, benzovindiflupyr, bixafen, fluxapyroxad, furametpyr, isopyrazam, penflufen, penthiopyrad, sedaxane, boscalid, pydiflumetofen, isoflucypram, pyraziflumid, and inpyrfluxam;
   (c) Complex III-ubiquinol oxidase Qo inhibitors: azoxystrobin, coumoxystrobin, coumethoxystrobin, enoxastrobin, flufenoxystrobin, picoxystrobin, pyraoxystrobin, pyraclostrobin, pyrametostrobin, triclopyricarb, kresoxim-methyl, trifloxystrobin, dimoxystrobin, fenaminstrobin, metominostrobin, orysastrobin, famoxadone, fluoxastrobin, fenamidone, pyribencarb, metyltetraprole, and mandestrobin;
   (d) Complex III-ubiquinol reductase Qi inhibitors: cyazofamid, amisulbrom, and fenpicoxamid;
   (e) Oxidative phosphorylation uncouplers: binapacryl, meptyldinocap, dinocap, fluazinam, and ferimzone;
   (f) Oxidative phosphorylation inhibitors (inhibitors of ATP synthase): fentin acetate, fentin chloride, and fentin hydroxide;
   (g) ATP production inhibitors: silthiofam; and
   (h) Complex III: cytochrome bc1 (ubiquinone reductase) Qx (unknown) inhibitor: ametoctradin;
(4) Amino acid and protein synthesis inhibitors:
   (a) Methionine biosynthesis inhibitors: andoprim, cyprodinil, mepanipyrim, and pyrimethanil; and
   (b) Protein synthesis inhibitors: blasticidin-S, kasugamycin, kasugamycin hydrochloride, streptomycin, and oxytetracycline;
(5) Signal transduction inhibitors:
   (a) Signal transduction inhibitors: quinoxyfen and proquinazid; and
   (b) MAP/histidine kinase inhibitors in osmotic signal transduction: fenpiclonil, fludioxonil, chlozolinate, iprodione, procymidone, and vinclozolin;
(6) Lipid and cell membrane synthesis inhibitors:
   (a) Phospholipid biosynthesis, methyltransferase inhibitors: edifenphos, iprobenfos, pyrazophos, and isoprothiolane;
   (b) Lipid peroxidants: biphenyl, chloroneb, dicloran, quintozene, tecnazene, tolclofos-methyl, and etridiazole;
   (c) Agents acting on cell membranes: iodocarb, propamocarb, propamocarb hydrochloride, propamocarb-fosetylate, and prothiocarb;
   (d) Microorganisms that disrupt cell membranes of pathogenic fungi: Bacillus subtilis, Bacillus subtilis strain QST713, Bacillus subtilis strain FZB24, Bacillus subtilis strain MBI600, Bacillus subtilis strain D747, and Bacillus amyloliquefaciens; and
   (e) Agents that disrupt cell membranes: cajeput (tea tree) extract
(7) Inhibitors of sterol biosynthesis in cell membranes:
   (a) Inhibitors of C14 demethylation in sterol biosynthesis: triforine, pyrifenox, pyrisoxazole, fenarimol, flurprimidol, nuarimol, imazalil, imazalil sulfate, oxpoconazole, oxpoconazole fumarate, pefurazoate, prochloraz, triflumizole, viniconazole, azaconazole, bitertanol, bromuconazole, cyproconazole, diclobutrazol, difenoconazole, diniconazole, diniconazole-M, epoxiconazole, etaconazole, fenbuconazole, fluquinconazole, flusilazole, flutriafol, fluconazole, fluconazole-cis, hexaconazole, imibenconazole, ipconazole, metconazole, myclobutanil, penconazole, propiconazole, fluquinconazole, simeconazole, tebuconazole, tetraconazole, triadimefon, triadimenol, triticonazole, prothioconazole, voriconazole, and mefentrifluconazole;
   (b) Inhibitors of Δ14 reductase and Δ8 to Δ7 isomerase in sterol biosynthesis: aldimorph, dodemorph, dodemorph acetate, fenpropimorph, tridemorph, fenpropidin, piperalin, and spiroxamine;
   (c) 3-Ketoreductase inhibitors in C4 demethylation of the sterol biosynthesis system: fenhexamid and fenpyrazamine; and
   (d) Squalene epoxidase inhibitors of the sterol biosynthesis system: pyributicarb, naftifine, and terbinafine
(8) Cell wall synthesis inhibitors:
   (a) Trehalase inhibitors: validamycin;
   (b) Chitin synthase inhibitors: polyoxin and polyoxorim; and
   (c) Cellulose synthase inhibitors: dimethomorph, flumorph, pyrimorph, benthiavalicarb, benthiavalicarb-isopropyl, iprovalicarb, valifenalate, and mandipropamid
(9) Melanin biosynthesis inhibitors:
   (a) Reductase inhibitors in melanin biosynthesis: fthalide, pyroquilon, and tricyclazole;
   (b) Dehydratase inhibitors in melanin biosynthesis: carpropamid, diclocymet, and fenoxanil; and
   (c) Polyketide synthase inhibitors in melanin biosynthesis: tolprocarb
(10) Host plant resistance inducers:
   (a) Agents acting on the salicylic acid synthesis pathway: acibenzolar-S-methyl; and
   (b) Other agents: probenazole, tiadinil, isotianil, diclobentiazox, ipfentrifluconazole, laminarin, giant knotweed extract, phosphorous acid, and phosphites
(11) Agents of unknown mode of action: cymoxanil, fosetyl-aluminum, phosphoric acid, phosphates, tecloftalam, triazoxide, flusulfamide, diclomezine, methasulfocarb, cyflufenamid, metrafenone, pyriofenone, dodine, dodine free base, and flutianil
(12) Agents having multiple points of action: copper and copper salts, Bordeaux mixture, copper hydroxide, copper naphthenate, copper oxide, copper oxychloride, copper sulfate, sulfur, sulfur products, calcium polysulfide, ferbam, mancozeb, maneb, mancopper, metiram, polycarbamate, propineb, thiram, zineb, ziram, captan, captafol, folpet, chlorothalonil, dichlofluanid, tolylfluanid, guazatine, guazatine triacetate (also known as iminoctadine triacetate), iminoctadine tris(albesilate), anilazine, dithianon, quinomethionate, and fluoroimide
(13) Other agents: DBEDC, fluorofolpet, guazatine acetate, bis(8-quinolinolato)copper(II), propamidine, chloropicrin, cyproflam, Agrobacterium, bethoxazin, diphenylamine, methyl isothiocyanate (MITC), mildiomycin, capsaicin, cufraneb, cyprosulfamide, dazomet, debacarb, dichlorophen, difenzoquat, difenzoquat methylsulfonate, flumetover, fosetyl-calcium, fosetyl-sodium, irumamycin, natamycin, nitrothal-isopropyl, oxamocarb, pyrrolnitrin, tebufloquin, tolnifanide, zarilamide, Algophase, amicarthiazol, oxathiapiprolin, fluoxapiprolin, zinc metiram, benthiazole, triclamide, uniconazole, oxyfenthiin, picarbutrazox, diclobentiazox, quinofumelin, thiuram, ambam, Agrobacterium radiobacter, Coniothyrium minitans, Pseudomonas fluorescens, Pseudomonas rhodesia, Talaromyces flavus, Trichoderma atroviride, non-pathogenic Erwinia carotovora, Bacillus simplex, Variovorax paradoxus, Lactobacillus plantarum, florylpicoxamid, pyrapropoyne, fluindapyr, aminopyrifen, pyridachlometyl, ipflufenoquin, and dipymetitrone;
   flufenoxadiazam, flumetylsulforim, and feneptamidquin;

Specific examples of insecticides, acaricides, nematicides, soil pest control agents, anthelmintics, and the like, as the active ingredient (II) that can be mixed with or used in combination with the agricultural and horticultural fungicide according to the embodiment of the present invention, are shown below.
(1) Acetylcholinesterase (AChE) inhibitors:
   (a) Carbamate-based acetylcholinesterase (AChE) inhibitors:
      alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC, xylylcarb, phenothiocarb, MIPC, MPMC, MTMC, aldoxycarb, alixycarb, aminocarb, bufencarb, cloethocarb, metam-sodium, and promecarb; and
   (b) Organophosphorus acetylcholinesterase (AChE) inhibitors:
      acephate, azamethiphos, azinphos-ethyl, azinphos-methyl, cadusafos, chlorethoxyphos, chlorfenvinphos, chlormephos, chlorpyrifos, chlorpyrifos-methyl, coumaphos, cyanophos, demeton-S-methyl, diazinon, dichlorvos, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isocarbophos, isopropyl=O-(methoxyaminothiophosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion, bromophos-ethyl, BRP, carbophenothion, cyanofenphos, CYAP, demeton-S-methylsulfone, dialifos, dichlofenthion, dioxabenzofos, etrimfos, fensulfothion, flupyrazofos, fonofos, formothion, phosmethylan, isazofos, iodofenphos, metacrifos, pirimiphos-ethyl, phosphocarb, propaphos, prothoate, and sulprofos
(2) GABA-gated chloride ion channel blockers:
   acetoprole, chlordane, endosulfan, ethiprole, fipronil, pyrafluprole, pyriprole, camphechlor, heptachlor, dienochlor, and flufiprole
(3) Sodium channel modulators:
   (a) Pyrethroid sodium channel modulators:
      acrinathrin, allethrin, d-cis-trans-allethrin, d-trans-allethrin, bifenthrin, bioallethrin, bioallethrin-S-cyclopentenyl isomer, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin [(1R)-trans isomer], deltamethrin, empenthrin [(EZ)-(1R)-isomer], esfenvalerate, etofenprox, fenpropathrin, fenvalerate, flucythrinate, flumethrin, tau-fluvalinate, halfenprox, imiprothrin, kadethrin, permethrin, phenothrin [(1R)-trans isomer], prallethrin, pyrethlam, pyrethrin, resmethrin, silafluofen, tefluthrin, tetramethrin, tetramethrin [(1R)-isomer], tralomethrin, transfluthrin, allethrin, pyrethrin I, pyrethrin II, profluthrin, dimefluthrin, bioethanomethrin, biopermethrin, transpermethrin, fenfluthrin, fenpyrithrin, flubrocythrinate, flufenprox, metofluthrin, protriphenbut, pyresmethrin, terallethrin, kappa-bifenthrin, chloroprallethrin, heptafluthrin, meperfluthrin, epsilon-metofluthrin, momfluorothrin, epsilon-momfluorothrin, kappa-tefluthrin, tetramethylfluthrin, and bioethanomethrin; and
   (b) Sodium channel modulators (DDTs):
      DDT and methoxychlor
(4) Nicotinic acetylcholine receptor (nAChR) competitive modulators:
   acetamiprid, clothianidin, dinotefuran, imidacloprid, nitenpyram, nithiazine, thiacloprid, thiamethoxam, nicotine, sulfoxaflor, flupyradifurone, triflumezopyrim, dichloromezotiaz, and flupyrimin
(5) Nicotinic acetylcholine receptor (nAChR) allosteric modulators:
   spinetoram and spinosad
(6) Glutamate-gated chloride ion channel (GluCl) allosteric modulators:
   abamectin, emamectin, emamectin benzoate, lepimectin, milbemectin, ivermectin, selamectin, doramectin, eprinomectin, ivermectin, moxidectin, selamectin, milbemycin, milbemycin oxime, and nemadectin
(7) Juvenile hormone mimics:
   hydroprene, kinoprene, methoprene, fenoxycarb, pyriproxyfen, diofenolan, epofenonane, and triprene
(8) Other non-specific (multi-site) inhibitors:
   methyl bromide, alkyl halides, chloropicrin, sodium aluminum fluoride, sulfuryl fluoride, borax, boric acid, disodium octaborate, sodium borate, sodium metaborate, tartar emetic, dazomet, metam, metam-potassium, and metam-sodium
(9) Chordotonal organ TRPV channel modulators:
   flonicamid, pymetrozine, pyrifluquinazon, and afidopyropen
(10) Mite growth inhibitors:
   clofentezine, diflovidazin, hexythiazox, and etoxazole
(11) Microbially derived disruptors of insect midgut membranes:
   Bacillus thuringiensis subsp. israelensis, Bacillus sphaericus, Bacillus thuringiensis subsp. aizawai, Bacillus thuringiensis subsp. kurstaki, Bacillus thuringiensis subsp. tenebrionis, proteins contained in Bt crops: Cry1Ab, Cry1Ac, Cry1Fa, Cry1A.105, Cry2Ab, Vip3A, mCry3A, Cry3Ab, Cry3Bb, Cry34Ab1/Cry35Ab1; and Bacillus sphaericus
(12) Mitochondrial ATP synthase inhibitors:
   diafenthiuron, azocyclotin, cyhexatin, fenbutatin oxide, propargite, and tetradifon
(13) Oxidative phosphorylation uncouplers that disrupt the proton gradient:
   chlorfenapyr, DNOC, sulfluramid, binapacryl, dinobuton, and dinocap
(14) Nicotinic acetylcholine receptor (nAChR) channel blockers:
   bensultap, cartap hydrochloride, nereistoxin, thiocyclam, and thiosultap-sodium
(15) Chitin biosynthesis inhibitors, type 0:
   bistrifluron, chlorfluazuron, diflubenzuron, flucycloxuron, flufenoxuron, hexaflumuron, lufenuron, novaluron, noviflumuron, teflubenzuron, triflumuron, buprofezin, and fluazuron
(16) Chitin biosynthesis inhibitors, type 1:
   buprofezin
(17) Molting inhibitors:
   cyromazine
(18) Ecdysone receptor agonists:
   chromafenozide, halofenozide, methoxyfenozide, and tebufenozide
(19) Octopamine receptor agonists:
   amitraz, demiditraz, and chlordimeform
(20) Mitochondrial electron transport chain complex III inhibitors:
   hydramethylnon, acequinocyl, fluacrypyrim, and bifenazate
(21) Mitochondrial electron transport chain complex I inhibitors (METI):
   fenazaquin, fenpyroximate, pyridaben, pyrimidifen, tebufenpyrad, tolfenpyrad, and rotenone
(22) Voltage-dependent sodium channel blockers:
   indoxacarb and metaflumizone
(23) Acetyl-CoA carboxylase inhibitors:
   spirodiclofen, spiromesifen, spirotetramat, and spiropidion
(24) Mitochondrial electron transport chain complex IV inhibitors:
   aluminum phosphide, calcium phosphide, zinc phosphide, phosphine, cyanide, calcium cyanide, sodium cyanide, and potassium cyanide
(25) Mitochondrial electron transport chain complex II inhibitors:
   cyenopyrafen, cyflumetofen, and pyflubumide
(28) Ryanodine receptor modulators:
   chlorantraniliprole, cyantraniliprole, cyclaniliprole, flubendiamide, cyhalodiamide, tetrachlorantraniliprole, and tetraniliprole
(29) Chordotonal organ modulators of undefined target site:
   flonicamid
(30) GABA-gated chloride ion channel allosteric modulators:
   broflanilide, fluxametamide, isocycloseram, afoxolaner, fluralaner, lotilaner, and sarolaner
(31) Other insecticides and acaricides:
   azadirachtin, benzoximate, bifenazate, bromopropylate, chinomethionat, cryolite, dicofol, lime sulfur, mancozeb, pyridalyl, benclothiaz, sulfur, amidoflumet, 1,3-dichloropropene, DCIP, phenisobromolate, benzomate, metaldehyde, chlorobenzilate, clothiazoben, dicyclanil, phenoxacrim, fentrifanil, flubenzimine, flufenazine, gossyplure, japonilure, methoxyadiazone, petroleum (oil), potassium oleate, tetrasul, triarathene, afidopyropen, flufiprole, fluensulfone, meperfluthrin, tetramethylfluthrin, tralopyril, dimefluthrin, methylneodecanamide, fluralaner, afoxolaner, fluxametamide, 5-[5-(3,5-dichlorophenyl)-5-trifluoromethyl-4,5-dihydroisoxazol-3-yl]-2-(1H-1,2,4-triazol-1-yl)benzonitrile (CAS: 943137-49-3), broflanilide, other metadiamides, Steinernema carpocapsae, Steinernema glaseri, Pasteuria penetrans, Paecilomyces tenuipes, Paecilomyces fumosoroseus, Beauveria bassiana, Beauveria brongniartii, Metarhizium anisopliae, Verticillium lecanii, acynonapyr, benzpyrimoxan, flometoquin, fluhexafon, oxazosulfyl, and tyclopyrazoflor
(32) Anthelmintics:
   (a) Benzimidazoles: fenbendazole, albendazole, triclabendazole, oxybendazole, mebendazole, oxfendazole, parbendazole, flubendazole, febantel, netobimin, thiophanate, thiabendazole, and cambendazole;
   (b) Salicylanilides: closantel, oxyclozanide, rafoxanide, and niclosamide;
   (c) Substituted phenols: nitroxynil and nitroscanate;
   (d) Pyrimidines: pyrantel and morantel;
   (e) Imidazothiazoles: levamisole and tetramisole;
   (f) Tetrahydropyrimidines: praziquantel and epsiprantel; and
   (g) Other anthelmintics: cyclodiene, ryania, clorsulon, metronidazole, demiditraz, piperazine, diethylcarbamazine, dichlorophen, monepantel, tribendimidine, amidantel, thiacetarsamide, melarsomine, and arsenamide

Specific examples of plant growth regulators as the active ingredient (II) that can be mixed with or used in combination with the agricultural and horticultural fungicide according to the embodiment of the present invention are shown below.
abscisic acid, kinetin, benzylaminopurine, 1,3-diphenylurea, forchlorfenuron, thidiazuron, chlorfenuron, dihydrozeatin, gibberellin A, gibberellin A4, gibberellin A7, gibberellin A3, 1-methylcyclopropene, N-acetylaminoethoxyvinylglycine (also known as aviglycine), aminooxyacetic acid, silver nitrate, cobalt chloride, IAA, 4-CPA, cloprop, 2,4-D, MCPB, indole-3-butyric acid, dichlorprop, phenothiol, 1-naphthylacetamide, ethychlozate, cloxyfonac, maleic hydrazide, 2,3,5-triiodobenzoic acid, salicylic acid, methyl salicylate, (-)-jasmonic acid, methyl jasmonate, (+)-strigol, (+)-deoxystrigol, (+)-orobanchol, (+)-sorgolactone, 4-oxo-4-(2-phenylethyl)aminobutyric acid, ethephon, chlormequat, mepiquat chloride, benzyladenine, 5-aminolevulinic acid, and daminozide

When the active ingredient (Z-I) and the active ingredient (II) are mixed or used in combination, a synergistic effect may be exhibited. The synergistic effect can be confirmed by using Colby's formula.

The test method is shown below.

### {Antifungal test for gray mold (growth inhibition rate)}

Conidia of gray mold fungus (Botrytis cinerea) are added to and dispersed in a yeast extract-sucrose medium, and further, the active ingredient (Z-I) and the active ingredient (II) dissolved in dimethyl sulfoxide at predetermined concentrations are added and mixed. These are dispensed onto a 96-well microplate, and cultured in a dark place at 20°C for 3 to 5 days. After culturing, turbidity at a wavelength of 405 nm is measured with a microplate reader, and a growth inhibition rate (%) of the gray mold fungus compared to the case of no treatment is determined. The test is preferably performed in duplicates or more.

From the obtained results, an expected value is calculated using Colby's formula. $E = M + N - MN / 100$

Here, E represents the expected value (%) of the growth inhibition rate, M represents the growth inhibition rate (%) when the active ingredient (Z-I) is used alone, and N represents the growth inhibition rate (%) when the active ingredient (II) is used alone.

When the actually measured growth inhibition rate (%) of the composition of the active ingredient (Z-I) and the active ingredient (II) is larger than the expected value E, the composition of the active ingredient (Z-I) and the active ingredient (II) exhibits a synergistic effect.

### {Formulation preparations}

The agricultural and horticultural fungicide according to the embodiment of the present invention is not particularly limited in terms of formulation type. Examples thereof include a wettable powder, an emulsifiable concentrate, a dust, a granule, a watersoluble powder, a suspension concentrate, a water-dispersible granule, and a tablet. A method for preparing the formulation is not particularly limited, and a known preparation method can be used depending on the formulation type.

Some Formulation Examples are shown below. The formulation preparations shown below are merely illustrative and may be modified without departing from the spirit of the present invention, and the present invention is not limited in any way by the following formulation examples. "Part" means "part by mass" unless otherwise specified.

### (Formulation Example 1: wettable powder)

40 parts of the 5-membered heteroaryl compound according to the embodiment of the present invention, 53 parts of diatomaceous earth, 4 parts of a higher alcohol sulfate, and 3 parts of an alkyl naphthalenesulfonate are uniformly mixed and then finely pulverized to obtain a wettable powder containing 40% by mass of the active ingredient.

### (Formulation Example 2: emulsifiable concentrate)

30 parts of the 5-membered heteroaryl compound according to the embodiment of the present invention, 33 parts of xylene, 30 parts of dimethylformamide, and 7 parts of a polyoxyethylene alkyl aryl ether are mixed and dissolved to obtain an emulsifiable concentrate containing 30% by mass of the active ingredient.

### (Formulation Example 3: granules)

5 parts of the 5-membered heteroaryl compound according to the embodiment of the present invention, 40 parts of talc, 38 parts of clay, 10 parts of bentonite, and 7 parts of a sodium alkyl sulfate are uniformly mixed, then finely pulverized, and granulated to a particle diameter of 0.5 to 1.0 mm to obtain granules containing 5% by mass of the active ingredient.

### (Formulation Example 4: granules)

5 parts of the 5-membered heteroaryl compound according to the embodiment of the present invention, 73 parts of clay, 20 parts of bentonite, 1 part of sodium dioctyl sulfosuccinate, and 1 part of potassium phosphate are uniformly mixed and pulverized, and then water is added and the mixture is kneaded. Thereafter, the kneaded product is granulated and dried to obtain granules containing 5% by mass of the active ingredient.

### (Formulation Example 5: suspension concentrate)

10 parts of the 5-membered heteroaryl compound according to the embodiment of the present invention, 4 parts of a polyoxyethylene alkyl aryl ether, 2 parts of sodium polycarboxylate, 10 parts of glycerin, 0.2 parts of xanthan gum, and 73.8 parts of water are mixed and wet-pulverized until a particle size of 3 µm or less is obtained, thereby obtaining a suspension concentrate containing 10% by mass of the active ingredient.

### (Formulation Example 6: water-dispersible granule)

40 parts of the 5-membered heteroaryl compound according to the embodiment of the present invention, 36 parts of clay, 10 parts of potassium chloride, 1 part of a sodium alkylbenzenesulfonate, 8 parts of sodium ligninsulfonate, and 5 parts of a formaldehyde condensate of a sodium alkylbenzenesulfonate are uniformly mixed and finely pulverized. Next, an appropriate amount of water is added thereto, and the mixture is kneaded into a clay-like mass. The clay-like mass is granulated and then dried to obtain a water-dispersible granule containing 40% by mass of the active ingredient.

Next, compound production examples are shown to describe the present invention more specifically. However, the present invention is not limited to the following examples.

### [Production Example 1]

1-(tert-Butyl)-4-(3-(trifluoromethyl)phenoxy)-1H-1,2,3-triazole-5-carboxylic acid (compound number: f-3) was produced by the following steps.

### Step 1: conversion to 4,5-dibromo-1-(tert-butyl)-1H-1,2,3-triazole

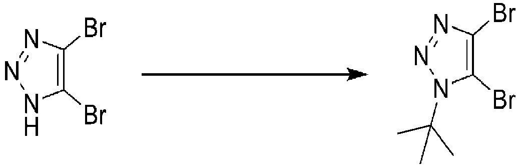

4,5-Dibromo-1H-1,2,3-triazole (3.4 g), tert-butanol (2.2 g), and p-toluenesulfonic acid monohydrate (0.57 g) were added to toluene (50 mL), and the mixture was stirred at 70°C for 3 hours.

After the solvent was removed, the residue was purified by silica gel column chromatography to obtain 4,5-dibromo-1-(tert-butyl)-1H-1,2,3-triazole (1.8 g).

### Step 2: conversion to 4-bromo-1-(tert-butyl)-1H-1,2,3-triazole-5-carboxylic acid

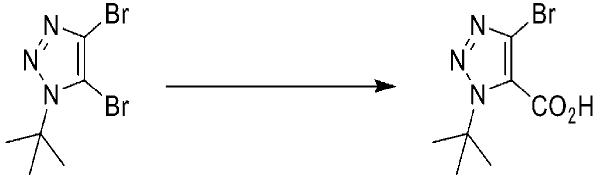

4,5-Dibromo-1-(tert-butyl)-1H-1,2,3-triazole (1.7 g) was dissolved in tetrahydrofuran (15 mL) at 0°C, isopropylmagnesium chloride-lithium chloride complex (1.4 g) was added thereto, and the mixture was stirred for 2 hours. Next, carbon dioxide (10 g) was bubbled through the reaction solution.

The reaction was quenched with 1 M HCl and then extracted with ethyl acetate, and the solvent was removed to obtain 4-bromo-1-(tert-butyl)-1H-1,2,3-triazole-5-carboxylic acid (1.5 g).

### Step 3: conversion to isopropyl 4-bromo-1-(tert-butyl)-1H-1,2,3-triazole-5-carboxylate

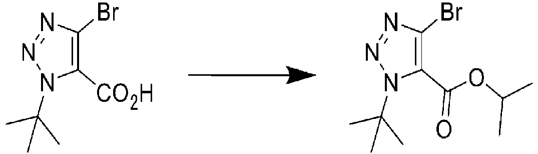

4-Bromo-1-(tert-butyl)-1H-1,2,3-triazole-5-carboxylic acid (1.5 g), isopropyl iodide (3.1 g), and potassium carbonate (2.5 g) were added to N,N-dimethylformamide (20 mL), and the mixture was stirred at 45°C for 3 hours.

Water was added to the reaction solution, the mixture was extracted with ethyl acetate, and the solvent was removed. The residue was purified by silica gel column chromatography to obtain isopropyl 4-bromo-1-(tert-butyl)-1H-1,2,3-triazole-5-carboxylate (1.4 g, yield: 80%).

### Step 4: conversion to isopropyl 1-(tert-butyl)-4-(3-(trifluoromethyl)phenoxy)-1H-1,2,3-triazole-5-carboxylate

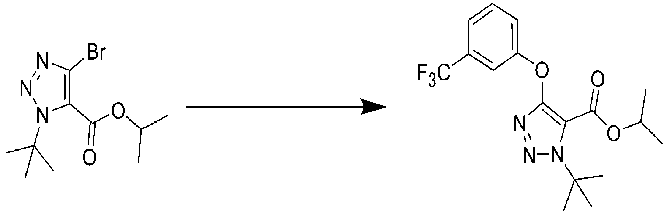

α,α,α-Trifluoro-m-cresol (0.32 g), isopropyl 4-bromo-1-(tert-butyl)-1H-1,2,3-triazole-5-carboxylate (0.29 g), N,N-dimethylglycine (21 mg), and copper(I) iodide (19 mg) were added to toluene (2.5 mL). The mixture was irradiated with microwaves at 150°C for 1.5 hours.

The residue was purified by silica gel column chromatography to obtain isopropyl 1-(tert-butyl)-4-(3-(trifluoromethyl)phenoxy)-1H-1,2,3-triazole-5-carboxylate (0.20 g).

### Step 5: conversion to 1-(tert-butyl)-4-(3-(trifluoromethyl)phenoxy)-1H-1,2,3-triazole-5-carboxylic acid

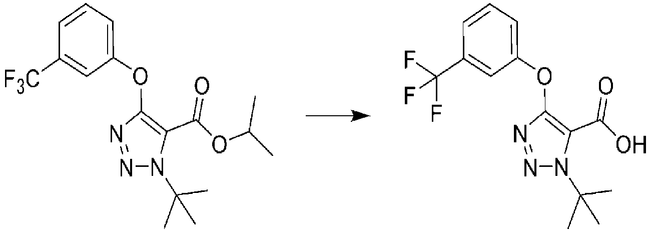

Isopropyl 1-(tert-butyl)-4-(3-(trifluoromethyl)phenoxy)-1H-1,2,3-triazole-5-carboxylate (0.20 g) was dissolved in tetrahydrofuran (0.50 mL), water (0.17 mL), and methanol (0.50 mL). Lithium hydroxide monohydrate (73 mg) was added thereto, and the mixture was stirred at 25°C for 16 hours.

The reaction was quenched with 1 N hydrochloric acid, the mixture was extracted with ethyl acetate, and the organic layer was dried over magnesium sulfate. The solvent was removed to obtain 1-(tert-butyl)-4-(3-(trifluoromethyl)phenoxy)-1H-1,2,3-triazole-5-carboxylic acid (0.12 g).

### [Production Example 2]

1-(tert-Butyl)-4-(3-chlorophenoxy)-N-((2,4-dimethylbenzyl)oxy)-1H-1,2,3-triazole-5-carboxamide (compound number: d-44) was produced by the following steps.

### Step 1: conversion to 4-bromo-1-(tert-butyl)-1H-1,2,3-triazole-5-carboxamide

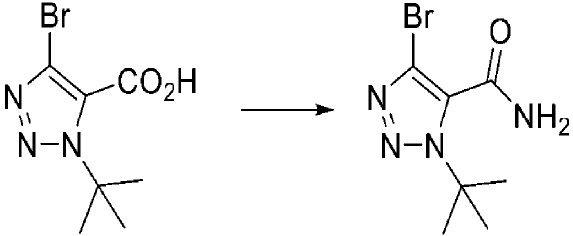

4-Bromo-1-(tert-butyl)-1H-1,2,3-triazole-5-carboxylic acid (0.50 g) was dissolved in N,N-dimethylformamide (6.7 mL). Ammonium chloride (0.21 g), 1-[bis(dimethylamino)methylene]-1H-1,2,3-triazolo[4,5-b]pyridinium 3-oxide hexafluorophosphate (HATU) (1.1 g), and triethylamine (0.61 g) were added thereto.

Water was added to the reaction solution, and the mixture was extracted with ethyl acetate. The obtained organic layer was concentrated with an evaporator, and the residue was purified by silica gel column chromatography to obtain 4-bromo-1-(tert-butyl)-1H-1,2,3-triazole-5-carboxamide (0.36 g, yield: 73%).

### Step 2: conversion to 4-bromo-1-(tert-butyl)-1H-1,2,3-triazole-5-carbonitrile

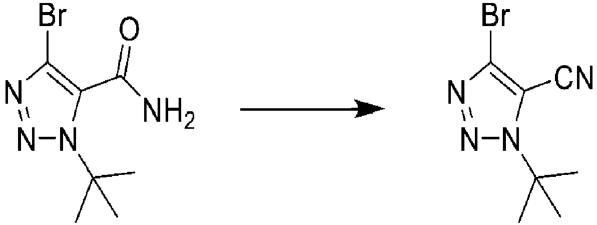

4-Bromo-1-(tert-butyl)-1H-1,2,3-triazole-5-carboxamide (0.050 g) was dissolved in dichloromethane (0.67 mL). Trifluoroacetic anhydride (0.13 g) and triethylamine (72 mg) were added thereto, and the mixture was stirred at 25°C for 1 hour.

The reaction was quenched by adding a saturated sodium bicarbonate aqueous solution to the reaction solution, and then the mixture was extracted with ethyl acetate. The obtained organic layer was concentrated with an evaporator. The residue was fractionated by silica gel column chromatography and then purified by amino silica gel column chromatography to obtain 4-bromo-1-(tert-butyl)-1H-1,2,3-triazole-5-carbonitrile (0.039 g, yield: 84%).

### Step 3: conversion to 1-(tert-butyl)-4-(3-chlorophenoxy)-1H-1,2,3-triazole-5-carbonitrile

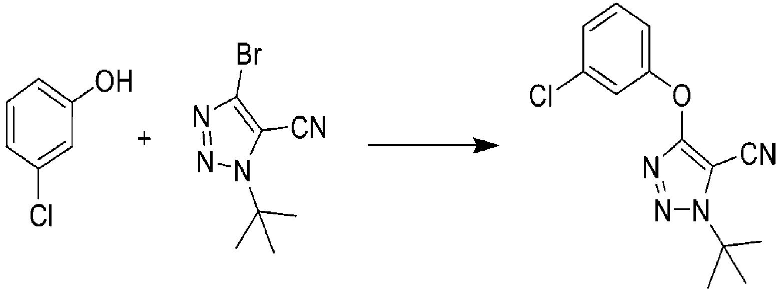

1-Chloro-3-hydroxybenzene (73 mg), 4-bromo-1-(tert-butyl)-1H-1,2,3-triazole-5-carbonitrile (0.10 g), and potassium carbonate (90 mg) were added to dimethyl sulfoxide (2.2 mL), and the mixture was stirred at 120°C for 4 hours.

Water was added to the reaction solution, and the mixture was extracted with ethyl acetate. The obtained organic layer was concentrated with an evaporator. The residue was purified by silica gel chromatography to obtain 1-(tert-butyl)-4-(3-chlorophenoxy)-1H-1,2,3-triazole-5-carbonitrile (0.09 g, yield: 70%).

### Step 4: conversion to 1-(tert-butyl)-4-(3-chlorophenoxy)-1H-1,2,3-triazole-5-carboxamide

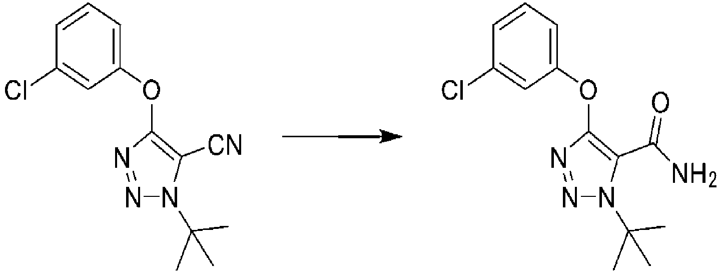

1-(tert-Butyl)-4-(3-chlorophenoxy)-1H-1,2,3-triazole-5-carbonitrile (0.15 g) and aqueous sodium hydroxide (4 M, 2.7 mL) were added to ethanol (2.7 mL), and the mixture was stirred at 100°C for 12 hours.

The reaction solution was quenched with 1 N hydrochloric acid, and then extracted with ethyl acetate. The obtained organic layer was dried over magnesium sulfate. The solvent was removed with an evaporator to obtain 1-(tert-butyl)-4-(3-chlorophenoxy)-1H-1,2,3-triazole-5-carboxamide (0.16 g).

### Step 5: conversion to 1-(tert-butyl)-4-(3-chlorophenoxy)-1H-1,2,3-triazole-5-carboxylic acid

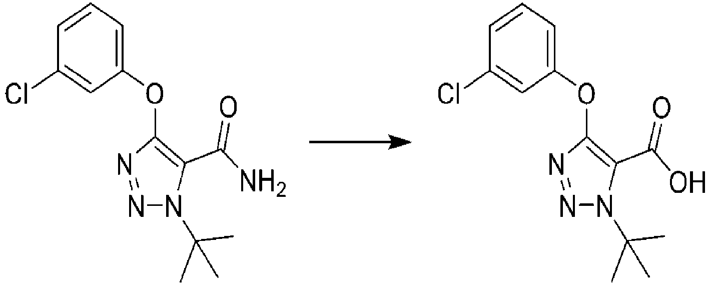

1-(tert-Butyl)-4-(3-chlorophenoxy)-1H-1,2,3-triazole-5-carboxamide (0.16 g) and sodium nitrite (0.11 g) were added to trifluoroacetic acid (1.8 mL) at 0°C, and the mixture was stirred at 0°C for 2 hours.

Water was added to the reaction solution, and then the mixture was extracted with ethyl acetate. The obtained organic layer was dried over magnesium sulfate. The solvent was removed with an evaporator to obtain 1-(tert-butyl)-4-(3-chlorophenoxy)-1H-1,2,3-triazole-5-carboxylic acid (0.13 g, yield: 81%).

### Step 6: conversion to 1-(tert-butyl)-4-(3-chlorophenoxy)-N-((2,4-dimethylbenzyl)oxy)-1H-1,2,3-triazole-5-carboxamide

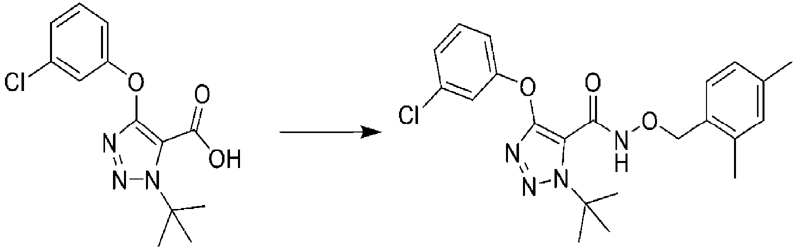

1-(tert-Butyl)-4-(3-chlorophenoxy)-1H-1,2,3-triazole-5-carboxylic acid (41 mg), O-(2,4-dimethylbenzyl)hydroxylamine hydrochloride (32 mg), 1-[bis(dimethylamino)methylene]-1H-1,2,3-triazolo[4,5-b]pyridinium 3-oxide hexafluorophosphate (HATU) (80 mg), and triethylamine (42 mg) were added to tetrahydrofuran (0.70 mL), and the mixture was stirred at 25°C for 16 hours.

The reaction solution was purified by preparative HPLC (developing solvent: water/acetonitrile) to obtain 1-(tert-butyl)-4-(3-chlorophenoxy)-N-((2,4-dimethylbenzyl)oxy)-1H-1,2,3-triazole-5-carboxamide (0.044 g, yield: 73%).

### [Production Example 3]

1-(tert-Butyl)-4-(3-chlorophenoxy)-N-((2-methoxy-4-methylbenzyl)oxy)-1H-1,2,3-triazole-5-carboxamide (compound number: d-72) was produced by the following steps.

### Step 1: conversion to 4-bromo-1-(tert-butyl)-1H-1,2,3-triazole-5-carbaldehyde

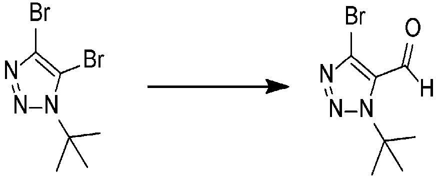

4,5-Dibromo-1-(tert-butyl)-1H-1,2,3-triazole (2.0 g) was dissolved in tetrahydrofuran (24 mL). At 0°C, isopropylmagnesium chloride-lithium chloride complex (1.3 M, 8.7 mL) was added thereto, and the mixture was stirred at 0°C for 2 hours. N,N-Dimethylformamide (5.2 g) was added to the mixture at -40°C, and stirred at -40°C for 1 hour. The reaction was quenched with dilute hydrochloric acid, the mixture was extracted with ethyl acetate, and the extract layer was washed with brine. Thereafter, the extract layer was dried over anhydrous magnesium sulfate, and the solvent was removed in vacuo to obtain 4-bromo-1-(tert-butyl)-1H-1,2,3-triazole-5-carbaldehyde (1.6 g, yield: 88%).

### Step 2: conversion to 1-(tert-butyl)-4-(3-chlorophenoxy)-1H-1,2,3-triazole-5-carbaldehyde

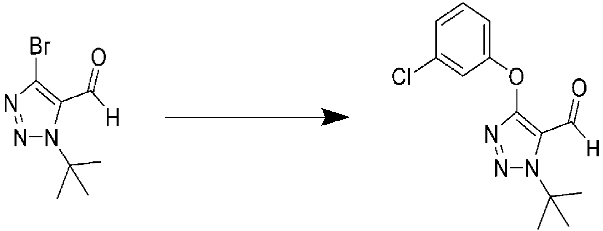

4-Bromo-1-(tert-butyl)-1H-1,2,3-triazole-5-carbaldehyde (0.050 g) was dissolved in N,N-dimethylformamide (0.7 mL). 3-Chlorophenol (0.055 g), copper (0.002 g), copper iodide (0.042 g), and potassium carbonate (0.12 g) were added thereto, and the mixture was stirred at 135°C for 2 hours. The reaction solution was purified by preparative high-performance liquid chromatography (developing solvent: acetonitrile/water) to obtain 1-(tert-butyl)-4-(3-chlorophenoxy)-1H-1,2,3-triazole-5-carbaldehyde (0.01 g, 16%).

### Step 3: conversion to 1-(tert-butyl)-4-(3-chlorophenoxy)-1H-1,2,3-triazole-5-carboxylic acid

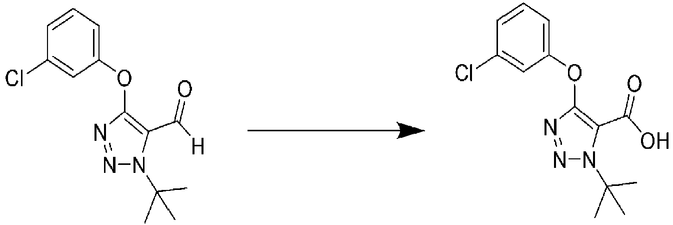

1-(tert-Butyl)-4-(3-chlorophenoxy)-1H-1,2,3-triazole-5-carbaldehyde (0.01 g) was dissolved in acetone (0.2 mL) and water (0.1 mL). Potassium permanganate (0.003 g) was added thereto, and the mixture was stirred at 25°C for 1 hour.

The reaction solution was quenched by adding 1 N hydrochloric acid aqueous solution, and then extracted with ethyl acetate. The obtained organic layer was concentrated with an evaporator to obtain 1-(tert-butyl)-4-(3-chlorophenoxy)-1H-1,2,3-triazole-5-carboxylic acid (0.01 g, yield: 100%).

### Step 4: conversion to 1-(tert-butyl)-4-(3-chlorophenoxy)-N-((2-methoxy-4-methylbenzyl)oxy)-1H-1,2,3-triazole-5-carboxamide

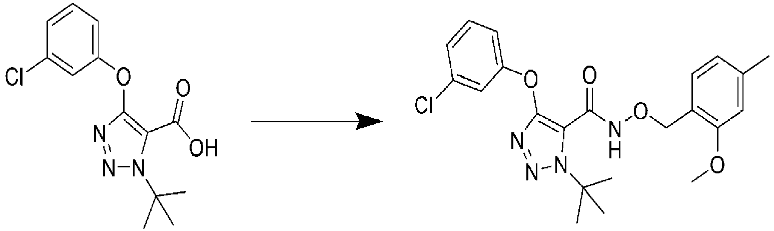

1-(tert-Butyl)-4-(3-chlorophenoxy)-1H-1,2,3-triazole-5-carboxylic acid (0.002 g), O-(2-methoxy-4-methylbenzyl)hydroxylamine (0.001 g), 1-[bis(dimethylamino)methylene]-1H-1,2,3-triazolo[4,5-b]pyridinium 3-oxide hexafluorophosphate (HATU) (0.002 g), and triethylamine (0.002 mL) were added to dichloromethane (0.70 mL), and the mixture was stirred at 25°C for 16 hours.

The reaction solution was purified by preparative high-performance liquid chromatography (developing solvent: acetonitrile/water) to obtain 1-(tert-butyl)-4-(3-chlorophenoxy)-N-((2-methoxy-4-methylbenzyl)oxy)-1H-1,2,3-triazole-5-carboxamide (0.002 g, 70%).

### [Production Example 4]

1-(tert-Butyl)-4-((6-chloropyridin-2-yl)oxy)-N-((2,4-dimethylbenzyl)oxy)-1H-1,2,3-triazole-5-carboxamide (compound number: d-108) was produced by the following steps.

### Step 1: conversion to 4-(benzyloxy)-1-(tert-butyl)-1H-1,2,3-triazole-5-carbonitrile

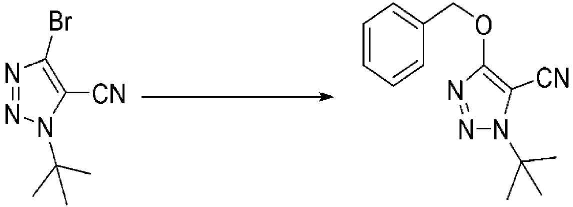

4-Bromo-1-(tert-butyl)-1H-1,2,3-triazole-5-carbonitrile (1.5 g) was dissolved in 1-methyl-2-pyrrolidone (22 mL). At room temperature, benzyl alcohol (4.3 g) and cesium carbonate (10.7 g) were added thereto, and the mixture was stirred at 125°C for 2 hours. Water was added to the reaction solution, the mixture was extracted with ethyl acetate, and the extracted phase was washed with brine. Thereafter, the extracted phase was dried over anhydrous magnesium sulfate, concentrated in vacuo, and purified by silica gel column chromatography (developing solvent: n-hexane/ethyl acetate) to obtain 4-(benzyloxy)-1-(tert-butyl)-1H-1,2,3-triazole-5-carbonitrile (0.15 g, yield: 9%).

### Step 2: conversion to 4-(benzyloxy)-1-(tert-butyl)-1H-1,2,3-triazole-5-carboxamide

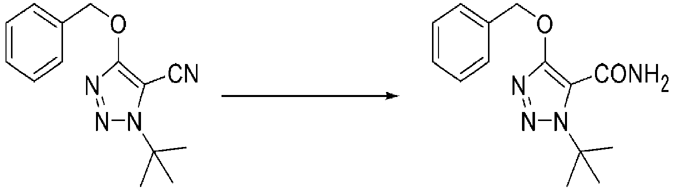

4-(Benzyloxy)-1-(tert-butyl)-1H-1,2,3-triazole-5-carbonitrile (0.15 g) was dissolved in ethanol (3.1 mL). Sodium hydroxide (0.25 g) was added thereto, and the mixture was heated to reflux for 1 hour.

1 N hydrochloric acid was added to the reaction solution, the mixture was extracted with ethyl acetate, and the extracted phase was washed with brine. Thereafter, the extracted phase was dried over anhydrous magnesium sulfate, and the solvent was removed in vacuo to obtain 4-(benzyloxy)-1-(tert-butyl)-1H-1,2,3-triazole-5-carboxamide (0.17 g, yield: 99%).

### Step 3: conversion to 4-(benzyloxy)-1-(tert-butyl)-1H-1,2,3-triazole-5-carboxylic acid

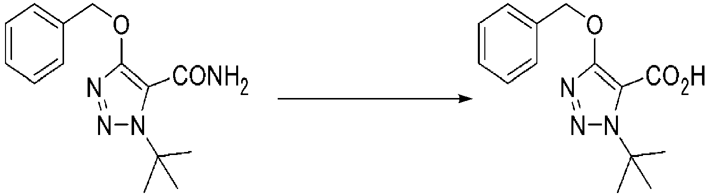

4-(Benzyloxy)-1-(tert-butyl)-1H-1,2,3-triazole-5-carboxamide (0.15 g) was dissolved in trifluoroacetic acid (1.1 mL). Sodium nitrite (0.11 g) was added thereto at 0°C, and the mixture was stirred at 0°C for 1 hour.

The reaction solution was quenched by adding water and then extracted with ethyl acetate. The obtained organic layer was concentrated with an evaporator to obtain 4-(benzyloxy)-1-(tert-butyl)-1H-1,2,3-triazole-5-carboxylic acid (0.12 g, yield: 80%).

### Step 4: conversion to isopropyl 4-(benzyloxy)-1-(tert-butyl)-1H-1,2,3-triazole-5-carboxylate

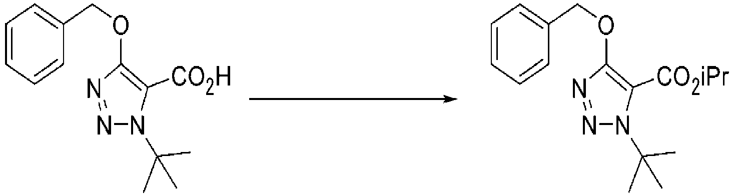

4-(Benzyloxy)-1-(tert-butyl)-1H-1,2,3-triazole-5-carboxylic acid (0.12 g), isopropyl iodide (0.19 g), and cesium carbonate (0.36 g) were added to N,N-dimethylformamide (1.7 mL), and the mixture was stirred at 35°C for 2 hours.

Water was added to the reaction solution, the mixture was extracted with ethyl acetate, and the extracted phase was washed with brine. Thereafter, the extracted phase was dried over anhydrous magnesium sulfate, concentrated in vacuo, and purified by silica gel column chromatography (developing solvent: n-hexane/ethyl acetate) to obtain isopropyl 4-(benzyloxy)-1-(tert-butyl)-1H-1,2,3-triazole-5-carboxylate (0.12 g, 69%).

### Step 5: conversion to isopropyl 1-(tert-butyl)-4-hydroxy-1H-1,2,3-triazole-5-carboxylate

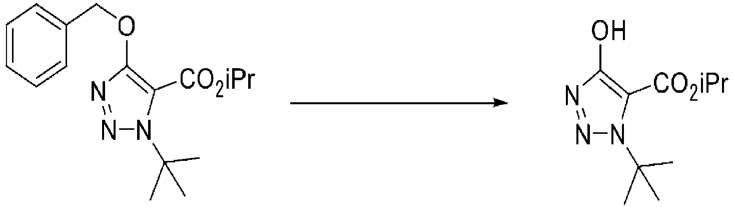

Isopropyl 4-(benzyloxy)-1-(tert-butyl)-1H-1,2,3-triazole-5-carboxylate (0.12 g) was dissolved in tetrahydrofuran (1.7 mL). At room temperature, 10% palladium on activated carbon (0.04 g) was added thereto, and the mixture was stirred under a hydrogen atmosphere for 18 hours.

The reaction solution was filtered through Celite, and the solvent was removed in vacuo to obtain isopropyl 1-(tert-butyl)-4-hydroxy-1H-1,2,3-triazole-5-carboxylate (0.05 g, 58%).

### Step 6: conversion to isopropyl 1-(tert-butyl)-4-((6-chloropyridin-2-yl)oxy)-1H-1,2,3-triazole-5-carboxylate

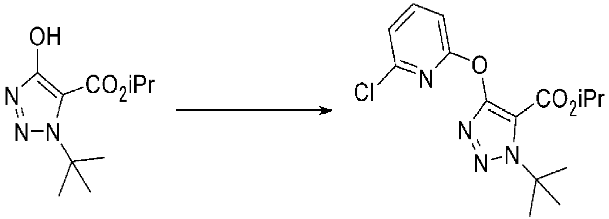

Isopropyl 1-(tert-butyl)-4-hydroxy-1H-1,2,3-triazole-5-carboxylate (0.04 g) was dissolved in N,N-dimethylformamide (1.8 mL). At room temperature, 6-chloro-2-fluoropyridine (0.19 g) and potassium carbonate (0.19 g) were added thereto, and the mixture was stirred at 120°C for 2 hours.

Water was added to the reaction solution, the mixture was extracted with ethyl acetate, and the extracted phase was washed with brine. Thereafter, the extracted phase was dried over anhydrous magnesium sulfate, concentrated in vacuo, and purified by silica gel column chromatography (developing solvent: n-hexane/ethyl acetate) to obtain isopropyl 1-(tert-butyl)-4-((6-chloropyridin-2-yl)oxy)-1H-1,2,3-triazole-5-carboxylate (0.035 g, 50%).

### Step 7: conversion to 1-(tert-butyl)-4-((6-chloropyridin-2-yl)oxy)-1H-1,2,3-triazole-5-carboxylic acid

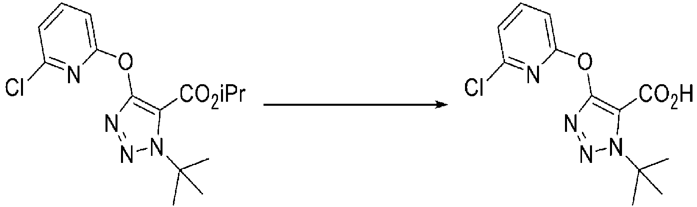

Isopropyl 1-(tert-butyl)-4-((6-chloropyridin-2-yl)oxy)-1H-1,2,3-triazole-5-carboxylate (0.035 g) was dissolved in tetrahydrofuran (1.2 mL), methanol (0.4 mL), and water (0.4 mL). At room temperature, lithium hydroxide monohydrate (0.043 g) was added thereto, and the mixture was stirred at room temperature for 18 hours.

1 N hydrochloric acid was added to the reaction solution, the mixture was extracted with ethyl acetate, and the extracted phase was washed with brine. Thereafter, the organic phase was dried over anhydrous magnesium sulfate, and the solvent was removed in vacuo to obtain 1-(tert-butyl)-4-((6-chloropyridin-2-yl)oxy)-1H-1,2,3-triazole-5-carboxylic acid (0.03 g, yield: 98%).

### Step 8: conversion to 1-(tert-butyl)-4-((6-chloropyridin-2-yl)oxy)-N-((2,4-dimethylbenzyl)oxy)-1H-1,2,3-triazole-5-carboxamide

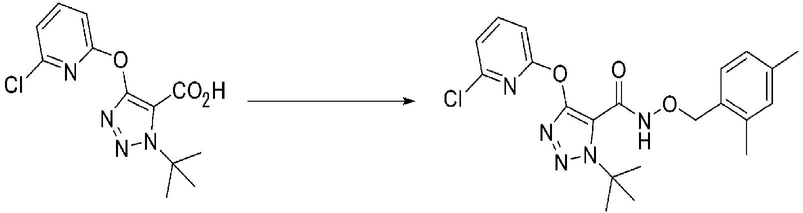

1-(tert-Butyl)-4-((6-chloropyridin-2-yl)oxy)-1H-1,2,3-triazole-5-carboxylic acid (0.008 g), O-(2,4-dimethylbenzyl)hydroxylamine hydrochloride (0.009 g), 1-[bis(dimethylamino)methylene]-1H-1,2,3-triazolo[4,5-b]pyridinium 3-oxide hexafluorophosphate (HATU) (0.021 g), and triethylamine (0.012 mL) were added to tetrahydrofuran (0.70 mL), and the mixture was stirred at 25°C for 16 hours.

The reaction solution was purified by silica gel column chromatography (developing solvent: n-hexane/ethyl acetate) to obtain 1-(tert-butyl)-4-((6-chloropyridin-2-yl)oxy)-N-((2,4-dimethylbenzyl)oxy)-1H-1,2,3-triazole-5-carboxamide (0.0087 g, 75%).

Compounds shown in the first table were produced through the same steps as in the above-described production examples. The melting point or the appearance (AMORPHOUS or VISCOUS OIL) is noted in the column of characteristic. For those having an appearance of AMORPHOUS or VISCOUS OIL, LCMS retention times are noted.

The LCMS (SQD2) retention time was determined by the following method.

Measurement was performed using a system including an ultra-performance liquid chromatograph (ACQUITY UPLC H-Class, manufactured by Waters Corporation), an HPLC/UHPLC detector [ACQUITY UPLC Photodiode Array (PDA) eλ Detector (manufactured by Waters Corporation)], and a single quadrupole MS detector (LC/MS) [SQ Detector 2 (positive and negative ion electrospray modes and atmospheric pressure chemical ionization mode)], equipped with a CORTECS UPLC C18 column (manufactured by Waters Corporation, 2.1 x 50 mm, 1.6 µm), at a temperature of 40°C, a flow rate of 0.6 mL/min, and an injection volume of 2 µL. Water containing 0.1% by mass of formic acid was used as a mobile phase (A), and acetonitrile was used as a mobile phase (B). The concentration of the mobile phase (B) was maintained at 30% by mass for 0.15 minutes; thereafter, the concentration of the mobile phase (B) was linearly increased from 30% by mass to 95% by mass over 1.35 minutes, maintained at 95% by mass for 0.5 minutes, then the concentration of the mobile phase (B) was immediately decreased to 30% by mass, and finally the concentration of the mobile phase (B) was maintained at 30% by mass for 0.50 minutes.

The LCMS (QDa) retention time was determined by the following method.

Measurement was performed using a system comprising an ultra-performance liquid chromatograph (ACQUITY UPLC H-Class, manufactured by Waters Corporation), an HPLC/UHPLC detector [ACQUITY UPLC Photodiode Array (PDA) eλ Detector (manufactured by Waters Corporation)], and a single quadrupole MS detector (LC/MS) [ACQUITY QDa Detector (positive and negative ion electrospray modes)], equipped with a CORTECS UPLC C18 column (manufactured by Waters Corporation, 2.1 x 50 mm, 1.6 µm), at a temperature of 40°C, a flow rate of 0.6 mL/min, and an injection volume of 2 µL. Water containing 0.1% by mass of formic acid was used as a mobile phase (A), and acetonitrile was used as a mobile phase (B). The concentration of the mobile phase (B) was maintained at 30% by mass for 0.15 minutes, linearly increased from 30% by mass to 95% by mass over 1.35 minutes, maintained at 95% by mass for 0.5 minutes, then the concentration of the mobile phase (B) was immediately decreased to 30% by mass, and finally the concentration of the mobile phase (B) was maintained at 30% by mass for 0.50 minutes.

**Table 1**

| Compound number | Structural formula | Characteristic | LCMS (SQD2) retention time | LCMS (QDa) retention time |
|---|---|---|---|---|
| d-35 | 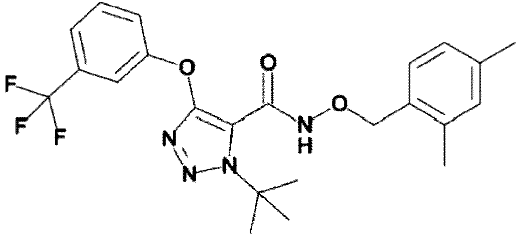 | VISCOUS OIL | 1.89 min | |
| d-36 | 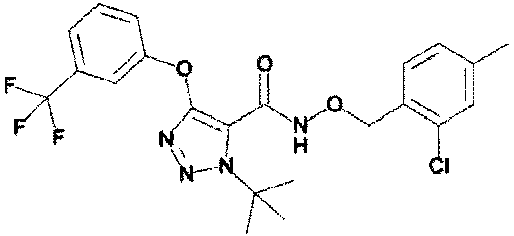 | VISCOUS OIL | 1.89 min | |
| d-44 | 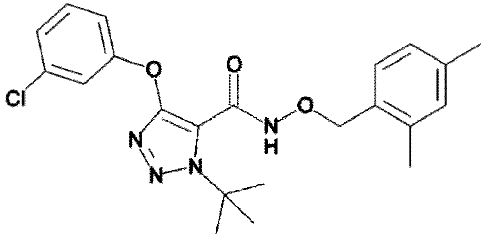 | AMORPHOUS | 1.85 min | |
| d-45 | 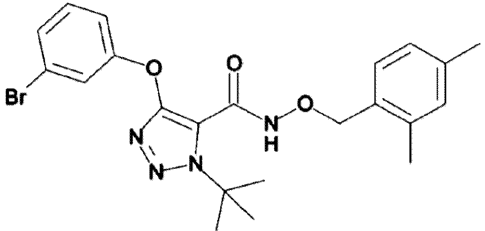 | AMORPHOUS | 1.88 min | |
| d-46 | 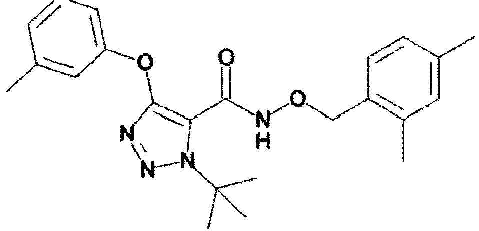 | AMORPHOUS | 1.84 min | |
| d-47 | 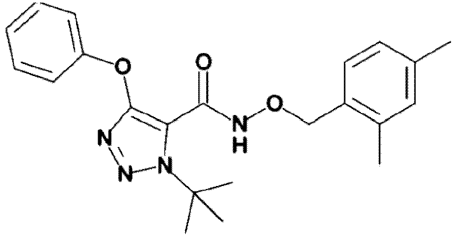 | AMORPHOUS | 1.76 min | |
| d-48 | 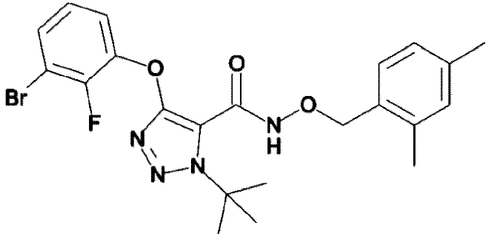 | AMORPHOUS | 1.88 min | |
| d-53 | 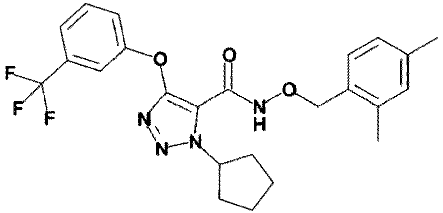 | VISCOUS OIL | 2.02 min | |
| d-54 | 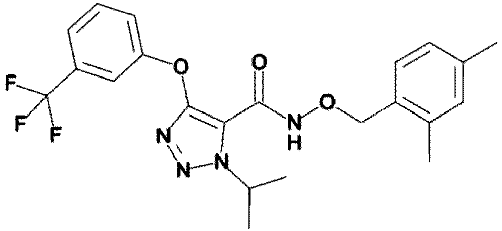 | VISCOUS OIL | 1.91 min | |
| d-55 | 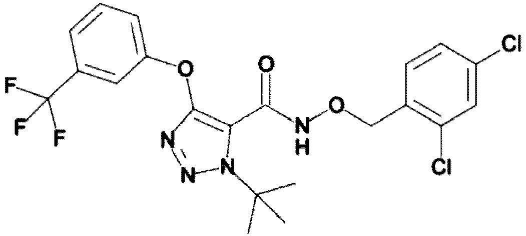 | AMORPHOUS | 1.92 min | |
| d-56 | 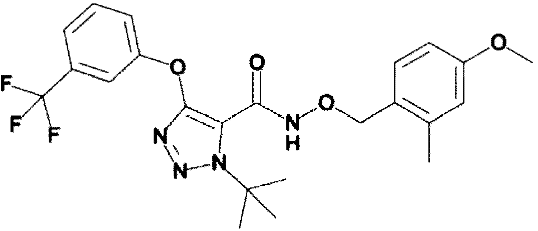 | VISCOUS OIL | 1.77 min | |
| d-57 | 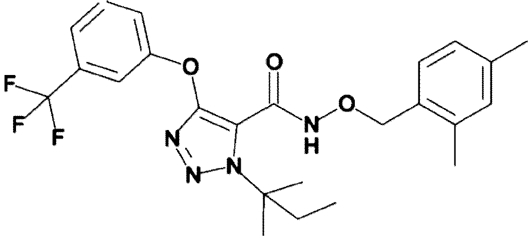 | m.p. 92°C to 94°C | | |
| d-58 | 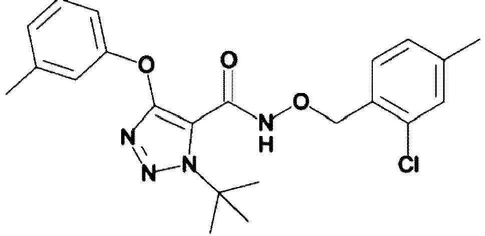 | m.p. 52°C to 55°C | | |
| d-59 | 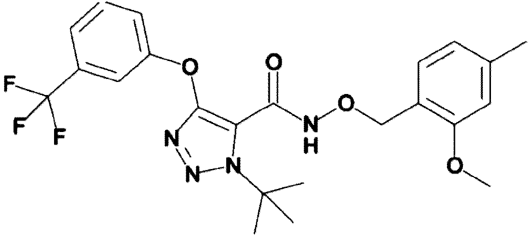 | VISCOUS OIL | | 1.79 min |
| d-60 | 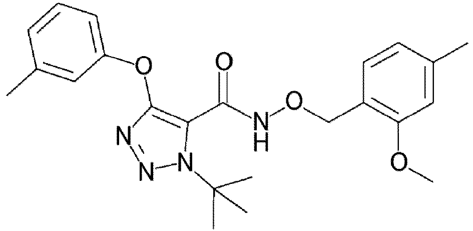 | VISCOUS OIL | | 1.73 min |
| d-61 | 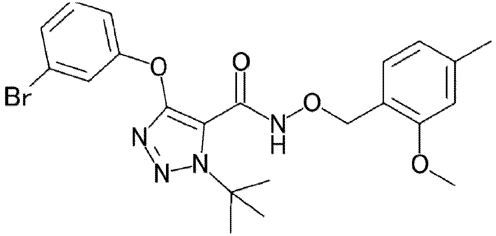 | VISCOUS OIL | | 1.79 min |
| d-63 | 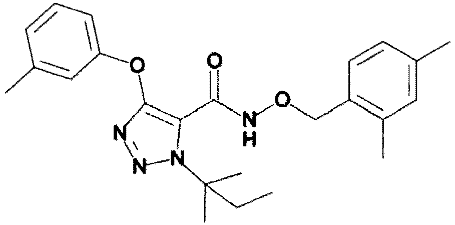 | m.p. 110°C to 112°C | | |
| d-66 | 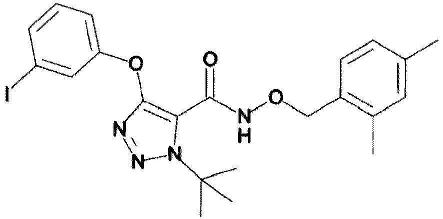 | VISCOUS OIL | 1.91 min | |
| d-67 | 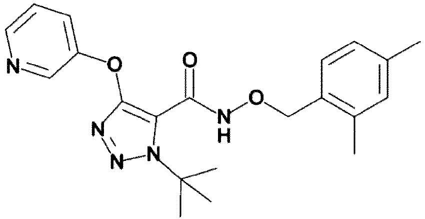 | VISCOUS OIL | 1.44 min | |
| d-68 | 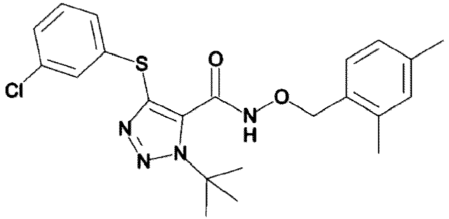 | VISCOUS OIL | 1.83 min | |
| d-69 | 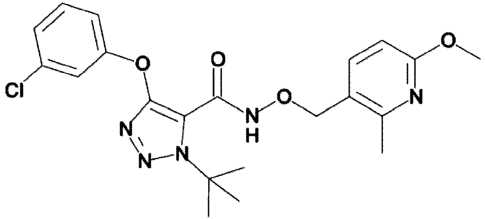 | AMORPHOUS | 1.58 min | |
| d-70 | 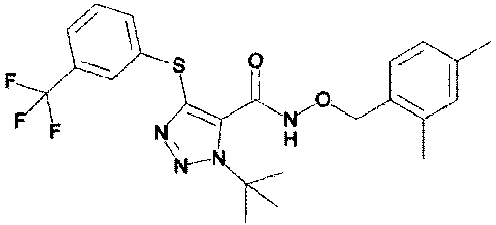 | VISCOUS OIL | 1.89 min | |
| d-71 | 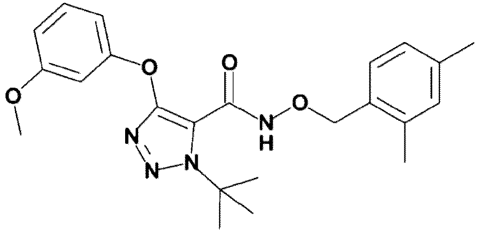 | VISCOUS OIL | 1.73 min | |
| d-72 | 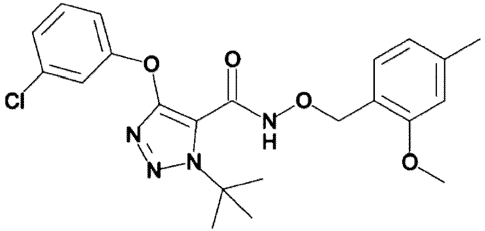 | VISCOUS OIL | 1.76 min | |
| d-73 | 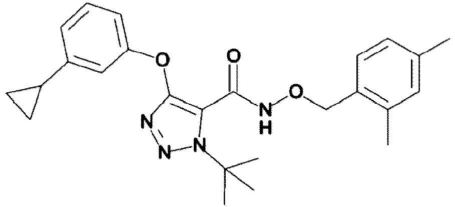 | VISCOUS OIL | 1.86 min | |
| d-74 | 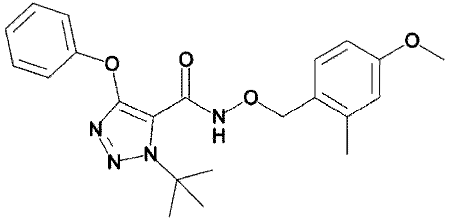 | VISCOUS OIL | 1.59 min | |
| d-75 | 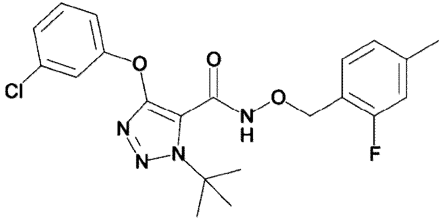 | VISCOUS OIL | 1.74 min | |
| d-76 | 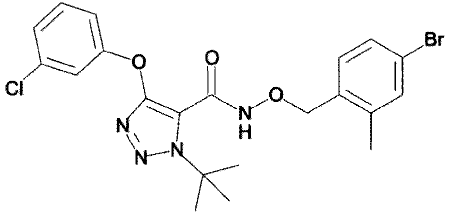 | m.p. 110°C to 112°C | | |
| d-77 | 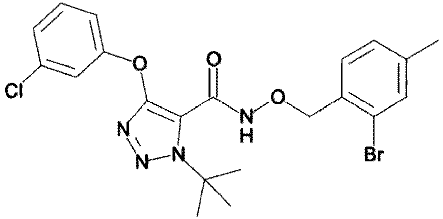 | VISCOUS OIL | 1.84 min | |
| d-78 | 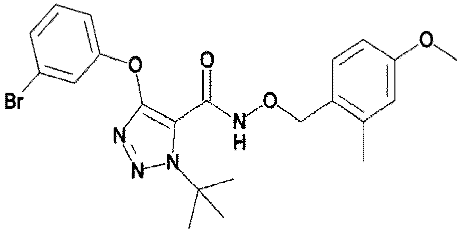 | VISCOUS OIL | 1.74 min | |
| d-79 | 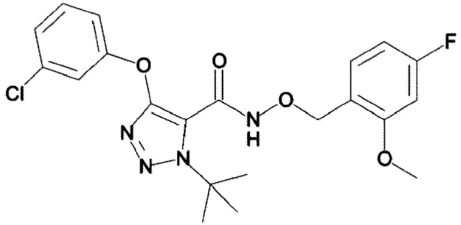 | AMORPHOUS | 1.66 min | |
| d-80 | 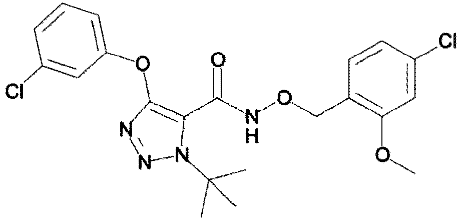 | AMORPHOUS | 1.77 min | |
| d-81 | 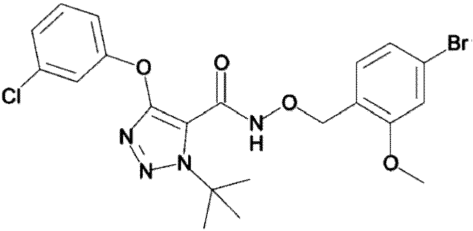 | AMORPHOUS | 1.79 min | |
| d-82 | 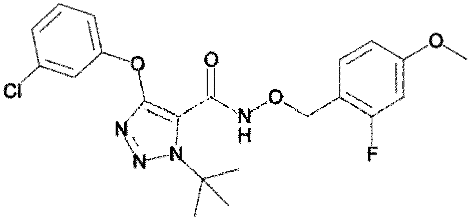 | VISCOUS OIL | 1.65 min | |
| d-83 | 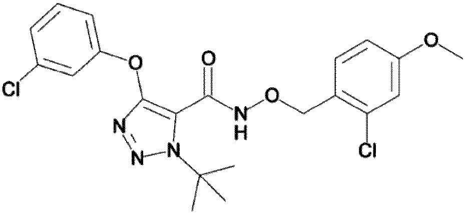 | AMORPHOUS | 1.72 min | |
| d-84 | 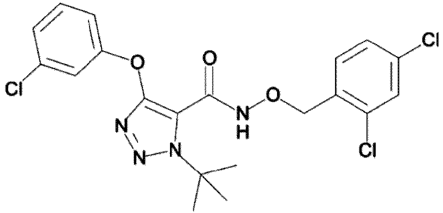 | VISCOUS OIL | 1.84 min | |
| d-85 | 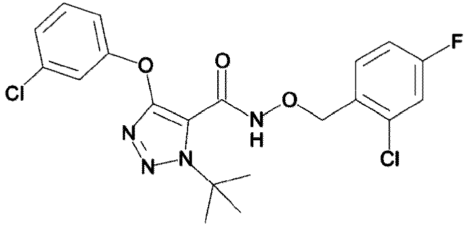 | VISCOUS OIL | 1.70 min | |
| d-86 | 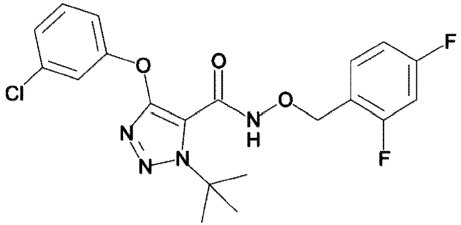 | VISCOUS OIL | 1.66 min | |
| d-87 | 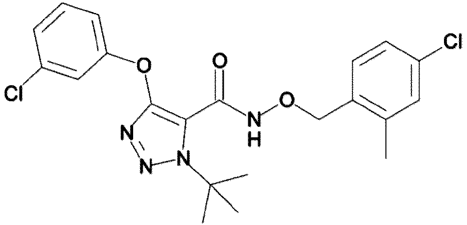 | VISCOUS OIL | 1.82 min | |
| d-88 | 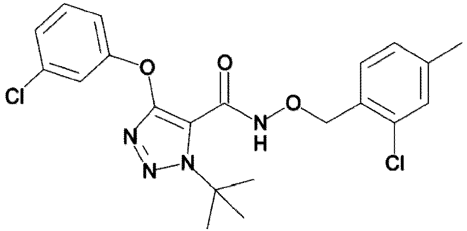 | VISCOUS OIL | 1.80 min | |
| d-89 | 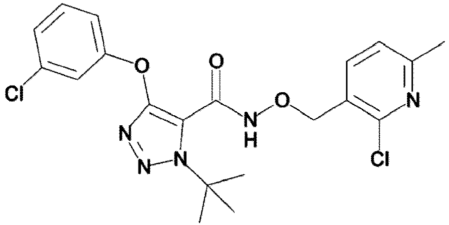 | AMORPHOUS | 1.55 min | |
| d-90 | 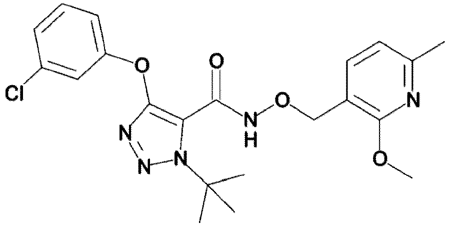 | VISCOUS OIL | 1.66 min | |
| d-91 | 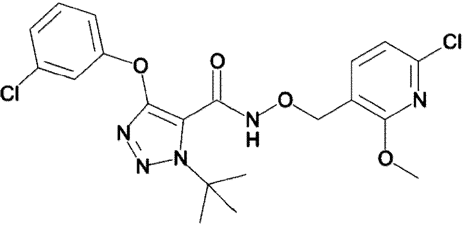 | AMORPHOUS | 1.72 min | |
| d-92 | 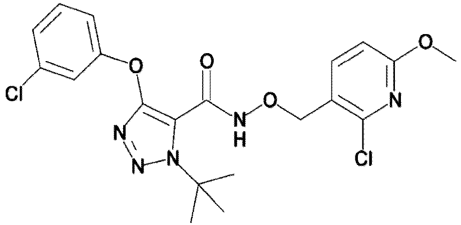 | AMORPHOUS | 1.66 min | |
| d-93 | 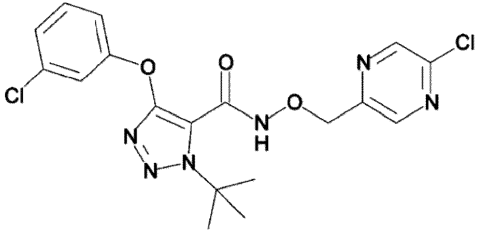 | VISCOUS OIL | 1.51 min | |
| d-94 | 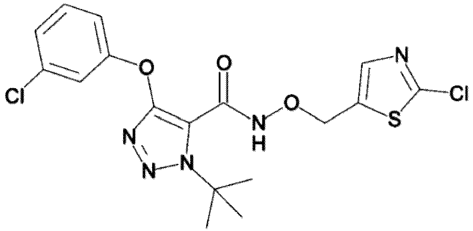 | VISCOUS OIL | 1.57 min | |
| d-95 | 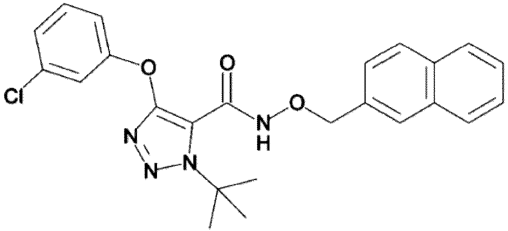 | VISCOUS OIL | 1.80 min | |
| d-96 | 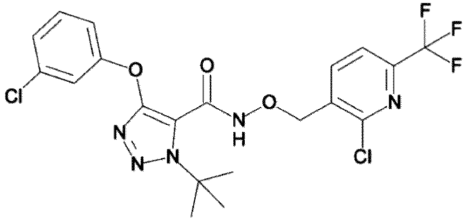 | m.p. 46°C to 52°C | | |
| d-97 | 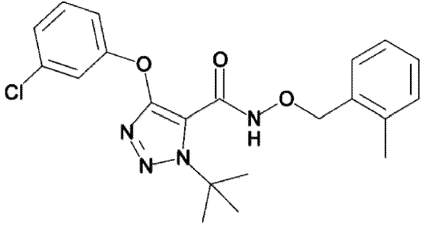 | VISCOUS OIL | 1.73 min | |
| d-98 | 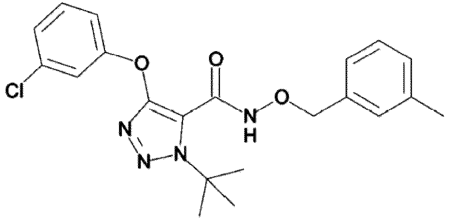 | VISCOUS OIL | 1.73 min | |
| d-99 | 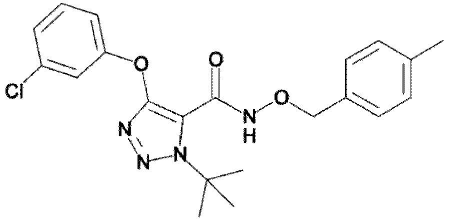 | VISCOUS OIL | 1.69 min | |
| d-100 | 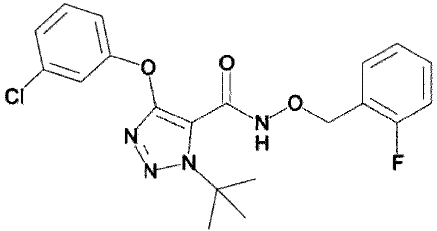 | VISCOUS OIL | 1.65 min | |
| d-101 | 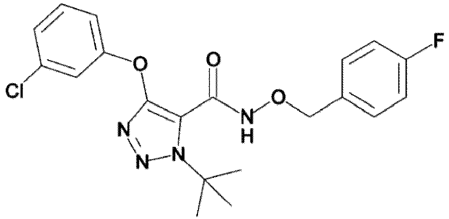 | AMORPHOUS | 1.64 min | |
| d-102 | 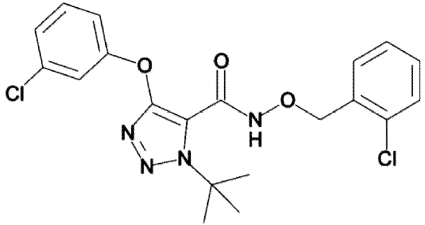 | VISCOUS OIL | 1.48 min | |
| d-103 | 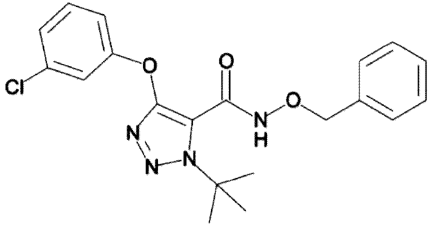 | VISCOUS OIL | 1.65 min | |
| d-104 | 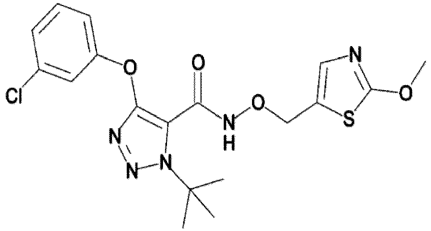 | VISCOUS OIL | 1.48 min | |
| d-105 | 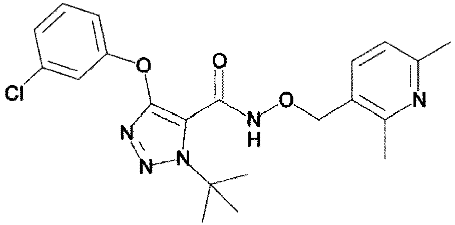 | AMORPHOUS | 0.50 min | |
| d-106 | 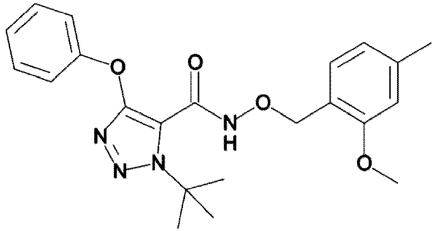 | VISCOUS OIL | 1.62 min | |
| d-107 | 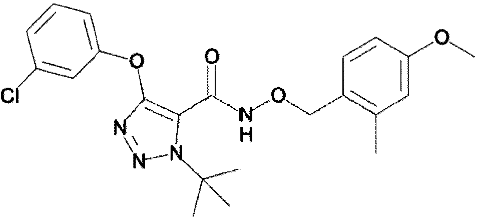 | VISCOUS OIL | 1.76 min | |
| d-108 | 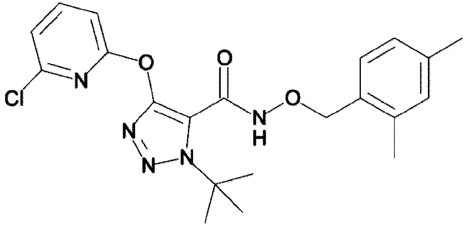 | VISCOUS OIL | 1.65 min | |
| d-109 | 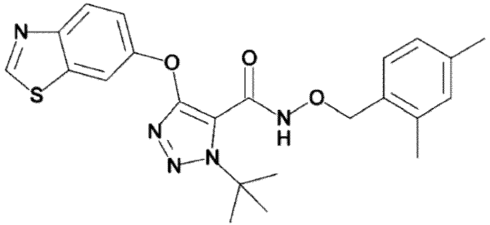 | VISCOUS OIL | 1.60 min | |
| d-110 | 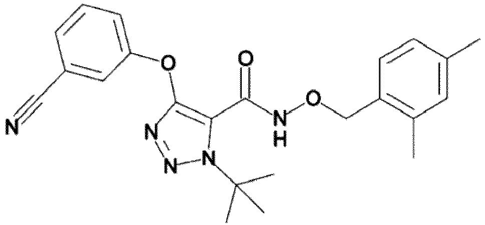 | VISCOUS OIL | 1.64 min | |
| d-111 | 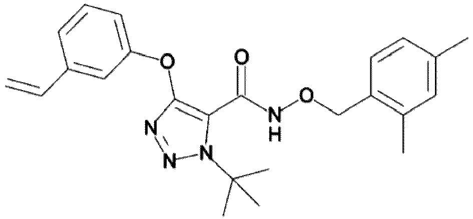 | VISCOUS OIL | 1.79 min | |
| d-112 | 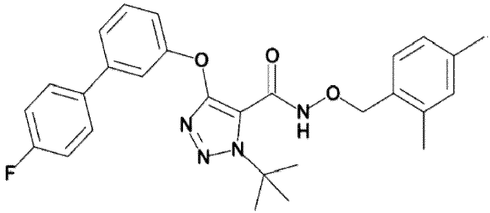 | VISCOUS OIL | 1.91 min | |
| d-113 | 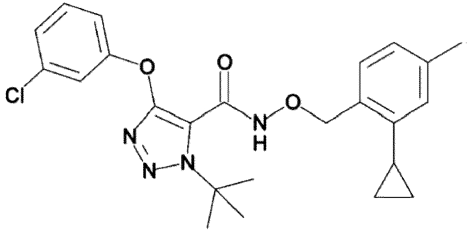 | VISCOUS OIL | 1.88 min | |

Compounds that can serve as production intermediates are shown in the second table and the third table. The LCMS (SQD2) retention time is noted together with the structural formula of the production intermediate.

**Table 2**

| Compound number | Structural formula | LCMS retention time |
|---|---|---|
| f-1 | 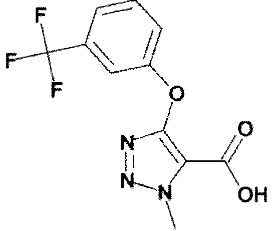 | 1.19 min |
| f-2 | 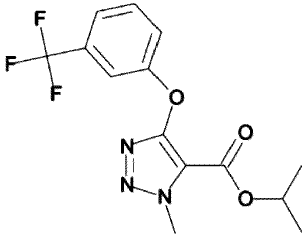 | 1.67 min |
| f-3 | 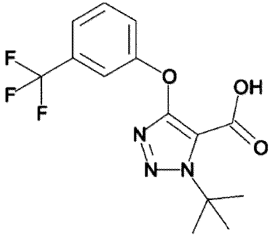 | 1.47 min |
| f-4 | 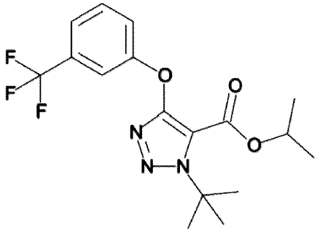 | 2.04 min |
| f-5 | 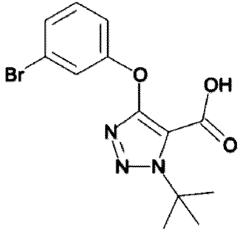 | 1.44 min |
| f-6 | 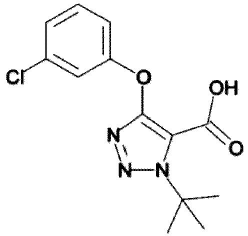 | 1.40 min |
| f-7 | 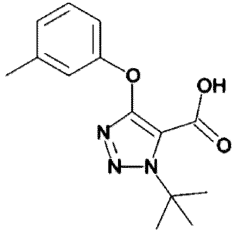 | 1.35 min |
| f-8 | 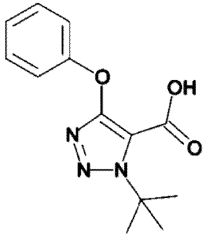 | 1.13 min |
| f-9 | 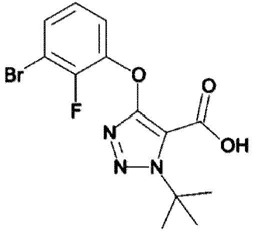 | 1.47 min |
| f-10 | 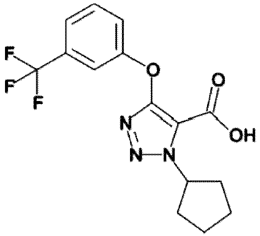 | 1.62 min |
| f-11 | 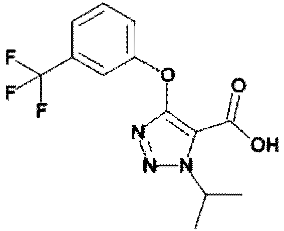 | 1.44 min |
| f-12 | 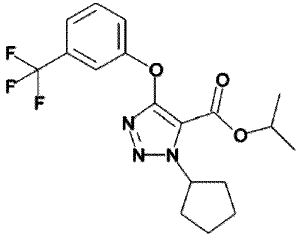 | 2.02 min |
| f-13 | 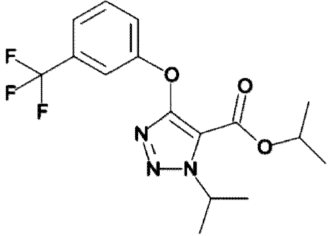 | 1.88 min |
| f-14 | 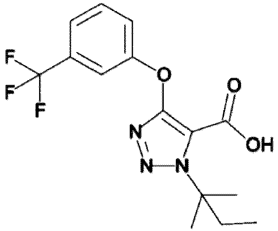 | 1.46 min (QDa) |
| f-15 | 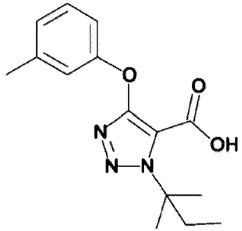 | 1.42 min |
| f-16 | 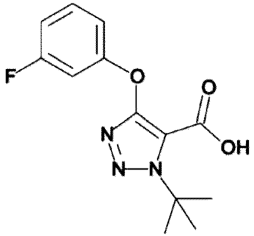 | 1.11 min (QDa) |
| f-17 | 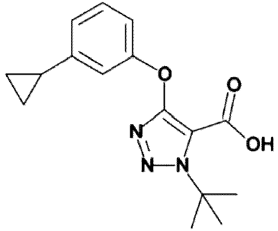 | 1.46 min |
| f-18 | 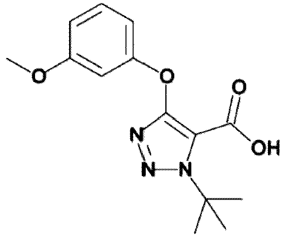 | 1.19 min |
| f-19 | 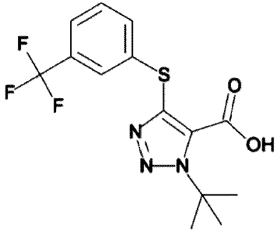 | 1.42 min |
| f-20 | 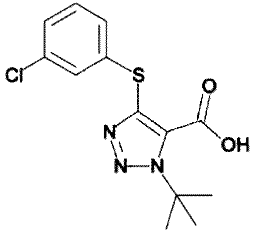 | 1.33 min |
| f-21 | 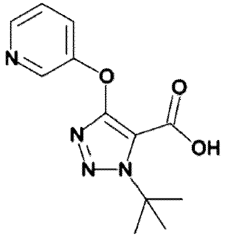 | 0.29 min |
| f-22 | 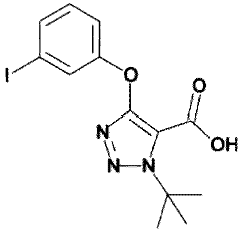 | 1.51 min |
| f-23 | 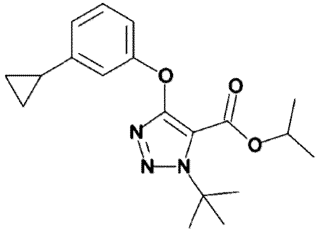 | 1.97 min |
| f-24 | 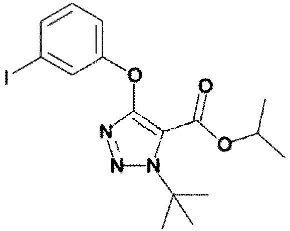 | 1.97 min |
| f-25 | 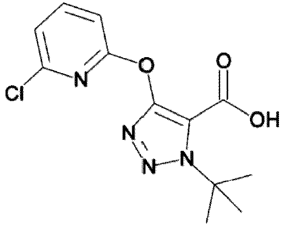 | 1.01 min |
| f-26 | 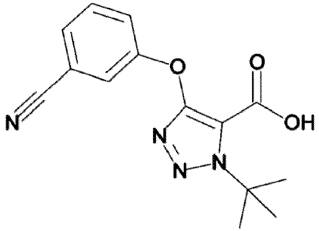 | 1.01 min |
| f-27 | 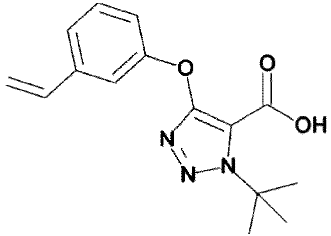 | 1.35 min |
| f-28 | 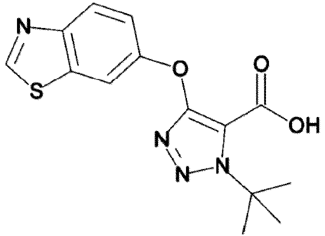 | 0.90 min |
| f-29 | 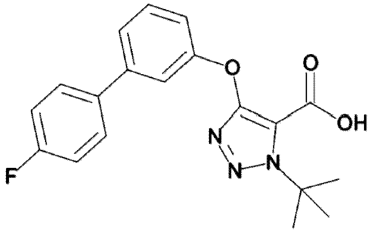 | 1.58 min |
| f-30 | 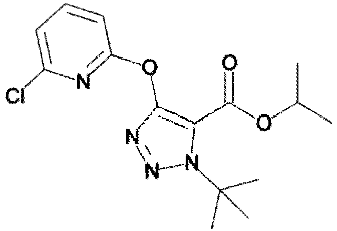 | 1.60 min |
| f-31 | 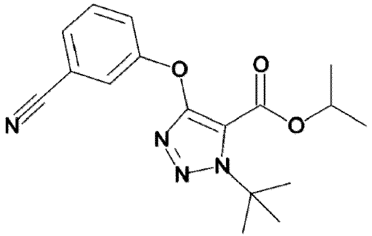 | 1.69 min |
| f-32 | 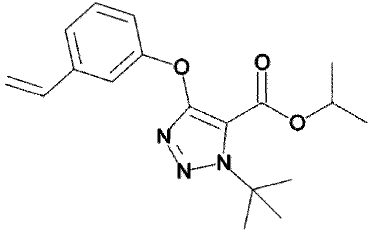 | 1.87 min |
| f-33 | 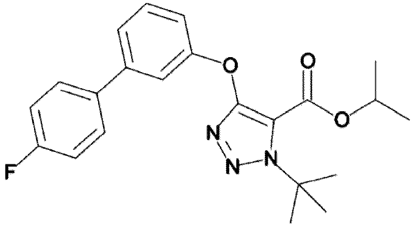 | 2.00 min |

**Table 3**

| Compound number | Structural formula | LCMS (SQD2) retention time |
|---|---|---|
| g-1 | 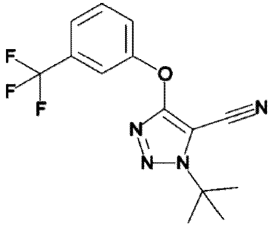 | 1.79 min |
| g-2 | 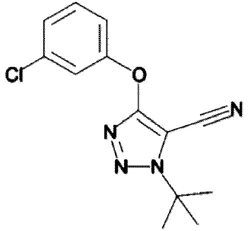 | 1.75 min |
| g-3 | 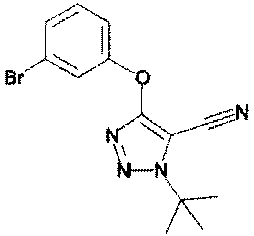 | 1.78 min |
| g-4 | 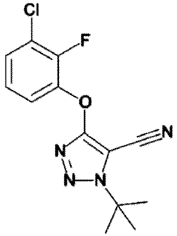 | 1.77 min |
| g-5 | 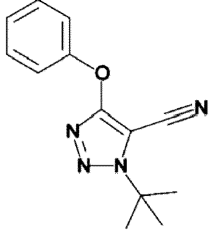 | 1.61 min |
| g-6 | 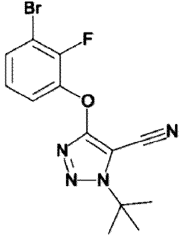 | 1.78 min |
| g-7 | 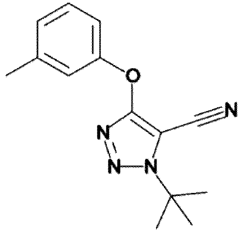 | 1.71 min |
| g-8 | 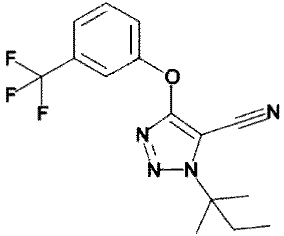 | 1.85 min |
| g-9 | 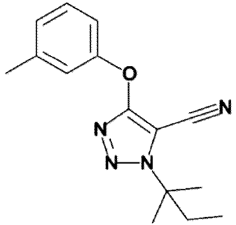 | 1.78 min |
| g-10 | 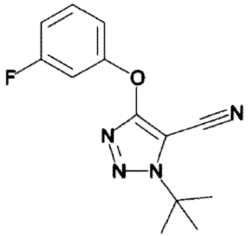 | 1.63 min |
| g-11 | 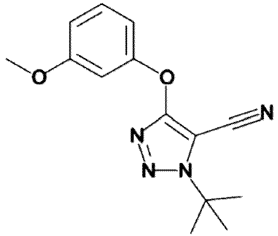 | 1.60 min |
| g-12 | 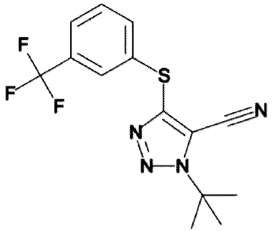 | 1.78 min |
| g-13 | 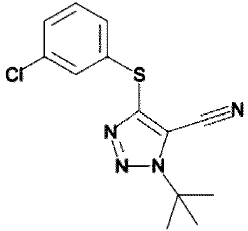 | 1.80 min |
| g-14 | 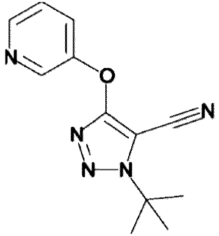 | 1.17 min |
| g-15 | 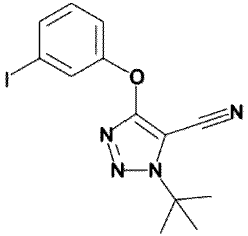 | 1.78 min |
| g-16 | 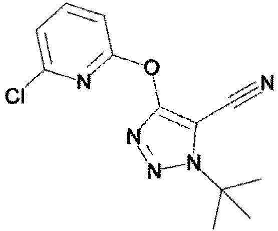 | 1.54 min |
| g-17 | 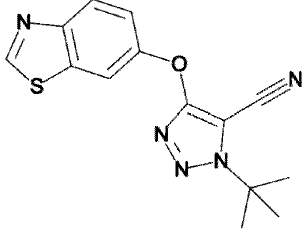 | 1.44 min |

The effects of the present invention are exemplified by the following fungicidal effect tests and disease control tests.

### (Fungicidal effect test against gray mold fungus)

A spore suspension of gray mold fungus (Botrytis cinerea, Vogel's medium) was prepared. The 5-membered heteroaryl compound according to the embodiment of the present invention was added to dimethyl sulfoxide to obtain a chemical solution. The chemical solution and the above-described spore suspension were mixed in equal amounts (based on volume) to obtain a test solution having a concentration of the 5-membered heteroaryl compound according to the embodiment of the present invention of 25 ppm by volume. The test solution was added dropwise to a 96-well microplate (treated plot).

Dimethyl sulfoxide and the above-described spore suspension were mixed in equal amounts (based on volume) to obtain a control solution. The control solution was added dropwise to a 96-well microplate (untreated control).

These were cultured by allowing them to stand in the dark at 20°C for 5 days. The turbidity OD at a wavelength of 405 nm was measured using a microplate reader. A growth inhibition rate (%) was calculated by the following formula. $Growth inhibition rate \left(%\right) = 100 - 100 x$
{[(OD of treated plot after culturing) - (OD of treated plot before culturing)]
/[(OD of untreated control after culturing) - (OD of untreated control before culturing)]}

The fungicidal effect test against the gray mold fungus was performed on the compounds of compound numbers d-35, d-36, d-44 to d-48, d-53 to d-61, d-63, d-66 to d-101, and d-103 to d-113. All of the compounds exhibited a growth inhibition rate of 75% or more.

### (Fungicidal effect test against grape ripe rot fungus)

The turbidity was measured and the growth inhibition rate was calculated in the same manner as in the fungicidal effect test against the gray mold fungus, except that the spore suspension of gray mold fungus (Botrytis cinerea, Vogel's medium) was changed to a spore suspension of grape ripe rot fungus (Glomerella cingulata, Vogel's medium), and the temperature and period during standing were changed to 25°C and 3 days.

The fungicidal effect test against the grape ripe rot fungus was performed on the compounds of compound numbers d-35, d-36, d-44 to d-48, d-53 to d-61, d-63, d-66 to d-101, and d-103 to d-113. All of the compounds exhibited a growth inhibition rate of 75% or more.

### (Fungicidal effect test against apple anthracnose fungus)

The turbidity was measured and the growth inhibition rate was calculated in the same manner as in the fungicidal effect test against the gray mold fungus, except that the spore suspension of gray mold fungus (Botrytis cinerea, Vogel's medium) was changed to a spore suspension of apple anthracnose fungus (Colletotrichum acutatum, Czapek medium), and the temperature and period during standing were changed to 25°C and 4 days.

The fungicidal effect test against the apple anthracnose fungus was performed on the compounds of compound numbers d-35, d-36, d-44 to d-48, d-53 to d-61, d-63, d-66 to d-92, d-95 to d-101, d-103 to d-108, and d-110 to d-113. All of the compounds exhibited a growth inhibition rate of 75% or more.

### (Fungicidal effect test against apple alternaria blotch fungus)

The turbidity was measured and the growth inhibition rate was calculated in the same manner as in the fungicidal effect test against the gray mold fungus, except that the spore suspension of gray mold fungus (Botrytis cinerea, Vogel's medium) was changed to a spore suspension of apple alternaria blotch fungus (Alternaria alternata, YBA medium), and the temperature during standing was changed to 25°C.

When the fungicidal effect test against the apple alternaria blotch fungus was performed on the compounds of compound numbers d-35, d-36, d-44 to d-48, d-53 to d-61, d-63, d-66 to d-92, d-94 to d-101, and d-103 to d-113, all of the compounds exhibited a growth inhibition rate of 75% or more.

### (Control test of cucumber gray mold)

5 parts by mass of the 5-membered heteroaryl compound according to the embodiment of the present invention, 93.5 parts by mass of N,N-dimethylformamide, and 1.5 parts by mass of polyoxyethylene sorbitan monolaurate were mixed to obtain an emulsifiable concentrate (1) containing 5% by mass of the active ingredient.

Water was added to the above-described emulsifiable concentrate (1) to obtain a chemical solution having a concentration of the 5-membered heteroaryl compound according to the embodiment of the present invention of 125 ppm by mass or 25 ppm by mass. Subsequently, the chemical solution was sprayed onto cucumber seedlings (cultivar "Jibai" line, cotyledon stage) grown in seedling pots, and then air-dried. Thereafter, a conidial suspension of cucumber gray mold fungus (Botrytis cinerea) was drop-inoculated onto the cucumber seedlings (treated plot). As a control, the conidial suspension of cucumber gray mold fungus (Botrytis cinerea) was drop-inoculated onto cucumber seedlings onto which the chemical solution had not been sprayed (untreated control). The seedlings were allowed to stand in a humid chamber at 20°C. Four days after inoculation, the leaves of the cucumber seedlings were visually observed, and a control value was calculated from the lesion area rate by the following formula.$\begin{matrix}Control value \left(%\right) = \\ 100 - \left\{Lesion area rate in treated plot/Lesion area rate in untreated control\right\} x \\ 100\end{matrix}$

The control test for cucumber gray mold was performed on the compounds of compound numbers d-35, d-36, d-44, d-45, d-47, d-48, d-53 to d-61, and d-66 to d-70 at a concentration of 125 ppm by mass. All of the compounds exhibited a control value of 75% or more.

### (Control test of apple scab)

Water was added to the emulsifiable concentrate (1) so that the concentration of the 5-membered heteroaryl compound according to the embodiment of the present invention was 125 ppm by mass, thereby obtaining a chemical solution. Subsequently, the chemical solution was sprayed onto apple seedlings (cultivar "Orin", 3 or 4 leaf stage) grown in seedling pots, and then air-dried. Thereafter, conidia of the apple scab fungus (Venturia inaequalis) were inoculated onto the seedlings (treated plot). As a control, the same inoculation was performed on apple seedlings onto which the chemical solution had not been sprayed (untreated control). The seedlings were allowed to stand in a humid chamber at 20°C under alternating 12-hour light and dark cycles.

Twelve to fourteen days after inoculation, the leaves of the apple seedlings were visually observed, and a control value was calculated from the lesion area rate using the formula employed for calculating the control value in the control test for cucumber gray mold described above.

The control test for apple scab was performed on the compounds of compound numbers d-35, d-36, d-55 to d-59, and d-61. All of the compounds exhibited a control value of 75% or more.

### (Control test of wheat powdery mildew)

Water was added to the emulsifiable concentrate (1) so that the concentration of the 5-membered heteroaryl compound according to the embodiment of the present invention was 125 ppm by mass, thereby obtaining a chemical solution. Subsequently, the chemical solution was sprayed onto wheat seedlings (cultivar "Chihoku", 1 or 2 leaf stage) grown in seedling pots, and air-dried. Thereafter, conidia of wheat powdery mildew fungus (Erysiphe graminis f. sp. tritici) were sprinkled and inoculated (treated plot). As a control, the conidia of wheat powdery mildew fungus (Erysiphe graminis f. sp. tritici) were sprinkled and inoculated onto wheat seedlings onto which the chemical solution had not been sprayed (untreated control). The seedlings were allowed to stand in a greenhouse at 20°C. Six days after inoculation, the leaves of the wheat seedlings were visually observed to determine the lesion area rate, and a control value was calculated in the same manner as in the previous test.

The control test for wheat powdery mildew was performed on the compounds of compound numbers d-35, d-36, d-44, d-45, d-55 to d-59, d-61, d-67, and d-69. All of the compounds exhibited a control value of 75% or more.

In addition to the above-described tests, the following tests can be used to evaluate translaminar activity of the compounds.

### (Translaminar activity test against cucumber gray mold)

5 parts by mass of the 5-membered heteroaryl compound according to the embodiment of the present invention, 93.5 parts by mass of N,N-dimethylformamide, and 1.5 parts by mass of polyoxyethylene sorbitan monolaurate were mixed to obtain an emulsifiable concentrate (1) containing 5% by mass of the active ingredient.

Water was added to the above-described emulsifiable concentrate (1) to obtain a chemical solution having a concentration of the 5-membered heteroaryl compound according to the embodiment of the present invention of 25 ppm by mass or 6.3 ppm by mass. Subsequently, the chemical solution was sprayed onto the abaxial surfaces of the leaves of cucumber seedlings (cultivar "Jibai" line, cotyledon stage) grown in seedling pots, air-dried, and then left to stand for 2 days. Thereafter, a conidial suspension of cucumber gray mold fungus (Botrytis cinerea) was drop-inoculated onto the adaxial surfaces of the cotyledons (treated plot). As a control, the conidial suspension of cucumber gray mold fungus (Botrytis cinerea) was drop-inoculated onto cucumber seedlings onto which the chemical solution had not been sprayed (untreated control). The seedlings were allowed to stand in a humid chamber at 20°C. Four days after inoculation, the leaves of the cucumber seedlings were visually observed, and a control value was calculated from the lesion area rate by the following formula.$\begin{matrix}Control value \left(%\right) = \\ 100 - \left\{Lesion area rate in treated plot/Lesion area rate in untreated control\right\} x \\ 100\end{matrix}$

Since the randomly selected 5-membered heteroaryl compounds according to the embodiment of the present invention each exhibited the above-described effects, it can be understood that the 5-membered heteroaryl compounds according to the embodiment of the present invention, containing compounds not specifically exemplified, have fungicidal effects and do not cause phytotoxicity to plants.

### INDUSTRIAL APPLICABILITY

The 5-membered heteroaryl compound according to the aspect of the present invention has excellent fungicidal activity against pathogenic fungi affecting agricultural and horticultural plants, has excellent safety, and can be industrially advantageously synthesized. The agricultural and horticultural fungicide according to the aspect of the present invention can effectively control diseases of agricultural and horticultural plants.

## Claims

1. A compound represented by Formula (Z-I) or a salt thereof, 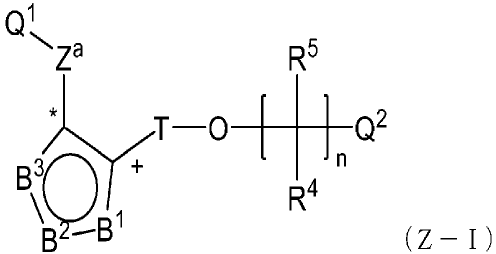 wherein, in Formula (Z-I),
Q¹ represents a substituted or unsubstituted phenyl group, a substituted or unsubstituted naphthyl group, a substituted or unsubstituted 5- or 6-membered heterocyclyl group, or a substituted or unsubstituted 9- or 10-membered heterocyclyl group,
Q² represents a substituted or unsubstituted phenyl group, a substituted or unsubstituted naphthyl group, a substituted or unsubstituted 5- or 6-membered heterocyclyl group, or a substituted or unsubstituted 9- or 10-membered heterocyclyl group,
in a partial structure of Formula (III), 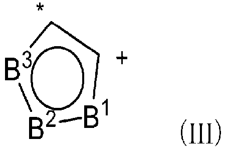
B¹ represents an oxygen atom, a nitrogen atom, a group represented by a formula: NR¹, or a group represented by a formula: CR²,
B² represents an oxygen atom, a nitrogen atom, a group represented by a formula: NR¹, or a group represented by a formula: CR²,
B³ represents an oxygen atom, a nitrogen atom, a group represented by a formula: NR¹, or a group represented by a formula: CR²,
any one of B¹, B², and B³ represents an oxygen atom or a group represented by the formula: NR¹, and the remaining two each represent a nitrogen atom or a group represented by the formula: CR²,
R¹'s each independently represent a substituted or unsubstituted C1-6 alkyl group or a substituted or unsubstituted C3-6 cycloalkyl group,
R²'s each independently represent a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted 5- or 6-membered heterocyclyl group, a substituted or unsubstituted phenyloxy group, a substituted or unsubstituted naphthyloxy group, a substituted or unsubstituted 5- or 6-membered heterocyclyloxy group, a substituted or unsubstituted amino group, a substituted or unsubstituted aminocarbonyl group, a nitro group, a cyano group, or a halogeno group, and
a carbon atom marked with * in the partial structure corresponds to a carbon atom marked with * in Formula (Z-I), and a carbon atom marked with + in the partial structure corresponds to a carbon atom marked with + in Formula (Z-I),
Z^{a} represents an oxygen atom or a sulfur atom,
T represents a partial structure (T-1) or a partial structure (T-2), 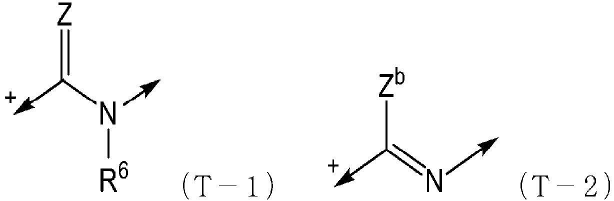
in the partial structure (T-1) or the partial structure (T-2), an arrow marked with + bonds to the carbon atom marked with + in the partial structure of Formula (III), and the other arrow bonds to an oxygen atom in Formula (Z-I),
in the partial structure (T-1), Z represents an oxygen atom or a sulfur atom,
R⁶ represents a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted 5- or 6-membered heterocyclyl group,
in the partial structure (T-2), Z^{b} represents a C1-6 alkoxy group or a C1-6 alkylthio group,
R⁴'s each independently represent a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted 5- or 6-membered heterocyclyl group, or a halogeno group,
R⁵'s each independently represent a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted 5- or 6-membered heterocyclyl group, or a halogeno group,
R⁴ and R⁵ on the same carbon atom may together form a divalent organic group, and
n represents any integer of 1 to 3.

2. The compound or a salt thereof according to Claim 1, wherein Formula (Z-I) is represented by Formula (I), 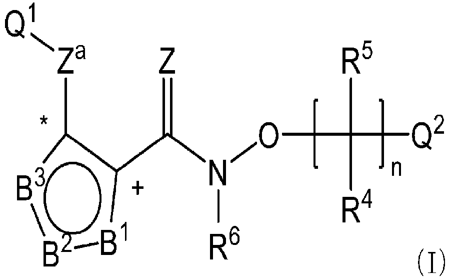 wherein, in Formula (I),
Q¹, Q², B¹, B², B³, Z^{a}, R⁴, R⁵, and n have the same meanings as Q¹, Q², B¹, B², B³, Z^{a}, R⁴, R⁵, and n in Formula (Z-I), and
Z and R⁶ have the same meanings as Z and R⁶ in the partial structure (T-1).

3. The compound or a salt thereof according to Claim 2,
wherein B¹ is a group represented by the formula: NR¹,
B² is a nitrogen atom,
B³ is a nitrogen atom,
Z^{a} is an oxygen atom,
Z is an oxygen atom,
R⁶ is a hydrogen atom, and
n is 1.

4. An agricultural and horticultural fungicide comprising, as an active ingredient: at least one selected from the group consisting of the compound according to any one of Claims 1 to 3 and a salt thereof.

5. The agricultural and horticultural fungicide according to Claim 4, wherein the agricultural and horticultural fungicide is for seed treatment.

6. The agricultural and horticultural fungicide according to Claim 4, further comprising: another active ingredient.

7. The agricultural and horticultural fungicide according to Claim 6, wherein the other active ingredient is at least one selected from the group consisting of the following compounds:
a nucleic acid biosynthesis inhibitor, a mitosis inhibitor, a cell division inhibitor, a respiration inhibitor, an amino acid or protein synthesis inhibitor, a signal transduction inhibitor, a lipid or cell membrane synthesis inhibitor, a sterol biosynthesis inhibitor in cell membranes, a cell wall synthesis inhibitor, a melanin biosynthesis inhibitor, a host plant resistance inducer, and an agent having multiple points of action; and
an acetylcholinesterase inhibitor, a GABA-gated chloride ion channel blocker, a sodium channel modulator, a nicotinic acetylcholine receptor competitive modulator, a nicotinic acetylcholine receptor allosteric modulator, a glutamate-gated chloride ion channel allosteric modulator, a juvenile hormone mimic, another non-specific inhibitor, a chordotonal organ TRPV channel modulator, a mite growth inhibitor, a microbially derived disruptor of insect midgut membranes, a mitochondrial ATP synthase inhibitor, an oxidative phosphorylation uncoupler that disrupts the proton gradient, a nicotinic acetylcholine receptor channel blocker, a chitin biosynthesis inhibitor type 0, a chitin biosynthesis inhibitor type 1, a molting inhibitor, an ecdysone receptor agonist, an octopamine receptor agonist, a mitochondrial electron transport chain complex III inhibitor, a mitochondrial electron transport chain complex I inhibitor, a voltage-dependent sodium channel blocker, an acetyl-CoA carboxylase inhibitor, a mitochondrial electron transport chain complex IV inhibitor, a mitochondrial electron transport chain complex II inhibitor, a ryanodine receptor modulator, a chordotonal organ modulator with an unspecified target site, and a GABA-gated chloride ion channel allosteric modulator.

8. The agricultural and horticultural fungicide according to Claim 6, wherein the other active ingredient is at least one selected from the group consisting of the following compounds:
benalaxyl, benalaxyl-M, furalaxyl, metalaxyl, metalaxyl-M, oxadixyl, clozylacon, and ofurace;
bupirimate, dimethirimol, and ethirimol;
hymexazol and octhilinone;
oxolinic acid;
benomyl, carbendazim, chlorfenazole, fuberidazole, thiabendazole, thiophanate, thiophanate-methyl, diethofencarb, zoxamide, and ethaboxam;
pencycuron;
fluopicolide and fluopimomide;
diflumetorim and tolfenpyrad;
benodanil, flutolanil, mepronil, isofetamid, fluopyram, fenfuram, flumecyclox, carboxin, oxycarboxin, thifluzamide, benzovindiflupyr, bixafen, fluxapyroxad, furametpyr, isopyrazam, penflufen, penthiopyrad, sedaxane, boscalid, pydiflumetofen, isoflucypram, pyraziflumid, and inpyrfluxam;
azoxystrobin, coumoxystrobin, coumethoxystrobin, enoxastrobin, flufenoxystrobin, picoxystrobin, pyraoxystrobin, pyraclostrobin, pyrametostrobin, triclopyricarb, kresoxim-methyl, trifloxystrobin, dimoxystrobin, fenaminstrobin, metominostrobin, orysastrobin, famoxadone, fluoxastrobin, fenamidone, pyribencarb, metyltetraprole, and mandestrobin;
cyazofamid, amisulbrom, and fenpicoxamid;
binapacryl, meptyldinocap, dinocap, fluazinam, and ferimzone;
fentin acetate, fentin chloride, and fentin hydroxide;
silthiofam;
ametoctradin;
andoprim, cyprodinil, mepanipyrim, and pyrimethanil;
blasticidin-S, kasugamycin, kasugamycin hydrochloride, streptomycin, and oxytetracycline;
quinoxyfen and proquinazid;
fenpiclonil, fludioxonil, chlozolinate, iprodione, procymidone, and vinclozolin;
edifenphos, iprobenfos, pyrazophos, and isoprothiolane;
biphenyl, chloroneb, dicloran, quintozene, tecnazene, tolclofos-methyl, and etridiazole;
iodocarb, propamocarb, propamocarb hydrochloride, propamocarb-fosetylate, and prothiocarb;
Bacillus subtilis, Bacillus subtilis strain QST713, Bacillus subtilis strain FZB24, Bacillus subtilis strain MBI600, Bacillus subtilis strain D747, and Bacillus amyloliquefaciens;
cajeput or tea tree extracts;
triforine, pyrifenox, pyrisoxazole, fenarimol, flurprimidol, nuarimol, imazalil, imazalil sulfate, oxpoconazole, oxpoconazole fumarate, pefurazoate, prochloraz, triflumizole, viniconazole, azaconazole, bitertanol, bromuconazole, cyproconazole, diclobutrazol, difenoconazole, diniconazole, diniconazole-M, epoxiconazole, etaconazole, fenbuconazole, fluquinconazole, flusilazole, flutriafol, fluconazole, fluconazole-cis, hexaconazole, imibenconazole, ipconazole, metconazole, myclobutanil, penconazole, propiconazole, fluquinconazole, simeconazole, tebuconazole, tetraconazole, triadimefon, triadimenol, triticonazole, prothioconazole, voriconazole, and mefentrifluconazole;
aldimorph, dodemorph, dodemorph acetate, fenpropimorph, tridemorph, fenpropidin, piperalin, and spiroxamine;
fenhexamid and fenpyrazamine;
pyributicarb, naftifine, and terbinafine;
validamycin;
polyoxin and polyoxorim;
dimethomorph, flumorph, pyrimorph, benthiavalicarb, benthiavalicarb-isopropyl, iprovalicarb, valifenalate, and mandipropamid;
fthalide, pyroquilon, and tricyclazole;
carpropamid, diclocymet, and fenoxanil;
tolprocarb;
acibenzolar-S-methyl;
probenazole, tiadinil, isotianil, diclobentiazox, ipfentrifluconazole, laminarin, giant knotweed extract, phosphorous acid, and phosphites;
cymoxanil, fosetyl-aluminum, phosphoric acid, phosphates, tecloftalam, triazoxide, flusulfamide, diclomezine, methasulfocarb, cyflufenamid, metrafenone, pyriofenone, dodine, dodine free base, and flutianil;
copper or copper salts, Bordeaux mixture, copper hydroxide, copper naphthenate, copper oxide, copper oxychloride, copper sulfate, sulfur, sulfur products, calcium polysulfide, ferbam, mancozeb, maneb, mancopper, metiram, polycarbamate, propineb, thiram, zineb, ziram, captan, captafol, folpet, chlorothalonil, dichlofluanid, tolylfluanid, guazatine, guazatine triacetate or iminoctadine triacetate, iminoctadine tris(albesilate), anilazine, dithianon, quinomethionate, and fluoroimide;
DBEDC, fluorofolpet, guazatine acetate, bis(8-quinolinolato)copper(II), propamidine, chloropicrin, cyproflam, Agrobacterium, bethoxazin, diphenylamine, methyl isothiocyanate or MITC, mildiomycin, capsaicin, cufraneb, cyprosulfamide, dazomet, debacarb, dichlorophen, difenzoquat, difenzoquat methylsulfonate, flumetover, fosetyl-calcium, fosetyl-sodium, irumamycin, natamycin, nitrothal-isopropyl, oxamocarb, pyrrolnitrin, tebufloquin, tolnifanide, zarilamide, Algophase, amicarthiazol, oxathiapiprolin, fluoxapiprolin, zinc metiram, benthiazole, triclamide, uniconazole, oxyfenthiin, picarbutrazox, diclobentiazox, quinofumelin, thiuram, ambam, Agrobacterium radiobacter, Coniothyrium minitans, Pseudomonas fluorescens, Pseudomonas rhodesia, Talaromyces flavus, Trichoderma atroviride, non-pathogenic Erwinia carotovora, Bacillus simplex, Variovorax paradoxus, Lactobacillus plantarum, florylpicoxamid, pyrapropoyne, fluindapyr, aminopyrifen, pyridachlometyl, ipflufenoquin, and dipymetitrone;
flufenoxadiazam, flumetylsulforim, and feneptamidquin;
alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC, xylylcarb, phenothiocarb, MIPC, MPMC, MTMC, aldoxycarb, alixycarb, aminocarb, bufencarb, cloethocarb, metam-sodium, and promecarb;
acephate, azamethiphos, azinphos-ethyl, azinphos-methyl, cadusafos, chlorethoxyphos, chlorfenvinphos, chlormephos, chlorpyrifos, chlorpyrifos-methyl, coumaphos, cyanophos, demeton-S-methyl, diazinon, dichlorvos, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isocarbophos, isopropyl=O-(methoxyaminothiophosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion, bromophos-ethyl, BRP, carbophenothion, cyanofenphos, CYAP, demeton-S-methylsulfone, dialifos, dichlofenthion, dioxabenzofos, etrimfos, fensulfothion, flupyrazofos, fonofos, formothion, phosmethylan, isazofos, iodofenphos, metacrifos, pirimiphos-ethyl, phosphocarb, propaphos, prothoate, and sulprofos;
acetoprole, chlordane, endosulfan, ethiprole, fipronil, pyrafluprole, pyriprole, camphechlor, heptachlor, dienochlor, and flufiprole;
acrinathrin, allethrin, d-cis-trans-allethrin, d-trans-allethrin, bifenthrin, bioallethrin, bioallethrin-S-cyclopentenyl isomer, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin [(1R)-trans isomer], deltamethrin, empenthrin [(EZ)-(1R)-isomer], esfenvalerate, etofenprox, fenpropathrin, fenvalerate, flucythrinate, flumethrin, tau-fluvalinate, halfenprox, imiprothrin, kadethrin, permethrin, phenothrin [(1R)-trans isomer], prallethrin, pyrethlam, pyrethrin, resmethrin, silafluofen, tefluthrin, tetramethrin, tetramethrin [(1R)-isomer], tralomethrin, transfluthrin, allethrin, pyrethrin I, pyrethrin II, profluthrin, dimefluthrin, bioethanomethrin, biopermethrin, transpermethrin, fenfluthrin, fenpyrithrin, flubrocythrinate, flufenprox, metofluthrin, protriphenbut, pyresmethrin, terallethrin, kappa-bifenthrin, chloroprallethrin, heptafluthrin, meperfluthrin, epsilon-metofluthrin, momfluorothrin, epsilon-momfluorothrin, kappa-tefluthrin, tetramethylfluthrin, and bioethanomethrin;
DDT and methoxychlor;
acetamiprid, clothianidin, dinotefuran, imidacloprid, nitenpyram, nithiazine, thiacloprid, thiamethoxam, nicotine, sulfoxaflor, flupyradifurone, triflumezopyrim, dichloromezotiaz, and flupyrimin;
spinetoram and spinosad;
abamectin, emamectin, emamectin benzoate, lepimectin, milbemectin, ivermectin, selamectin, doramectin, eprinomectin, ivermectin, moxidectin, selamectin, milbemycin, milbemycin oxime, and nemadectin;
hydroprene, kinoprene, methoprene, fenoxycarb, pyriproxyfen, diofenolan, epofenonane, and triprene;
methyl bromide, alkyl halides, chloropicrin, sodium aluminum fluoride, sulfuryl fluoride, borax, boric acid, disodium octaborate, sodium borate, sodium metaborate, tartar emetic, dazomet, metam, metam-potassium, and metam-sodium;
flonicamid, pymetrozine, pyrifluquinazon, and afidopyropen;
clofentezine, diflovidazin, hexythiazox, and etoxazole;
Bacillus thuringiensis subsp. israelensis, Bacillus sphaericus, Bacillus thuringiensis subsp. aizawai, Bacillus thuringiensis subsp. kurstaki, Bacillus thuringiensis subsp. tenebrionis, Cry1Ab, Cry1Ac, Cry1Fa, Cry1A.105, Cry2Ab, Vip3A, mCry3A, Cry3Ab, Cry3Bb, Cry34Ab1/Cry35Ab1, and Bacillus sphaericus;
diafenthiuron, azocyclotin, cyhexatin, fenbutatin oxide, propargite, and tetradifon;
chlorfenapyr, DNOC, sulfluramid, binapacryl, dinobuton, and dinocap;
bensultap, cartap hydrochloride, nereistoxin, thiocyclam, and thiosultap-sodium;
bistrifluron, chlorfluazuron, diflubenzuron, flucycloxuron, flufenoxuron, hexaflumuron, lufenuron, novaluron, noviflumuron, teflubenzuron, triflumuron, buprofezin, and fluazuron;
buprofezin;
cyromazine;
chromafenozide, halofenozide, methoxyfenozide, and tebufenozide;
amitraz, demiditraz, and chlordimeform;
hydramethylnon, acequinocyl, fluacrypyrim, and bifenazate;
fenazaquin, fenpyroximate, pyridaben, pyrimidifen, tebufenpyrad, tolfenpyrad, and rotenone;
indoxacarb and metaflumizone;
spirodiclofen, spiromesifen, spirotetramat, and spiropidion;
aluminum phosphide, calcium phosphide, zinc phosphide, phosphine, cyanide, calcium cyanide, sodium cyanide, and potassium cyanide;
cyenopyrafen, cyflumetofen, and pyflubumide;
chlorantraniliprole, cyantraniliprole, cyclaniliprole, flubendiamide, cyhalodiamide, tetrachlorantraniliprole, and tetraniliprole;
flonicamid;
broflanilide, fluxametamide, isocycloseram, afoxolaner, fluralaner, lotilaner, and sarolaner;
azadirachtin, benzoximate, bifenazate, bromopropylate, chinomethionat, cryolite, dicofol, lime sulfur, mancozeb, pyridalyl, benclothiaz, sulfur, amidoflumet, 1,3-dichloropropene, DCIP, phenisobromolate, benzomate, metaldehyde, chlorobenzilate, clothiazoben, dicyclanil, phenoxacrim, fentrifanil, flubenzimine, flufenazine, gossyplure, japonilure, methoxyadiazone, petroleum or oil, potassium oleate, tetrasul, triarathene, afidopyropen, flufiprole, fluensulfone, meperfluthrin, tetramethylfluthrin, tralopyril, dimefluthrin, methylneodecanamide, fluralaner, afoxolaner, fluxametamide, 5-[5-(3,5-dichlorophenyl)-5-trifluoromethyl-4,5-dihydroisoxazol-3-yl]-2-(1H-1,2,4-triazol-1-yl)benzonitrile, broflanilide, other metadiamides, Steinernema carpocapsae, Steinernema glaseri, Pasteuria penetrans, Paecilomyces tenuipes, Paecilomyces fumosoroseus, Beauveria bassiana, Beauveria brongniartii, Metarhizium anisopliae, Verticillium lecanii, acynonapyr, benzpyrimoxan, flometoquin, fluhexafon, oxazosulfyl, and tyclopyrazoflor;
fenbendazole, albendazole, triclabendazole, oxybendazole, mebendazole, oxfendazole, parbendazole, flubendazole, febantel, netobimin, thiophanate, thiabendazole, and cambendazole;
closantel, oxyclozanide, rafoxanide, and niclosamide;
nitroxynil and nitroscanate;
pyrantel and morantel;
levamisole and tetramisole;
praziquantel and epsiprantel;
cyclodiene, ryania, clorsulon, metronidazole, demiditraz, piperazine, diethylcarbamazine, dichlorophen, monepantel, tribendimidine, amidantel, thiacetarsamide, melarsomine, and arsenamide; and
abscisic acid, kinetin, benzylaminopurine, 1,3-diphenylurea, forchlorfenuron, thidiazuron, chlorfenuron, dihydrozeatin, gibberellin A, gibberellin A4, gibberellin A7, gibberellin A3, 1-methylcyclopropene, N-acetylaminoethoxyvinylglycine or aviglycine, aminooxyacetic acid, silver nitrate, cobalt chloride, IAA, 4-CPA, cloprop, 2,4-D, MCPB, indole-3-butyric acid, dichlorprop, phenothiol, 1-naphthylacetamide, ethychlozate, cloxyfonac, maleic hydrazide, 2,3,5-triiodobenzoic acid, salicylic acid, methyl salicylate, (-)-jasmonic acid, methyl jasmonate, (+)-strigol, (+)-deoxystrigol, (+)-orobanchol, (+)-sorgolactone, 4-oxo-4-(2-phenylethyl)aminobutyric acid, ethephon, chlormequat, mepiquat chloride, benzyladenine, 5-aminolevulinic acid, and daminozide.

9. A production method for a compound represented by Formula (I-3), the production method comprising: a step of reacting a compound represented by Formula (im-5) with a compound represented by Formula (im-6) or Formula (im-6-2) in the presence of a condensing agent when U¹ in Formula (im-5) is a hydroxyl group, or in the presence of an acid scavenger when U¹ is a halogeno group, 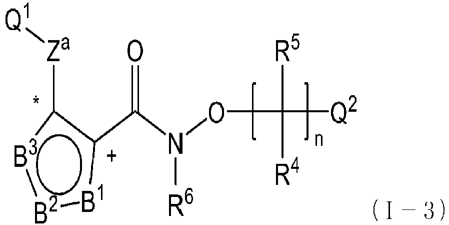 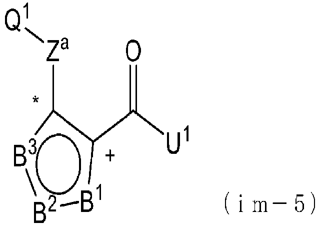 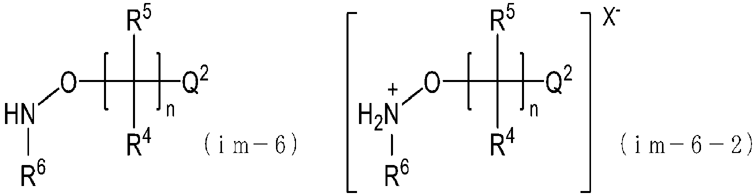 wherein, in the formulae,
Q¹ represents a substituted or unsubstituted phenyl group, a substituted or unsubstituted naphthyl group, a substituted or unsubstituted 5- or 6-membered heterocyclyl group, or a substituted or unsubstituted 9- or 10-membered heterocyclyl group,
Q² represents a substituted or unsubstituted phenyl group, a substituted or unsubstituted naphthyl group, a substituted or unsubstituted 5- or 6-membered heterocyclyl group, or a substituted or unsubstituted 9- or 10-membered heterocyclyl group,
in the following partial structure, 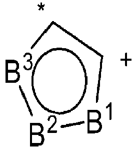
B¹ represents an oxygen atom, a nitrogen atom, a group represented by a formula: NR¹, or a group represented by a formula: CR²,
B² represents an oxygen atom, a nitrogen atom, a group represented by a formula: NR¹, or a group represented by a formula: CR²,
B³ represents an oxygen atom, a nitrogen atom, a group represented by a formula: NR¹, or a group represented by a formula: CR²,
any one of B¹, B², and B³ represents an oxygen atom or a group represented by the formula: NR¹, and the remaining two each represent a nitrogen atom or a group represented by the formula: CR²,
R¹'s each independently represent a substituted or unsubstituted C1-6 alkyl group or a substituted or unsubstituted C3-6 cycloalkyl group,
R²'s each independently represent a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted 5- or 6-membered heterocyclyl group, a substituted or unsubstituted phenyloxy group, a substituted or unsubstituted naphthyloxy group, a substituted or unsubstituted 5- or 6-membered heterocyclyloxy group, a substituted or unsubstituted amino group, a substituted or unsubstituted aminocarbonyl group, a nitro group, a cyano group, or a halogeno group,
a carbon atom marked with * in the partial structure corresponds to a carbon atom marked with * in Formula (I-3) or Formula (im-5), and a carbon atom marked with + in the partial structure corresponds to a carbon atom marked with + in Formula (I-3) or Formula (im-5),
Z^{a} represents an oxygen atom or a sulfur atom,
R⁴'s each independently represent a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted 5- or 6-membered heterocyclyl group, or a halogeno group,
R⁵'s each independently represent a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted 5- or 6-membered heterocyclyl group, or a halogeno group,
R⁴ and R⁵ on the same carbon atom may together form a divalent organic group,
n represents any integer of 1 to 3,
R⁶ represents a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted 5- or 6-membered heterocyclyl group,
X⁻ represents a counter anion, and
U¹ represents a hydroxyl group or a halogeno group.

10. A compound represented by Formula (im-5B) or a salt thereof, 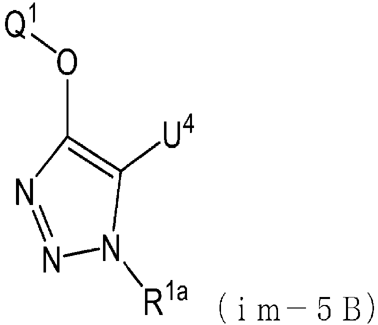 wherein, in Formula (im-5B),
Q¹ represents a substituted or unsubstituted phenyl group, a substituted or unsubstituted naphthyl group, a substituted or unsubstituted 5- or 6-membered heterocyclyl group, or a substituted or unsubstituted 9- or 10-membered heterocyclyl group,
R^{1a} represents a substituted or unsubstituted C2-6 alkyl group or a substituted or unsubstituted C3-6 cycloalkyl group, and
U⁴ represents a carboxyl group, a C1-6 alkoxycarbonyl group, or a halogenocarbonyl group.

11. A compound represented by Formula (im-25) or a salt thereof, 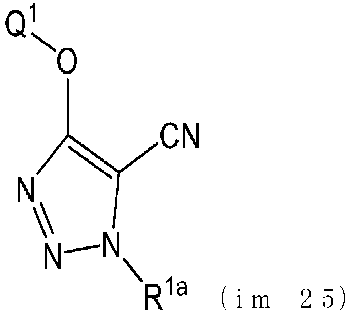 in Formula (im-25),
Q¹ represents a substituted or unsubstituted phenyl group, a substituted or unsubstituted naphthyl group, a substituted or unsubstituted 5- or 6-membered heterocyclyl group, or a substituted or unsubstituted 9- or 10-membered heterocyclyl group, and
R^{1a} represents a substituted or unsubstituted C2-6 alkyl group or a substituted or unsubstituted C3-6 cycloalkyl group.
